# EUROPEAN PATENT SPECIFICATION

(11) **EP 2 094 279 B1**
(45) Date of publication and mention of the grant of the patent: **07.01.2015**
(21) Application number: 07874280.6
(22) Date of filing: 15.11.2007
(51) Int. Cl.: A61K 31/7088, C07K 16/18, A61P 31/16, A61K 39/395

(54) **METHODS AND COMPOSITIONS FOR TREATING INFLUENZA**
VERFAHREN UND ZUSAMMENSETZUNGEN ZUR BEHANDLUNG VON INFLUENZA
PROCÉDÉS ET COMPOSITIONS POUR TRAITER LA GRIPPE

(30) Priority: 15.11.2006 US 858920 P
(43) Date of publication of application: 02.09.2009
(73) Proprietor: Eli Lilly and Company, Indianapolis, IN 46285 (US)
(72) Inventor: LI, Limin, Shenzhen, Guangdon 518040 (CN); KINCH, Michael, Gaithersburg, MD 20878 (US); GOLDBLATT, Michael, Gaithersburg, MD 20878 (US)
(74) Representative: Sexton, Jane Helen
(86) International application number: PCT/US2007/084759
(87) International publication number: WO 2008/140571

(56) References cited:
- WO-A2-2005/003297
- US-A1- 2003 148 388
- US-A1- 2005 170 334
- SHAO J ET AL: "Aberrant expression of PTCH (patched gene) and Smo (smoothened gene) in human pancreatic cancerous tissues and its association with hyperglycemia", PANCREAS 200607 US LNKD- DOI:10.1097/01.MPA.0000222319.59360.21, vol. 33, no. 1, July 2006 (2006-07), pages 38-44, XP008129001, ISSN: 0885-3177
- LI WU-BO ET AL: "Identification of PTCH1 requirement for influenza virus using random homozygous gene perturbation.", AMERICAN JOURNAL OF TRANSLATIONAL RESEARCH 2009 LNKD- PUBMED:19956436, vol. 1, no. 3, 2009, pages 259-266, XP002609813, ISSN: 1943-8141
- KOLB ET AL.: 'Resistance to influenza virus infection of Mx transgenic mice expressing Mx protein under the control of two constitutive promoters.' J. VIROL. MARCH vol. 66, no. 3, March 1992, pages 1709 - 1716, XP008109794
- PAVLOVIC ET AL.: 'Enhanced virus resistance of transgenic mice expressing the human MxA protein.' J. VIROL. vol. 69, no. 7, July 1995, pages 4506 - 4510, XP008110717

## Description

The present invention relates generally to the treatment of viral diseases, and in particular to diseases caused by influenza virus. The invention also relates to influenza resistant genes, polynucleotides transcribed from these genes and polypeptides encoded by these genes.

### BACKGROUND OF THE INVENTION

Influenza, also known as the flu, is a contagious disease that is caused by the influenza virus. It attacks the respiratory tract in humans (nose, throat, and lungs). There are three types of influenza viruses, influenza A, B and C. Influenza A can infect humans and other animals while influenza B and C infect only humans.

Most people who get influenza will recover in one to two weeks, but some people will develop life-threatening complications (such as pneumonia) as a result of the flu. Millions of people in the United States - about 5% to 20% of U.S. residents - will get influenza each year. An average of about 36,000 people per year in the United States die from influenza, and 114,000 per year have to be admitted to the hospital as a result of influenza. People age 65 years and older, people of any age with chronic medical conditions, and very young children are more likely to get complications from influenza. Pneumonia, bronchitis, and sinus and ear infections are three examples of complications from flu. The flu can also make chronic health problems worse. For example, people with asthma may experience asthma attacks while they have the flu, and people with chronic congestive heart failure may have worsening of this condition that is triggered by the flu.

Vaccination is the primary method for preventing influenza and its severe complications. Studies revealed that vaccination is associated with reductions in influenza- related respiratory illness and physician visits among all age groups, hospitalization and death among persons at high risk, otitis media among children, and work absenteeism among adults (18). The major problem with vaccination is that new vaccine has to be prepared for each flu season and the vaccine production is a tedious and costly process.

Although influenza vaccination remains the cornerstone for the control and treatment of influenza, three antiviral drugs (amantadine, rimantadine, and oseltamivir) have been approved for preventing and treating flu. When used for prevention, they are about 70% to 90% effective for preventing illness in healthy adults. When used for treating flu, these drugs can reduce the symptoms of the flu and shorten the time you are sick by 1 or 2 days. They also can make you less contagious to others. However, the treatment must begin within 2 days of the onset of symptoms for it to be effective. There is a need in the art for improved methods for treating influenza.

Shao J et al, 2006, Pancreas, vol. 33, no. 1 relates to aberrant expression of PTCH (patched gene) and Smo (smoothened gene) in human pancreatic cancerous tissues and its association with hyperglycemia.

### SUMMARY OF THE INVENTION

One aspect of the present invention relates to influenza resistant genes (IRGs) and the gene products (IRG products), which include the polynucleotides transcribed from the IRGs (IRGPNs) and the polypeptides encoded by the IRGs (IRGPPs). More specifically the invention provides an antibody which binds to a polypeptide comprising the amino acid sequence of SEQ ID NO: 17 (PTCH) for use in a method for enhancing the resistance of a mammal to infection by an influenza virus or a method to treat influenza virus infection in a mammal.

In one embodiment, the present invention provides pharmaceutical compositions for the treatment of influenza. The invention provides a pharmaceutical composition for use in a method of enhancing the resistance of a mammal to influenza virus, wherein said composition comprises an antibody suspended in a pharmaceutically acceptable carrier, which antibody binds to a polypeptide comprising the amino acid sequence of SEQ ID NO: 17 (PTCH).

The patient to be treated may be afflicted with influenza, in which case the methods provide treatment for the disease. The patient may also be considered at risk for influenza, in which case the methods provide prevention for disease development.

### BRIEF DESCRIPTION OF FIGURES

Figure 1 depicts the process for screening influenza resistant clones.
Figure 2A is the alignment of the 5'-end flanking sequences obtained from three subclones of influenza resistant clone 26-8-7; Figure 2B depicts the genomic site of the RHKO integration; and Figure 2C is a schematic map of integration.
Figure 3A is the alignment of the 5'-end flanking sequences obtained from two subclones of influenza resistant clone R18-6; Figure 3B depicts the genomic site of the RHKO integration; and Figure 3C is a schematic map of integration.
Figure 4A is the alignment of the 5'-end flanking sequences obtained from three subclones of influenza resistant clone 26-8-11; Figure 4B depicts the genomic site of the RHKO integration; and Figure 4C is a schematic map of integration.
Figure 5A is the alignment of the 5'-end flanking sequences obtained from three subclones of influenza resistant clone R15-6; Figure 5B depicts the genomic site of the RHKO integration; and Figure 5C is a schematic map of integration.
Figure 6A is the alignment of the 5'-end flanking sequences obtained from three subclones of influenza resistant clone R21-1; Figure 6B depicts the genomic site of the RHKO integration; and Figure 6C is a schematic map of integration.
Figure 7 depicts the genomic site of the RHKO integration in influenza resistant clone R27-32.
Figure 8A is the alignment of the 5'-end flanking sequences obtained from two subclones of influenza resistant clone R27-3-33; Figure 8B depicts the genomic site of the RHKO integration; and Figure 8C is a schematic map of integration.
Figure 9A depicts the genomic site of RHKO integration in influenza resistant clone R27-3-35 and Figure 9B is a schematic map of integration.

### DETAILED DESCRIPTION OF THE INVENTION

The preferred embodiments of the invention are described below. Unless specifically noted, it is intended that the words and phrases in the specification and claims be given the ordinary and accustomed meaning to those of ordinary skill in the applicable art or arts. If any other meaning is intended, the specification will specifically state that a special meaning is being applied to a word or phrase.

It is further intended that the inventions not be limited only to the specific structure, material or acts that are described in the preferred embodiments, but in addition, include any and all structures, materials or acts that perform the claimed function, along with any and all known or later-developed equivalent structures, materials or acts for performing the claimed function.

Further examples exist throughout the disclosure, and it is not applicant's intention to exclude from the scope of his invention the use of structures, materials, methods, or acts that are not expressly identified in the specification, but nonetheless are capable of performing a claimed function.

The present invention is generally directed to compositions and methods for the treatment and prevention of influenza; and to the identification of novel therapeutic agents for influenza. The present invention is based on the finding that modulation of certain gene expression leads to resistance to the infection by influenza virus.

### Definitions and Terms

To facilitate an understanding of the present invention, a number of terms and phrases are defined below:

As used herein, the term "influenza resistant gene (IRG)" refer to a gene whose inhibition or over-expression leads to resistance to infection by influenza virus. IRGs generally refer to the genes listed in Table 3.

As used herein, the terms "IRG-related polynucleotide", "IRG- polynucleotide" and "IRGPN" are used interchangeably. The terms include a transcribed polynucleotide (e.g., DNA, cDNA or mRNA) that comprises one of the IRG sequences or a portion thereof.

As used herein, the terms "IRG-related polypeptide (IRGPP)", "IRG protein" and "IRGPP" are used interchangeably. The terms include polypeptides encoded by an IRG, an IRGPN, or a portion of an IRG or IRGPN.

As used herein, an "IRG product" includes a nucleic acid sequence and an amino acid sequence (e.g., a polynucleotide or polypeptide) generated when an IRG is transcribed and/or translated. Specifically, IRG products include IRGPNs and IRGPPs.

As used herein, a "variant of a polynucleotide" includes a polynucleotide that differs from the original polynucleotide by one or more substitutions, additions, deletions and/or insertions such that the activity of the encoded polypeptide is not substantially changed (e.g., the activity may be diminished or enhanced, by less than 50%, and preferably less than 20%) relative to the polypeptide encoded by the original polynucleotide.

A variant of a polynucleotide also includes polynucleotides that are capable of hybridizing under reduced stringency conditions, more preferably stringent conditions, and most preferably highly stringent conditions to the original polynucleotide (or a complementary sequence). Examples of conditions of different stringency are listed in Table 2.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention.

As used herein, a "variant of a polypeptide" is a polypeptide that differs from a native polypeptide in one or more substitutions, deletions, additions and/or insertions, such that the bioactivity or immunogenicity of the native polypeptide is not substantially diminished. In other words, the bioactivity of a variant polypeptide or the ability of a variant polypeptide to react with antigen-specific antisera may be enhanced or diminished by less than 50%, and preferably less than 20%, relative to the native polypeptide. Variant polypeptides include those in which one or more portions, such as an N-terminal leader sequence or transmembrane domain, have been removed. Other preferred variants include variants in which a small portion (e.g., 1-30 amino acids, preferably 5-15 amino acids) has been removed from the N-and/or C-terminal of the mature protein.

Modifications and changes can be made in the structure of a polypeptide of the present invention and still obtain a molecule having biological activity and/or immunogenic properties. Because it is the interactive capacity and nature of a polypeptide that defines that polypeptide's biological activity, certain amino acid sequence substitutions can be made in a polypeptide sequence (or, of course, its underlying DNA coding sequence) and nevertheless obtain a polypeptide with like properties.

In making such changes, the hydropathic index of amino acids can be considered. The importance of the hydropathic amino acid index in conferring interactive biologic function on a polypeptide is generally understood in the art. It is believed that the relative hydropathic character of the amino acid residue determines the secondary and tertiary structure of the resultant polypeptide, which in turn defines the interaction of the polypeptide with other molecules, such as enzymes, substrates, receptors, antibodies, antigens, and the like. It is known in the art that an amino acid can be substituted by another amino acid having a similar hydropathic index and still obtain a functionally equivalent polypeptide. In such changes, the substitution of amino acids whose hydropathic indices are within +/-2 is preferred, those that are within +/-1 are particularly preferred, and those within +/-0.5 are even more particularly preferred.

Substitution of like amino acids can also be made on the basis of hydrophilicity, particularly where the biological functional equivalent polypeptide or polypeptide fragment, is intended for use in immunological embodiments. U.S. Patent 4,554,101, states that the greatest local average hydrophilicity of a polypeptide, as governed by the hydrophilicity of its adjacent amino acids, correlates with its immunogenicity and antigenicity, i.e. with a biological property of the polypeptide.

As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); proline (-0.5 ± 1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent polypeptide. In such changes, the substitution of amino acids whose hydrophilicity values are within ±2 is preferred, those that are within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine (See Table 1, below). The present invention thus contemplates functional or biological equivalents of an IRGPP as set forth above.

**TABLE 1, Amino Acid Substitutions**

| Original Residue | Exemplary Residue Substitution |
|---|---|
| Ala | Gly; Ser |
| Arg | Lys |
| Asn | Gln; His |
| Asp | Glu |
| Cys | Ser |
| Gln | Asn |
| Glu | Asp |
| Gly | Ala |
| His | Asn; Gln |
| He | Leu; Val |
| Leu | He; Val |
| Lys | Arg |
| Met | Leu; Tyr |
| Ser | Thr |
| Thr | Ser |
| Trp | Tyr |
| Tyr | Trp; Phe |
| Val | Ile; Leu |

A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure, tertiary structure, and hydropathic nature of the polypeptide.

Polypeptide variants preferably exhibit at least about 70%, more preferably at least about 90% and most preferably at least about 95% sequence homology to the original polypeptide.

A polypeptide variant also includes a polypeptide that is modified from the original polypeptide by either natural processes, such as post-translational processing, or by chemical modification techniques which are well known in the art. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from post-translation natural processes or may be made by synthetic methods. Modifications include acetylation, acylation, ADP-ribosylation, amidation, covalent attachment of flavin, covalent attachment of a fluorophore or a chromophore, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination.

As used herein, a "biologically active portion" of an IRGPP includes a fragment of an IRGPP comprising amino acid sequences sufficiently homologous to or derived from the amino acid sequence of the IRGPP, which includes fewer amino acids than the full length IRGPP, and exhibits at least one activity of the IRGPP. Typically, a biologically active portion of an IRGPP comprises a domain or motif with at least one activity of the IRGPP. A biologically active portion of an IRGPP can be a polypeptide which is, for example, 10, 25, 50, 100, 200 or more amino acids in length. Biologically active portions of an IRGPP can be used as targets for developing agents which modulate an IRGPP-mediated activity.

As used herein, an "immunogenic portion," an "antigen," an "immunogen," or an "epitope" of an IRGPP includes a fragment of an IRGPP comprising an amino acid sequence sufficiently homologous to, or derived from, the amino acid sequence of the IRGPP, which includes fewer amino acids than the full length IRGPP and can be used to induce an anti-IRGPP humoral and /or cellular immune response.

As used herein, the term "modulation" includes, in its various grammatical forms (e.g., "modulated", "modulation", "modulating", etc.), up-regulation, induction, stimulation, potentiation, and/or relief of inhibition, as well as inhibition and/or down-regulation or suppression.

As used herein, the term "control sequences" or "regulatory sequences" refers to DNA sequences necessary for the expression of an operably linked coding sequence in a particular host organism. The term "control/regulatory sequence" is intended to include promoters, enhancers and other expression control elements (e.g., polyadenylation signals). Control/regulatory sequences include those which direct constitutive expression of a nucleotide sequence in many types of host cells and those which direct expression of the nucleotide sequence only in certain host cells (e.g., tissue-specific regulatory sequences).

A nucleic acid sequence is "operably linked" to another nucleic acid sequence when the former is placed into a functional relationship with the latter. For example, a DNA for a presequence or secretory leader peptide is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, synthetic oligonucleotide adaptors or linkers are used in accordance with conventional practice.

As used herein, the "stringency" of a hybridization reaction refers to the difficulty with which any two nucleic acid molecules will hybridize to one another. The present disclosure also includes polynucleotides capable of hybridizing under reduced stringency conditions, more preferably stringent conditions, and most preferably highly stringent conditions, to polynucleotides described herein. Examples of stringency conditions are shown in Table 2 below: highly stringent conditions are those that are at least as stringent as conditions A-F; stringent conditions are at least as stringent as conditions G-L; and reduced stringency conditions are at least as stringent as conditions M-R.

**Table 2. Stringency Condition**

| Stringency Condition | Polynucleotide Hybrid | Hybrid Length (bp)¹ | Hybridization Temperature and Buffer^{H} | Wash Temp. and Buffer¹¹ |
|---|---|---|---|---|
| A | DNA:DNA | >50 | 65°C; 1xSSC -or-42°C; 1xSSC, 50% formamide | 65°C; 0.3xSSC |
| B | DNA:DNA | <50 | T_{B}*; 1xSSC | T_{B}*; 1xSSC |
| C | DNA:RNA | >50 | 67°C; 1xSSC -or-45°C; 1xSSC, 50% formamide | 67°C; 0.3xSSC |
| D | DNA:RNA | <50 | T_{D}*;1xSSC | T_{D}*; 1xSSC |
| E | RNA:RNA | >50 | 70°C; 1xSSC -or-50°C; 1xSSC, 50% formamide | 70°C; 0.3xSSC |
| F | RNA:RNA | <50 | T_{F}*; 1xSSC | T_{F}*; 1xSSC |
| G | DNA:DNA | >50 | 65°C; 4xSSC -or-42°C; 4xSSC, 50% formamide | 65°C; 1xSSC |
| H | DNA:DNA | <50 | T_{H}*; 4xSSC | T_{H}*; 4xSSC |
| I | DNA:RNA | >50 | 67°C; 4xSSC -or-45°C; 4xSSC, 50% formamide | 67°C; 1xSSC |
| J | DNA:RNA | <50 | T_{J}*; 4xSSC | T_{J}*; 4xSSC |
| K | RNA:RNA | >50 | 70°C; 4xSSC -or-50°C; 4xSSC, 50% formamide | 67°C; 1xSSC |
| L | RNA:RNA | <50 | T_{L}*; 2xSSC | T_{L}*; 2xSSC |
| M | DNA:DNA | >50 | 50°C; 4xSSC -or-40°C; 6xSSC, 50% formamide | 50°C; 2xSSC |
| N | DNA:DNA | <50 | T_{N}*; 6xSSC | T_{N}*; 6xSSC |
| O | DNA:RNA | >50 | 55°C; 4xSSC -or-42°C; 6xSSC, 50% formamide | 55°C; 2xSSC |
| P | DNA:RNA | <50 | T_{P}*; 6xSSC | T_{P}*; 6xSSC |
| Q | RNA:RNA | >50 | 60°C; 4xSSC -or-45°C; 6xSSC, 50% formamide | 60°C; 2xSSC |
| R | RNA:RNA | <50 | T_{R}*; 4xSSC | T_{R}*; 4xSSC |

| | | | | |
|---|---|---|---|---|
| ¹: The hybrid length is that anticipated for the hybridized region(s) of the hybridizing polynucleotides. When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. ^{H}: SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes after hybridization is complete. T_{B}* - T_{R}*: The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tm(°C) = 81.5 ⁺ 16.6(log₁₀Na⁺)⁺ 0.41(%G⁺C) - (600/N), where N is the number of bases in the hybrid, and Na⁺ is the concentration of sodium ions in the hybridization buffer (Na⁺ for 1xSSC = 0.165M). | | | | |

As used herein, the terms "immunospecific binding" and "specifically bind to" refer to antibodies that bind to an antigen with a binding affinity of 10⁵ M⁻¹.

As used herein, the terms "treating," "treatment," and "therapy" refer to curative therapy, prophylactic therapy, and preventative therapy.

Various aspects of the invention are described in further detail in the following subsections. The subsections below describe in more detail the present invention. The use of subsections is not meant to limit the invention; subsections may apply to any aspect of the invention.

### Influenza resistant genes (IRGs)

One aspect of the present invention relates to influenza resistance genes (IRGs). Briefly, Madin Darby Canine Kidney (MDCK) cells were infected with a retro-viral based random homozygous knock-out (RHKO) vector. Cells containing the stably integrated vector were selected and subjected to influenza infection using the MOI which would result in 100% killing of parental cells between 48 to 72 hour. The influenza resistant cells were expanded and subject to additional rounds of influenza infection with higher multiplicity of infection (MOI). The resistant clones that survived multiple rounds of influenza infection were recovered. The influenza resistant phenotype was validated by testing the clones' resistance to multiple strains of influenza virus and by correlation of the phenotype with RHKO integration. The RHKO integration sites in the resistant cells were then cloned and identified. The affected genes are identified by aligning the flanking sequences at the integration site to the Genbank database. It should be noted that the affected genes, which are referred to as influenza resistant genes hereinafter, are either under-expressed (i.e., inhibited by RHKO integration) or over-expressed (i.e., enhanced by RHKO integration) in the influenza resistant cells.

Table 3 provides a list of the genes that, when over-expressed or under-expressed in a cell, lead to resistance to influenza virus infection. Accordingly, genes listed in Table 3 are designated as influenza resistance genes (IRGs).

| Gene | Locus ID | 5'-flanking seq at insertion site | cDNA sequence | Amino acid sequence | predicted effect of integration |
|---|---|---|---|---|---|
| PTCH | 5727 | SEQ ID NO:1 | SEQ ID NO:9 | SEQ ID NO:17 | antisense |
| PSMD2 | 5708 | SEQ ID NO:2 | SEQ ID NO:10 | SEQ ID NO:18 | over-expression |
| NMT1 | 4836 | SEQ ID NO:3 | SEQ ID NO:11 | SEQ ID NO:19 | over-expression |
| MARCO | 8685 | SEQ ID NO:4 | SEQ ID NO:12 | SEQ ID NO:20 | disruption of promoter |
| CDK6 | 1021 | SEQ ID NO:5 | SEQ ID NO:13 | SEQ ID NO:21 | disruption of promoter |
| FLJ16046 | 389208 | SEQ ID NO:6 | SEQ ID NO:14 | SEQ ID NO:22 | over-expression |
| PCSK6 | 5046 | SEQ ID NO:7 | SEQ ID NO:15 | SEQ ID NO:23 | antisense |
| PTGDR | 5729 | SEQ ID NO:8 | SEQ ID NO:16 | SEQ ID NO:24 | antisense |

Briefly, PTCH (patched homolog of Drosophila) encodes a member of the patched gene family. The encoded protein is the receptor for sonic hedgehog, a secreted molecule implicated in the formation of embryonic structures and in tumorigenesis. This gene functions as a tumor suppressor. Mutations of this gene have been associated with nevoid basal cell carcinoma syndrome, esophageal squamous cell carcinoma, trichoepitheliomas, transitional cell carcinomas of the bladder, as well as holoprosencephaly. Alternative spliced variants have been described, but their full length sequences have not be determined.

PSMD2 (proteasome (prosome, macropain) 26S subunit, non-ATPase 2) encodes a multicatalytic proteinase complex with a highly ordered structure composed of 2 complexes, a 20S core and a 19S regulator. The 20S core is composed of 4 rings of 28 non-identical subunits; 2 rings are composed of 7 alpha subunits and 2 rings are composed of 7 beta subunits. The 19S regulator is composed of a base, which contains 6 ATPase subunits and 2 non-ATPase subunits, and a lid, which contains up to 10 non-ATPase subunits. Proteasomes are distributed throughout eukaryotic cells at a high concentration and cleave peptides in an ATP/ubiquitin-dependent process in a non-lysosomal pathway. An essential function of a modified proteasome, the immunoproteasome, is the processing of class I MHC peptides. This gene encodes one of the non-ATPase subunits of the 19S regulator lid. In addition to participation in proteasome function, this subunit may also participate in the TNF signalling pathway since it interacts with the tumor necrosis factor type 1 receptor. A pseudogene has been identified on chromosome 1.

NMT1 (N-myristoyltransferase 1) encodes N-Myristoyltransferase which is an essential eukaryotic enzyme that catalyzes the cotranslational and/or posttranslational transfer of myristate to the amino terminal glycine residue of a number of important proteins especially the non-receptor tyrosine kinases whose activity is important for tumorigenesis. Human NMT was found to be phosphorylated by non-receptor tyrosine kinase family members of Lyn, Fyn and Lck and dephosphorylated by the Ca(2+)/calmodulin-dependent protein phosphatase, calcineurin. NMT has been assoicated with HIV particle formation and budding. Chronically HIV-1-infected T-cell line CEM/LAV-1 exhibited low expression levels ofNMT (Takamune et al., FEBS Lett. 506:81-84, 2001).

MARCO (macrophage receptor with collagenous structure) encodes a member of the class A scavenger receptor family which is part of the innate antimicrobial immune system. The protein may bind both Gram-negative and Gram-positive bacteria via an extracellular, C-terminal, scavenger receptor cysteine-rich (SRCR) domain. In addition to short cytoplasmic and transmembrane domains, there is an extracellular spacer domain and a long, extracellular collagenous domain. The protein may form a trimeric molecule by the association of the collagenous domains of three identical polypeptide chains.

CDK6 (cyclin-dependent kinase) encodes a member of the cyclin-dependent protein kinase (CDK) family. CDK family members are highly similar to the gene products of Saccharomyces cerevisiae cdc28, and Schizosaccharomyces pombe cdc2, and are known to be important regulators of cell cycle progression. This kinase is a catalytic subunit of the protein kinase complex that is important for cell cycle G1 phase progression and G1/S transition. The activity of this kinase first appears in mid-G1 phase, which is controlled by the regulatory subunits including D-type cyclins and members of INK4 family of CDK inhibitors. This kinase, as well as CDK4, has been shown to phosphorylate, and thus regulate the activity of, tumor suppressor protein Rb.

FLJ16046 encodes the last exon of a novel protein. The protein share some homology with a domain found in sea urchin sperm protein, enterokinase, and the trans membrane domain of tyrosine-like serine protease.

PCSK6 (proprotein convertase subtilisin/kexin type 6) encodes a protein of the subtilisin-like proprotein convertase family. The members of this family are proprotein convertases that process latent precursor proteins into their biologically active products. This encoded protein is a calcium-dependent serine endoprotease that can cleave precursor protein at their paired basic amino acid processing sites. Some of its substrates are - transforming growth factor beta related proteins, proalbumin, and von Willebrand factor. This gene is thought to play a role in tumor progression. There are eight alternatively spliced transcript variants encoding different isoforms described for this gene.

PTGDR (prostaglandin D2 receptor (DP)) encodes a G-protein-coupled receptor that has been shown to function as a prostanoid DP receptor. The activity of this receptor is mainly mediated by G-S proteins that stimulate adenylate cyclase resulting in an elevation of intracellular cAMP and Ca²⁴. Knockout studies in mice suggest that the ligand of this receptor, prostaglandin D2 (PGD2), functions as a mast cell-derived mediator to trigger asthmatic responses.

### IRGs and IRG products as therapeutic targets for influenza

In general, Table 3 provides genes that relate to a cell's susceptibility to influenza virus infection. The IRGs of Table 3, as well as the corresponding IRG products (IRGPN and IRGPP) may become novel therapeutic targets for the treatment and prevention of influenza. The IRGs can be used to produce antibodies specific to IRG products, and to construct gene therapy vectors that inhibit the development of influenza. In addition, the IRG products themselves may be used as therapeutic agent for influenza.

The IRGs listed in Table 3 can be administered for gene therapy purposes, including the administration of antisense nucleic acids and RNAi. The IRG products (including IRGPPs and IRGPNs) and modulator of IRG products (such as anti-TRGPP antibodies) can also be administered as therapeutic drugs.

For example, the inhibition of IRG PTCH expression leads to resistance to influenza virus infection. Accordingly, influenza may be prevented or treated by down-regulating the PTCH expression. Similarly, the over-expression of IRG NMT1 leads to resistance to influenza virus infection. Accordingly, influenza may be prevented or treated by enhancing NMT1 expression.

### Sources of IRG Products

The IRG products (IRGPNs and IRGPPs) described herein may be isolated from any tissue or cell of a subject. It will be apparent to one skilled in the art that bodily fluids, such as blood, may also serve as sources from which the IRG product described herein may be assessed. A biological sample may comprise biological components such as blood plasma, serum, erythrocytes, leukocytes, blood platelets, lymphocytes, macrophages, fibroblast cells, mast cells, fat cells, neuronal cells or epithelial cells. The tissue samples containing one or more of the IRG product themselves may be useful in the methods described herein and one skilled in the art will be cognizant of the methods by which such samples may be conveniently obtained, stored and/or preserved.

### Isolated polynucleotides

Described herein are isolated polynucleotides. Also described are isolated polynucleotide fragments sufficient for use as hybridization probes to identify an IRGPN in a sample, as well as nucleotide fragments for use as PCR probes/primers of the amplification or mutation of the nucleic acid molecules which encode the IRGPP described herein.

An IRGPN molecule described wherein, e.g., a polynucleotide molecule having the nucleotide sequence of one of the IRGs listed in Table 3, or homologs thereof, or a portion thereof, can be isolated using standard molecular biology techniques and the sequence information provided herein, as well as sequence information known in the art. Using all or a portion of the polynucleotide sequence of one of the IRGs listed Table 3 (or a homolog thereof) as a hybridization probe, an IRG of the invention or an IRGPN of the invention can be isolated using standard hybridization and cloning techniques.

An IRGPN described herein can be amplified using cDNA, mRNA or alternatively, genomic DNA, as a template and appropriate oligonucleotide primers according to standard PCR amplification techniques. The polynucleotide so amplified can be cloned into an appropriate vector and characterized by DNA sequence analysis. Furthermore, oligonucleotides corresponding to IRG nucleotide sequences described herein can be prepared by standard synthetic techniques, e.g., using an automated DNA synthesizer.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. One such amplification technique is inverse PCR, which uses restriction enzymes to generate a fragment in the known region of the gene. A variation on this procedure, which employs two primers that initiate extension in opposite directions from the known sequence, is described in WO 96/38591.

Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:11-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

In another preferred embodiment, an isolated polynucleotide molecule described herein can comprise a polynucleotide molecule which is a complement of the nucleotide sequence of an IRG listed in Table 3, or homolog thereof, an IRGPN described herein, or a portion of any of these nucleotide sequences. A polynucleotide molecule which is complementary to such a nucleotide sequence is one which is sufficiently complementary to the nucleotide sequence such that it can hybridize to the nucleotide sequence, thereby forming a stable duplex.

The polynucleotide molecule described herein, moreover, can comprise only a portion of the polynucleotide sequence of an IRG, for example, a fragment which can be used as a probe or primer. The probe/primer typically comprises a substantially purified oligonucleotide. The oligonucleotide typically comprises a region of nucleotide sequence that hybridizes under stringent conditions to at least about 7 or 15, preferably about 25, more preferably about 50, 75, 100, 125, 150, 175, 200, 225, 250, 275, 300, 325, 350, 400 or more consecutive nucleotides of an IRG or an IRGPN described herein.

Probes based on the nucleotide sequence of an IRG or an IRGPN described herein can be used to detect transcripts or genomic sequences corresponding to the IRG or IRGPN described herein. In preferred embodiments, the probe comprises a label group attached thereto, e.g., the label group can be a radioisotope, a fluorescent compound, an enzyme, or an enzyme cofactor. Such probes can be used as a part of a diagnostic kit for identifying cells or tissue which misexpress (e.g., over- or under-express) an IRG, or which have greater or fewer copies of an IRG. For example, a level of an IRG product in a sample of cells from a subject may be determined, or the presence of mutations or deletions of an IRG described herein may be assessed.

Also described are polynucleotide molecules that differ from the polynucleotide sequences of the IRGs listed in Table 3 but encode the same proteins as those encoded by the genes shown in Table 3 due to degeneracy of the genetic code.

Also described are homologs of the IRGs listed in Table 3 of other species. Gene homologs are well understood in the art and are available using databases or search engines such as the Pubmed-Entrez database.

Also described are polynucleotide molecules which are structurally different from the molecules described above (i.e., which have a slight altered sequence), but which have substantially the same properties as the molecules above (e.g., encoded amino acid sequences, or which are changed only in non-essential amino acid residues). Such molecules include allelic variants, and are described in greater detail in subsections herein.

In addition to the nucleotide sequences of the IRGs listed in Table 3, it will be appreciated by those skilled in the art that DNA sequence polymorphisms that lead to changes in the amino acid sequences of the proteins encoded by the IRGs listed in Table 3 may exist within a population (e.g., the human population). Such genetic polymorphism in the IRGs listed in Table 3 may exist among individuals within a population due to natural allelic variation. An allele is one of a group of genes which occur alternatively at a given genetic locus. In addition it will be appreciated that DNA polymorphisms that affect RNA expression levels can also exist that may affect the overall expression level of that gene (e.g., by affecting regulation or degradation). As used herein, the phrase "allelic variant" includes a nucleotide sequence which occurs at a given locus or to a polypeptide encoded by the nucleotide sequence.

Polynucleotide molecules corresponding to natural allelic variants and homologs of the IRGs can be isolated based on their homology to the IRGs listed in Table 3, using the cDNAs disclosed herein, or a portion thereof, as a hybridization probe according to standard hybridization techniques under stringent hybridization conditions. Polynucleotide molecules corresponding to natural allelic variants and homologs of the IRGs described herein can further be isolated by mapping to the same chromosome or locus as the IRGs described herein.

In another embodiment, an isolated polynucleotide molecule described herein can be at least 15, 20, 25, 30, 50, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000 or more nucleotides in length and hybridizes under stringent conditions to a polynucleotide molecule corresponding to a nucleotide sequence of an IRG described herein. Preferably, the isolated polynucleotide molecule described herein hybridizes under stringent conditions to the sequence of one of the IRGs set forth in Table 3, or corresponds to a naturally-occurring polynucleotide molecule.

In addition to naturally-occurring allelic variants of the IRG described herein that may exist in the population, the skilled artisan will further appreciate that changes can be introduced by mutation into the nucleotide sequences of the IRGs described herein, thereby leading to changes in the amino acid sequence of the encoded proteins, without altering the functional activity of these proteins. For example, nucleotide substitutions leading to amino acid substitutions at "non-essential" amino acid residues can be made. A "non-essential" amino acid residue is a residue that can be altered from the wild-type sequence of a protein without altering the biological activity, whereas an "essential" amino acid residue is required for biological activity. For example, amino acid residues that are conserved among allelic variants or homologs of a gene (e.g., among homologs of a gene from different species) are predicted to be particularly unamenable to alteration.

Polynucleotides of a IRG may comprise one or more mutations. An isolated polynucleotide molecule encoding a protein with a mutation in an IRGPP described herein can be created by introducing one or more nucleotide substitutions, additions or deletions into the nucleotide sequence of the gene encoding the IRGPP, such that one or more amino acid substitutions, additions or deletions are introduced into the encoded protein. Such techniques are well known in the art. Mutations can be introduced into the IRG described herein by standard techniques, such as site-directed mutagenesis and PCR-mediated mutagenesis. Preferably, conservative amino acid substitutions are made at one or more predicted non-essential amino acid residues. Alternatively, mutations can be introduced randomly along all or part of a coding sequence of a IRG described herein, such as by saturation mutagenesis, and the resultant mutants can be screened for biological activity to identify mutants that retain activity. Following mutagenesis, the encoded protein can be expressed recombinantly and the activity of the protein can be determined.

A polynucleotide may be further modified to increase stability *in vivo*. Possible modifications include, but are not limited to, the addition of flanking sequences at the 5' and/or 3' ends; the use of phosphorothioate or 2 O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Also described are isolated polynucleotide molecules, which are antisense to the IRGs described herein. An "antisense" polynucleotide comprises a nucleotide sequence which is complementary to a "sense" polynucleotide encoding a protein, e.g., complementary to the coding strand of a double-stranded cDNA molecule or complementary to an mRNA sequence. Accordingly, an antisense polynucleotide can hydrogen bond to a sense polynucleotide. The antisense polynucleotide can be complementary to an entire coding strand of a genedescribed herein or to only a portion thereof. In one embodiment, an antisense polynucleotide molecule is antisense to a "coding region" of the coding strand of a nucleotide sequence described herein. The term "coding region" includes the region of the nucleotide sequence comprising codons which are translated into amino acids. In another embodiment, the antisense polynucleotide molecule is antisense to a "noncoding region" of the coding strand of a nucleotide sequence described herein.

Antisense polynucleotides described herein can be designed according to the rules of Watson and Crick base pairing. The antisense polynucleotide molecule can be complementary to the entire coding region of an mRNA corresponding to a gene described herein but more preferably is an oligonucleotide which is antisense to only a portion of the coding or noncoding region. An antisense oligonucleotide can be, for example, about 5, 10, 15, 20, 25, 30, 35, 40, 45 or 50 nucleotides in length. An antisense polynucleotide described herein can be constructed using chemical synthesis and enzymatic ligation reactions using procedures known in the art. For example, an antisense polynucleotide can be chemically synthesized using naturally occurring nucleotides or variously modified nucleotides designed to increase the biological stability of the molecules or to increase the physical stability of the duplex formed between the antisense and sense polynucleotides, e.g., phosphorothioate derivatives and acridine substituted nucleotides can be used. Examples of modified nucleotides which can be used to generate the antisense polynucleotide include 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-mcthylguaninc, 5-methylaminomethyluracil, 5-methoxyaminomcthyl-2-thiouracil, beta-D-mannosylqueosine, 5'-methoxycarboxymethyluracil, 5-methoxyuracil, 2-methylthio-N6-isopcntcnyladenosine, unacil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acidmethylester, 3-(3-amino-3-N-2-carboxypropyl) uracil, (acp3)w, and 2,6-diaminopurine. Alternatively, the antisense polynucleotide can be produced biologically using an expression vector into which a polynucleotide has been subcloned in an antisense orientation (i.e., RNA transcribed from the inserted polynucleotide will be of an antisense orientation to a target polynucleotide of interest, described further in the following subsection).

The antisense polynucleotide molecules described herein are typically administered to a subject or generated in situ such that they hybridize with or bind to cellular mRNA and/or genomic DNA encoding an IRGPP described herein to thereby inhibit expression of the protein, e.g., by inhibiting transcription and/or translation. The hybridization can be by conventional nucleotide complementarity to form a stable duplex or, for example, in the cases of an antisense polynucleotide molecule which binds to DNA duplexes, through specific interactions in the major groove of the double helix. An example of a route of administration of antisense polynucleotide molecules described herein include direct injection at a tissue site. Alternatively, antisense polynucleotide molecules can be modified to target selected cells and then administered systemically. For example, for systemic administration, antisense molecules can be modified such that they specifically bind to receptors or antigens expressed on a selected cell surface, e.g., by linking the antisense polynucleotide molecules to peptides or antibodies which bind to cell surface receptors or antigens. The antisense polynucleotide molecules can also be delivered to cells using the vectors described herein. To achieve sufficient intracellular concentrations of the antisense molecules, vector constructs comprising the antisense polynucleotide molecules arc preferably placed under the control of a strong promoter.

In yet another embodiment, the antisense polynucleotide molecule described herein can be an -anomeric polynucleotide molecule. An -anomeric polynucleotide molecule forms specific double-stranded hybrids with complementary RNA in which, contrary to the usual, -units, the strands run parallel to each other. The antisense polynucleotide molecule can also comprise a 2'-o-methylribonucleotide or a chimeric RNA-DNA analogue.

In still another embodiment, an antisense polynucleotidedescribed herein is a ribozyme. Ribozymes are catalytic RNA molecules with ribonuclease activity which are capable of cleaving a single-stranded polynucleotide, such as an mRNA, to which they have a complementary region. Thus, ribozymes (e.g., hammerhead ribozymes) can be used to catalytically cleave mRNA transcripts of the IRGs described herein to thereby inhibit translation of the mRNA. A ribozyme having specificity for an IRGPN can be designed based upon the nucleotide sequence of the IRGPN. Alternatively, mRNA transcribed from an IRG can be used to select a catalytic RNA having a specific ribonuclease activity from a pool of RNA molecules. Alternatively, expression of an IRG described herein can be inhibited by targeting nucleotide sequences complementary to the regulatory region of the IRG (e.g., the promoter and/or enhancers) to form triple helical structures that prevent transcription of the gene in target cells.

Expression of the IRGs described herein can also be inhibited using RNA interference ("RNAᵢ"). This is a technique for post-transcriptional gene silencing ("PTGS"), in which target gene activity is specifically abolished with cognate double-stranded RNA ("dsRNA"). RNAi involves a process in which the dsRNA is cleaved into ∼23 bp short interfering RNAs (siRNAs) by an enzyme called Dicer (Hamilton & Baulcombe, Science 286:950,1999), thus producing multiple "trigger" molecules from the original single dsRNA. The siRNA-Dicer complex recruits additional components to form an RNA-induced Silencing Complex (RISC) in which the unwound siRNA base pairs with complementary mRNA, thus guiding the RNAi machinery to the target mRNA resulting in the effective cleavage and subsequent degradation of the mRNA (Hammond ct al., Nature 404: 293-296,2000; Zamore et al., Cell 101: 25-33; 2000; Pham et al., Cell 117: 83-94,2004). In this way, the activated RISC could potentially target multiple mRNAs, and thus function catalytically.

RNAᵢ technology is disclosed, for example, in U.S. Patent No. 5,919,619 and PCT Publication Nos. WO99/14346 and WO01/29058. Typically, dsRNA of about 21 nucleotides, homologous to the target gene, is introduced into the cell and a sequence specific reduction in gene activity is observed.

In yet another embodiment, the polynucleotide molecules described herein can be modified at the base moiety, sugar moiety or phosphate backbone to improve the stability, hybridization, or solubility of the molecule. For example, the deoxyribose phosphate backbone of the polynucleotide molecules can be modified to generate peptide polynucleotides. As used herein, the terms "peptide polynucleotides" or "PNAs" refer to polynucleotide mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols.

PNAs can be used in therapeutic and diagnostic applications. For example, PNAs can be used as antisense agents for sequence-specific modulation of IRG expression by, for example, inducing transcription or translation arrest or inhibiting replication. PNAs of the polynucleotide molecules described herein can be used in the analysis of single base pair mutations in a gene, (e.g., by PNA-directed PCR clamping). They may also serve as artificial restriction enzymes when used in combination with other enzymes (e.g., S1 nucleases) or as probes or primers for DNA sequencing or hybridization.

In another embodiment, PNAs can be modified, (e.g., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of the polynucleotide molecules described herein can be generated which may combine the advantageous properties of PNA and DNA. Such chimeras allow DNA recognition enzymes, (e.g., DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation. The synthesis of PNA-DNA chimeras can be performed. For example, a DNA chain can be synthesized on a solid support using standard phosphoramidite coupling chemistry. Modified nucleoside analogs, such as 5'-(4-methoxytrityl)amino-5'-deoxy-thymidine phosphoramidite, can be used as a spacer between the PNA and the 5' end of DNA. PNA monomers are then coupled in a stepwise manner to produce a chimeric molecule with a 5' PNA segment and a 3' DNA segment. Alternatively, chimeric molecules can be synthesized with a 5' DNA segment and a 3' PNA segment.

In other embodiments, the oligonucleotide may include other appended groups such as peptides (e.g., for targeting host cell receptors *in vivo*), or agents facilitating transport across the cell membrane or the blood-kidney barrier (see, e.g. PCT Publication No. WO 89/10134). In addition, oligonucleotides can be modified with hybridization-triggered cleavage agents or intercalating agents. To this end, the oligonucleotide may be conjugated to another molecule (e.g., a peptide, hybridization triggered cross-linking agent, transport agent, or hybridization-triggered cleavage agent). Finally, the oligonucleotide may be detectably labeled, either such that the label is detected by the addition of another reagent (e.g., a substrate for an enzymatic label), or is detectable immediately upon hybridization of the nucleotide (e.g., a radioactive label or a fluorescent label).

### Isolated Polypeptides

Described herein are isolated IRGPPs, and biologically active portions thereof, as well as polypeptide fragments suitable for use as immunogens to raise anti-IRGPP antibodies. In one embodiment, native IRGPPs can be isolated from cells or tissue sources by an appropriate purification scheme using standard protein purification techniques. Standard purification methods include electrophoretic, molecular, immunological and chromatographic techniques, including ion exchange, hydrophobic, affinity, and reverse-phase HPLC chromatography, and chromatofocusing. For example, an IRGPP may be purified using a standard anti-IRGPP antibody column. Ultrafiltration and diafiltration techniques, in conjunction with protein concentration, are also useful. The degree of purification necessary will vary depending on the use of the IRGPP. In some instances no purification will be necessary.

In another embodiment, IRGPPs or mutated IRGPPs are produced by recombinant DNA techniques.. Alternative to recombinant expression, an IRGPP or mutated IRGPP can be synthesized chemically using standard peptide synthesis techniques.

Also described are variants of IRGPPs. The variant of an IRGPP is substantially homologous to the native IRGPP encoded by an IRG listed in Table 3, and retains the functional activity of the native IRGPP, yet differs in amino acid sequence due to natural allelic variation or mutagenesis, as described in detail above. Accordingly, in another embodiment, the variant of an IRGPP is a protein which comprises an amino acid sequence at least about 60%, 65%, 70%, 75%, 80%, 85%, 90%, 95%, 98% or more homologous to the amino acid sequence of the original IRGPP.

In a non-limiting example, as used herein, proteins are referred to as "homologs" and "homologous" where a first protein region and a second protein region are compared in terms of identity. To determine the percent identity of two amino acid sequences or of two polynucleotide sequences, the sequences are aligned for optimal comparison purposes (e.g., gaps can be introduced in one or both of a first and a second amino acid or polynucleotide sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleotide "identity" is equivalent to amino acid or nucleotide "homology"). The percent identity between the two sequences is a function of the number of identical positions shared by the sequences, taking into account the number of gaps and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (J. Mol. Biol. 48:444-453, 1970) algorithm which has been incorporated into the GAP program in the GCG software package, using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using a NWSgapdna.CMP matrix and a gap weight of 40, 50, 60, 70, or 80 and a length weight of 1, 2, 3, 4, 5, or 6.

The polynucleotide and protein sequences described herein can be further used as a "query sequence" to perform a search against public databases to, for example, identify other family members or related sequences. Such searches can be performed using BLAST programs available at the BLAST website maintained by the National Center of biotechnology Information (NCBI), National Library of Medicine, Washington DC. USA.

Also described are chimeric or fusion IRGPPs. Within a fusion IRGPP the polypeptide can correspond to all or a portion of an IRGPP. In a preferred embodiment, a fusion IRGPP comprises at least one biologically active portion of an IRGPP. Within the fusion protein, the term "operatively linked" is intended to indicate that the IRGPP-related polypeptide and the non-IRGPP-related polypeptide are fused in-frame to each other. The non-IRGPP-related polypeptide can be fused to the N-terminus or C-terminus of the IRGPP-related polypeptide.

A peptide linker sequence may be employed to separate the IRGPP-related polypeptide from non-IRGPP-related polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors:
(1) their ability to adopt a flexible extended conformation; (2) their inability to adopt a secondary structure that could interact with functional epitopes on the IRGPP-related polypeptide and non-IRGPP-related polypeptide; and (3) the lack of hydrophobic or charged residues that might react with the polypeptide functional epitopes. Preferred peptide linker sequences contain gly, asn and ser residues. Other near neutral amino acids, such as thr and ala may also be used in the linker sequence. Amino acid sequences which may be used as linkers are well known in the art. The linker sequence may generally be from 1 to about 50 amino acids in length. Linker sequences are not required when the IRGPP-related polypeptide and non-IRGPP-related polypeptide have non-essential N-terminal amino acid regions that can be used to separate the functional domains and prevent steric interference.

For example, in one embodiment, the fusion protein is a glutathione S-transferase (GST)-IRGPP fusion protein in which the IRGPP sequences are fused to the C-terminus of the GST sequences. Such fusion proteins can facilitate the purification of recombinant IRGPPs.

The IRGPP-fusion proteins described herein can be incorporated into pharmaceutical compositions and administered to a subject *in vivo*, as described herein. The IRGPP-fusion proteins can be used to affect the bioavailability of an IRGPP substrate. IRGPP-fusion proteins may be useful therapeutically for the treatment of, or prevention of, damages caused by, for example, (i) aberrant modification or mutation of an IRG; (ii) mis-regulation of an IRG; and (iii) aberrant post-translational modification of,an IRGPP.

Moreover, the IRGPP-fusion proteins described herein can be used as immunogens to produce anti-IRGPP antibodies in a subject, to purify IRGPP ligands, and to identify molecules which inhibit the interaction of an IRGPP with an IRGPP substrate in screening assays.

Preferably, an IRGPP-chimeric or fusion protein described herein is produced by standard recombinant DNA techniques. For example, DNA fragments coding for the different polypeptide sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence. Moreover, many expression vectors are commercially available that already encode a fusion moiety (e.g., a GST polypeptide). An IRGPP-encoding polynucleotide can be cloned into such an expression vector such that the fusion moiety is linked in-frame to the IRGPP.

A signal sequence can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the disclosure pertains to the described polypeptides having a signal sequence, as well as to polypeptides from which the signal sequence has been proteolytically cleaved (i.e., the cleavage products).

In one embodiment, a polynucleotide sequence encoding a signal sequence can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a GST domain.

The present disclosure also pertains to variants of the IRGPPs described herein which function as either agonists or as antagonists to the IRGPPs. In one embodiment, antagonists or agonists of IRGPPs are used as therapeutic agents. For example, antagonists of an up-regulated IRG that can decrease the activity or expression of such a gene and therefore ameliorate influenza in a subject wherein the IRG is abnormally increased in level or activity. In this embodiment, treatment of such a subject may comprise administering an antagonist wherein the antagonist provides decreased activity or expression of the targeted IRG.

In certain embodiments, an agonist of the IRGPPs can retain substantially the same, or a subset, of the biological activities of the naturally occurring form of an IRGPP or may enhance an activity of an IRGPP. In certain embodiments, an antagonist of an IRGPP can inhibit one or more of the activities of the naturally occurring form of the IRGPP by, for example, competitively modulating an activity of an IRGPP. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the IRGPP.

Mutants of an IRGPP which function as either IRGPP agonists or as IRGPP antagonists can be identified by screening combinatorial libraries of mutants, e.g., truncation mutants, of an IRGPP for IRGPP agonist or antagonist activity. In certain embodiments, such mutants may be used, for example, as a therapeutic protein as described herein. A diverse library of IRGPP mutants can be produced by, for example, enzymatically ligating a mixture of synthetic oligonucleotides into gene sequences such that a degenerate set of potential IRGPP sequences is expressible as individual polypeptides, or alternatively, as a set of larger fusion proteins (e.g., for phage display) containing the set of IRGPP sequences therein. There arc a variety of methods which can be used to produce libraries of potential IRGPP variants from a degenerate oligonucleotide sequence. Chemical synthesis of a degenerate gene sequence can be performed in an automatic DNA synthesizer, and the synthetic gene is then ligated into an appropriate expression vector. Use of a degenerate set of genes allows for the provision, in one mixture, of all of the sequences encoding the desired set of potential IRGPP sequences. Methods for synthesizing degenerate oligonucleotides are known in the art.

In addition, libraries of fragments of a protein coding sequence corresponding to an IRGPP as described herein can be used to generate a diverse or heterogenous population of IRGPP fragments for screening and subsequent selection of variants of an IRGPP. in one embodiment, a library of coding sequence fragments can be generated by treating a double-stranded PCR fragment of an IRGPP coding sequence with a nuclease under conditions wherein nicking occurs only about once per molecule, denaturing the double-stranded DNA, renaturing the DNA to form double-stranded DNA which can include sense/antisense pairs from different nicked products, removing single-stranded portions from reformed duplexes by treatment with S1 nuclease, and ligating the resulting fragment library into an expression vector. By this method, an expression library can be derived which encodes N-terminal, C-terminal and internal fragments of various sizes of the IRGPP.

Several techniques are known in the art for screening gene products of combinatorial libraries made by point mutations or truncation, and for screening cDNA libraries for gene products having a selected property. The most widely used techniques, which are amenable to high-throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify IRGPP variants (Delgrave et al. Protein Engineering 6:327-331, 1993).

Portions of an IRGPP or variants of an IRGPP having less than about 100 amino acids, and generally less than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. Equipment for automated synthesis of polypeptides is commercially available from suppliers such as Perkin Elmer/Applied BioSystems Division (Foster City, CA), and may be operated according to the manufacturer's instructions.

Methods and compositions for screening for protein inhibitors or activators are known in the art (see U.S. Patent Nos. 4,980,281, 5,266,464, 5,688,635, and 5,877,007).

It is contemplated in the present invention that IRGPPs are cleaved into fragments for use in further structural or functional analysis, or in the generation of reagents such as IRGPP and IRGPP-specific antibodies. This can be accomplished by treating purified or unpurified polypeptide with a proteolytic enzyme (i.e., a proteinase) including, but not limited to, serine proteinases (e.g., chymotrypsin, trypsin, plasmin, elastase, thrombin, substilin) metal proteinases (e.g., carboxypeptidase A, carboxypeptidase B, leucine aminopeptidase, thermolysin, collagenase), thiolproteinases (e.g., papain, bromelain, Streptococcal proteinase, clostripain) and/or acid proteinases (e.g., pepsin, gastricsin, trypsinogen). Polypeptide fragments are also generated using chemical means such as treatment of the polypeptide with cyanogen bromide (CNBr), 2-nitro-5-thiocyanobenzoic acid, isobenzoic acid, BNPA-skatole, hydroxylamine or a dilute acid solution. Recombinant techniques are also used to produce specific fragments of an IRGPP.

In addition, the invention also contemplates that compounds sterically similar to a particular IRGPP may be formulated to mimic the key portions of the peptide structure, called peptidomimetics or peptide mimetics. Mimetics are peptide-containing molecules which mimic elements of polypeptide secondary structure. See, for example, U.S. Patent No. 5,817,879. The underlying rationale behind the use of peptide mimetics is that the peptide backbone of polypeptides exists chiefly to orient amino acid side chains in such a way as to facilitate molecular interactions, such as those of receptor and ligand. Recently, peptide and glycoprotein mimetic antigens have been described which elicit protective antibody to Neisseria meningitidis serogroup B, thereby demonstrating the utility of mimetic applications (Moe ct al., Int. Rev. Immunol. 20:201-20, 2001; Berezin et al., J MolNeurosci. 22:33-39, 2004). Successful applications of the peptide mimetic concept have thus far focused on mimetics of b-turns within polypeptides. Likely b-turn structures within an IRGPP can be predicted by computer-based algorithms. For example, U.S. Patent No. 5,933,819 describes a neural network based method and system for identifying relative peptide binding motifs from limited experimental data. In particular, an artificial neural network (ANN) is trained with peptides with known sequence and function (i.e., binding strength) identified from a phage display library. The ANN is then challenged with unknown peptides, and predicts relative binding motifs. Analysis of the unknown peptides validate the predictive capability of the ANN. Once the component amino acids of the turn are determined, mimetic can be constructed to achieve a similar spatial orientation of the essential elements of the amino acid side chains, as discussed in U.S. Patent No. 6,420119 and U.S. Patent No. 5,817,879, and in Kyte and Doolittle, J. Mol. Biol., 157:105-132, 1982; Moe and Granoff, Int. Rev. Immunol., 20:201-20, 2001; and Granoff et al., J. Immunol., 167:6487-96, 2001.

### Antibodies

In another aspect, the invention includes an antibody which binds to a polypeptide comprising the amino acid sequence of SEQ ID NO: 17 (PTCH) for use in a method for enhancing the resistance of a mammal to infection by an influenza virus or a method to treat influenza virus infection in a mammal. Also described are antibodies that a specific to IRGPPs described herein or their variants. Preferably the antibodies are monoclonal, and most preferably, the antibodies are humanized, as per the description of antibodies described below.

An isolated IRGPP, or a portion or fragment thereof, can be used as an immunogen to generate antibodies that bind the IRGPP using standard techniques for polyclonal and monoclonal antibody preparation. A full-length IRGPP can be used or, alternatively, the disclosure provides antigenic peptide fragments of the IRGPP for use as immunogens. The antigenic peptide of an IRGPP comprises at least 8 amino acid residues of an amino acid sequence encoded by an IRG set forth in Table 3 or an homolog thereof, and encompasses an epitope of an IRGPP such that an antibody raised against the peptide forms a specific immune complex with the IRGPP. Preferably, the antigenic peptide comprises at least 8 amino acid residues, more preferably at least 12 amino acid residues, even more preferably at least 16 amino acid residues, and most preferably at least 20 amino acid residues.

Immunogenic portions (epitopes) may generally be identified using well known techniques. Such techniques include screening polypeptides for the ability to react with antigen-specific antibodies, antisera and/or T-cell lines or clones. As used herein, antisera and antibodies are "antigen-specific" if they bind to an antigen with a binding affinity equal to, or greater than 10⁵ M⁻¹. Such antisera and antibodies may be prepared as described herein, and using well known techniques. An epitope of an IRGPP is a portion that reacts with such antisera and/or T-cells at a level that is not substantially less than the reactivity of the full length polypeptide (e.g., in an ELISA and/or T-cell reactivity assay). Such epitopes may react within such assays at a level that is similar to or greater than the reactivity of the full length polypeptide. Such screens may generally be performed using methods well known to those of ordinary skill in the art. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

Preferred epitopes encompassed by the antigenic peptide are regions of the IRGPP that are located on the surface of the protein, e.g., hydrophilic regions, as well as regions with high antigenicity.

An IRGPP immunogen typically is used to prepare antibodies by immunizing a suitable non-human subject, (e.g., rabbit, goat, mouse or other mammal) with the immunogen. An appropriate immunogenic preparation can contain, for example, recombinantly expressed IRGPP or a chemically synthesized IRGPP. The preparation can further include an adjuvant, such as Freund's complete or incomplete adjuvant, or a similar immunostimulatory agent. Immunization of a suitable non-human subject with an immunogenic IRGPP preparation induces a polyclonal anti-IRGPP antibody response. Techniques for preparing, isolating and using antibodies are well known in the art.

Accordingly, another aspect of the invention pertains to monoclonal or polyclonal anti-IRGPP antibodies and immunologically active portions of the antibody molecules, including F(ab) and F(ab')₂ fragments which can be generated by treating the antibody with an enzyme such as pepsin.

Polyclonal anti-IRGPP antibodies can be prepared as described above by immunizing a suitable non-human subject with an IRGPP. The anti-IRGPP antibody titer in the immunized subject can be monitored over time by standard techniques, such as with an enzyme linked immunosorbent assay (ELISA) using immobilized IRGPP. If desired, the antibody molecules directed against IRGPPs can be isolated from the non-human subject (e.g., from the blood) and further purified by well known techniques, such as protein A chromatography, to obtain the IgG fraction. At an appropriate time after immunization, e.g., when the anti-IRGPP antibody titers are highest, antibody-producing cells can be obtained from the non-human subject and used to prepare monoclonal antibodies by standard techniques, such as the hybridoma technique, human B cell hybridoma technique, the EBV-hybridoma technique, or trioma techniques. The technology for producing monoclonal antibody hybridomas is well known. Briefly, an immortal cell line (typically a myeloma) is fused to lymphocytes (typically splenocytes) from a mammal immunized with an IRGPP immunogen as described above, and the culture supernatants of the resulting hybridoma cells are screened to identify a hybridoma producing a monoclonal antibody that binds to an IRGPP as described herein. Any of the many well known protocols used for fusing lymphocytes and immortalized cell lines can be applied for the purpose of generating an anti-IRGPP monoclonal antibody. Moreover, the ordinarily skilled worker will appreciate that there are many variations of such methods which also would be useful.

Alternative to preparing monoclonal antibody-secreting hybridomas, a monoclonal anti-IRGPP antibody can be identified and isolated by screening a recombinant combinatorial immunoglobulin library (e.g., an antibody phase display library) with IRGPP to thereby isolate immunoglobulin library members that bind to an IRGPP. Kits for generating and screening phage display libraries are commercially available.

The anti-IRGPP antibodies also include "Single-chain Fv" or "scFv" antibody fragments. The scFv fragments comprise the V_{H} and V_{L} domains of antibody, wherein these domains are present in a single polypeptide chain. Generally, the Fv polypeptide further comprises a polypeptide linker between the V_{H} and V_{L} domains which enables the scFv to form the desired structure for antigen binding.

Additionally, recombinant anti-IRGPP antibodies, such as chimeric and humanized monoclonal antibodies, comprising both human and non-human portions, which can be made using standard recombinant DNA techniques, are described herein and such antibodies which bind to a polypeptide comprising the amino acid sequences of SEQ ID NO: 17 (PTCH) are within the scope of the invention. Such chimeric and humanized monoclonal antibodies can be produced by recombinant DNA techniques known in the art (see e.g., U.S. Patent Nos. 6,677,436 and 6,808,901).

Humanized antibodies are particularly desirable for therapeutic treatment of human subjects. Humanized forms of non-human (e.g., murine) antibodies are chimeric molecules of immunoglobulins, immunoglobulin chains or fragments thereof (such as Fv, Fab, Fab', F(ab')₂ or other antigen-binding subsequences of antibodies), which contain minimal sequence derived from non-human immunoglobulin. Humanized antibodies include human immunoglobulins (recipient antibody) in which residues forming a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the constant regions being those of a human immunoglobulin consensus sequence. The humanized antibody will preferably also comprise at least a portion of an immunoglobulin constant region (Fc), typically that of a human immunoglobulin.

Such humanized antibodies can be produced using transgenic mice which are incapable of expressing endogenous immunoglobulin heavy and light chain genes, but which can express human heavy and light chain genes. The transgenic mice arc immunized in the normal fashion with a selected antigen, e.g., all or a portion of a polypeptide corresponding to an IRGPP as described herein. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies.

Humanized antibodies which recognize a selected epitope can be generated using a technique referred to as "guided selection." In this approach a selected non-human monoclonal antibody, e.g., a murine antibody, is used to guide the selection of a humanized antibody recognizing the same epitope.

In a preferred embodiment, the antibodies to TRGPP are capable of reducing or eliminating the biological function of IRGPP, as is described below. That is, the addition of anti-IRGPP antibodies (either polyclonal or preferably monoclonal) to IRGPP (or cells containing IRGPP) may reduce or eliminate the IRGPP activity. Generally, at least a 25% decrease in activity is preferred, with at least about 50% being particularly preferred and about a 95-100% decrease being especially preferred.

An anti-IRGPP antibody can be used to isolate an IRGPP as described herein by standard techniques, such as affinity chromatography or immunoprecipitation. An anti-IRGPP antibody can facilitate the purification of natural IRGPPs from cells and of recombinantly produced IRGPPs expressed in host cells. Moreover, an anti-IRGPP antibody can be used to detect an IRGPP (e.g., in a cellular lysate or cell supernatant on the cell surface) in order to evaluate the abundance and pattern of expression of the IRGPP. Anti-IRGPP antibodies can be used diagnostically to monitor protein levels in tissue as part of a clinical testing procedure, for example, to determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive materials include ¹²⁵I, ¹³¹I, ³⁵S or ³H.

Anti-IRGPP antibodies as described herein, such as the antibody of the invention, which binds to a polypeptide comprising the amino acid sequence of SEQ ID NO: 17 (PTCH), are also useful for targeting a therapeutic to a cell or tissue comprising the antigen of the anti-IRGPP antibody. A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

As is well known in the art, a given polypeptide or polynucleotide may vary in its immunogenicity. It is often necessary therefore to couple the immunogen (e.g., a polypeptide or polynucleotide) as described herein with a carrier. Exemplary and preferred carriers are CRM197, E coli (LT) toxin, V. cholera (CT) toxin, keyhole limpet hemocyanin (KLH) and bovine serum albumin (BSA). Other albumins such as ovalbumin, mouse serum albumin or rabbit serum albumin can also be used as carriers.

Where an IRGPP (or a fragment thereof) and a carrier protein are conjugated (i.e., covalently associated), conjugation may be any chemical method, process or genetic technique commonly used in the art. For example, an IRGPP (or a fragment thereof) and a carrier protein, may be conjugated by techniques, including, but not limited to: (1) direct coupling via protein functional groups (e.g., thiol-thiol linkage, amine-carboxyl linkage, amine-aldehyde linkage; enzyme direct coupling); (2) homobifunctional coupling of amines (e.g., using bis-aldehydes); (3) homobifunctional coupling of thiols (e.g., using bismaleimides); (4) homobifunctional coupling via photoactivated reagents (5) heterobifunctional coupling of amines to thiols (e.g., using maleimides); (6) heterobifunctional coupling via photoactivated reagents (e.g., the-carbonyldiazo family); (7) introducing amine-reactive groups into a poly- or oligosaccharide via cyanogen bromide activation or carboxymethylation; (8) introducing thiol-reactive groups into a poly- or oligosaccharide via a heterobifunctional compound such as maleimido-hydrazide; (9) protein-lipid conjugation via introducing a hydrophobic group into the protein and (10) protein-lipid conjugation via incorporating a reactive group into the lipid. Also, contemplated are heterobifunctional "non-covalent coupling" techniques such the Biotin-Avidin interaction. For a comprehensive review of conjugation techniques, see Aslam and Dent (Aslam and Dent, "Bioconjugation: Protein Coupling Techniques for the Biomedical Sciences," Macmillan Reference Ltd., London, England, 1998).

In a specific embodiment, antibodies to an IRGPP may be used to eliminate the IRGPP *in vivo* by activating the complement system or mediating antibody-dependent cellular cytotoxicity (ADCC), or cause uptake of the antibody coated cells by the receptor-mediated endocytosis (RE) system.

### Vectors

Also described herein are vectors containing a polynucleotide encoding an IRGPP, a variant of an IRGPP, or a portion thereof. One type of vector is a "plasmid," which includes a circular double-stranded DNA loop into which additional DNA segments can be ligated. In the present specification, "plasmid" and "vector" can be used interchangeably as the plasmid is the most commonly used form of vector. Vectors also include expression vectors and gene delivery vectors.

The expression vectors described herein comprise a polynucleotide encoding an IRGPP or a portion thereof in a form suitable for expression of the polynucleotide in a host cell, which means that the expression vectors include one or more regulatory sequences, selected on the basis of the host cells to be used for expression, and operatively linked to the polynucleotide sequence to be expressed. It will be appreciated by those skilled in the art that the design of the expression vector can depend on such factors as the choice of the host cell to be transformed and the level of expression of protein desired. The expression vectors described herein can be introduced into host cells to thereby produce proteins or peptides, such as IRGPPs, mutant forms of IRGPPs and IRGPP-fusion proteins.

The expression vectors described herein can be designed for expression of IRGPPs in prokaryotic or eukaryotic cells. For example, IRGPPs can be expressed in bacterial cells such as E. coli, insect cells (using baculovirus expression vectors), yeast cells or mammalian cells. Alternatively, the expression vector can be transcribed and translated *in vitro*, for example using T7 promoter regulatory sequences and T7 polymerase.

The expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of the recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Purified fusion proteins can be utilized in IRGPP activity assays, (e.g., direct assays or competitive assays described in detail below), or to generate antibodies specific for IRGPPs.

One strategy to maximize recombinant protein expression in E. coli is to express the protein in a host bacteria with an impaired capacity to proteolytically cleave the recombinant protein. Another strategy is to alter the polynucleotide sequence of the polynucleotide to be inserted into an expression vector so that the individual codons for each amino acid are those preferentially utilized in E. coli. Such alteration of polynucleotide sequences described herein can be carried out by standard DNA synthesis techniques.

In another embodiment, the IRGPP expression vector is a yeast expression vector. Examples of vectors for expression in yeast S. cerevisiae include pYepSec1, pMFa, pJRY88, pYES2 and picZ (Invitrogen Corp, San Diego, CA).

Alternatively, IRGPPs described herein can be expressed in insect cells using baculovirus expression vectors. Baculovirus vectors available for expression of proteins in cultured insect cells (e.g., Sf9 cells) include the pAc series and the pVL series.

In yet another embodiment a polynucleotide described herein is expressed in mammalian cells using a mammalian expression vector. When used in mammalian cells, the expression vector's control functions are often provided by viral regulatory elements. For example, commonly used promoters are derived from polyoma, adenovirus 2 and 5, cytomegalovirus and Simian Virus 40.

In another embodiment, the mammalian expression vector is capable of directing expression of the polynucleotide preferentially in a particular cell type (e.g., tissue-specific regulatory elements are used to express the polynucleotide). Tissue-specific regulatory elements are known in the art and may include epithelial cell-specific promoters. Other nonlimiting examples of suitable tissue-specific promoters include the liver-specific promoter (e.g., albumin promoter), lymphoid-specific promoters, promoters of T cell receptors and immunoglobulins, neuron-specific promoters (e.g., the neurofilament promoter), pancreas-specific promoters (e.g., insulin promoter), and mammary gland-specific promoters (e.g., milk whey promoter). Developmentally-regulated promoters (e.g., the -fetoprotein promoter) are also encompassed.

Also described herein is a recombinant expression vector comprising a polynucleotide encoding an IRGPP cloned into the expression vector in an antisense orientation. That is, the DNA molecule is operatively linked to a regulatory sequence in a manner which allows for expression (by transcription of the DNA molecule) of an RNA molecule which is antisense to mRNA corresponding to an IRG described herein. Regulatory sequences operatively linked to a polynucleotide cloned in the antisense orientation can be chosen which direct the continuous expression of the antisense RNA molecule in a variety of cell types, for instance, viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense polynucleotides arc produced under the control of a high efficiency regulatory region, the activity of which can be determined by the cell type into which the vector is introduced.

Also described herein are gene delivery vehicles for delivery of polynucleotides to cells, tissues, or a mammal for expression. For example, a polynucleotide sequence described herein can be administered either locally or systemically in a gene delivery vehicle. These constructs can utilize viral or non-viral vector approaches in *in vivo* or *ex vivo* modality. Expression of the coding sequence can be induced using endogenous mammalian or heterologous promoters. Expression of the coding sequence *in vivo* can be either constituted or regulated. Also described are gene delivery vehicles capable of expressing the contemplated polynucleotides. The gene delivery vehicle is preferably a viral vector and, more preferably, a retroviral, lentiviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, togavirus viral vector.

The delivery of gene therapy constructs described herein into cells is not limited to the above mentioned viral vectors. Other delivery methods and media may be employed such as, for example, nucleic acid expression vectors, polycationic condensed DNA linked or unlinked to killed adenovirus alone, ligand linked DNA, liposomes, eukaryotic cell delivery vehicles cells, deposition of photopolymerized hydrogel materials, handheld gene transfer particle gun, ionizing radiation, nucleic charge neutralization or fusion with cell membranes. Particle mediated gene transfer may be employed. Briefly, DNA sequence can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose or transferrin. Naked DNA may also be employed.

Also described is expression of IRGPPs using a regulatable expression system. Examples of regulatable systems include the Tct-on/off system of BD Biosciences (San Jose, CA), the ecdysone system of Invitrogen (Carlsbad, CA, the mifepristone/progesterone system of Valentis (Burlingame, CA), and the rapamycin system of Ariad (Cambridge, MA).

### Immunogens and Immunogenic Compositions

Within certain aspects, IRGPP, IRGPN, IRGPP-specific T cell, IRGPP-presenting APC, IRG-containing vectors, including but are not limited to expression vectors and gene delivery vectors, may be utilized as vaccines for influenza. Vaccines may comprise one or more such compounds/cells and an immunostimulant. An immunostimulant may be any substance that enhances or potentiates an immune response (antibody and/or cell-mediated) to an exogenous antigen. Examples of immunostimulants include adjuvants, biodegradable microspheres (e.g., polylactic galactide) and liposomes (into which the compound is incorporated). Vaccines described herein may also contain other compounds, which may be biologically active or inactive. For example, one or more immunogenic portions of other antigens may be present, either incorporated into a fusion polypeptide or as a separate compound, within the composition of vaccine.

A vaccine may contain DNA encoding one or more IRGPP or portion of IRGPP, such that the polypeptide is generated *in situ.* As noted above, the DNA may be present within any of a variety of delivery systems known to those of ordinary skill in the art, including nucleic acid expression vectors, gene delivery vectors, and bacteria expression systems. Numerous gene delivery techniques are well known in the art. Appropriate nucleic acid expression systems contain the necessary DNA sequences for expression in the patient (such as a suitable promoter and terminating signal). Bacterial delivery systems involve the administration of a bacterium (such as Bacillus-Calmette-Guerrin) that expresses an immunogenic portion of the polypeptide on its cell surface or secretes such an epitope. The DNA may be introduced using a viral expression system (e.g., vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993.

It will be apparent that a vaccine may contain pharmaceutically acceptable salts of the polynucleotides and polypeptides provided herein. Such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

Any of a variety of immunostimulants may be employed in the vaccines described herein.

For example, an adjuvant may be included. As defined previously, an "adjuvant" is a substance that serves to enhance the immunogenicity of an antigen. Thus, adjuvants are often given to boost the immune response and are well known to the skilled artisan. Examples of adjuvants contemplated include, but are not limited to, aluminum salts (alum) such as aluminum phosphate and aluminum hydroxide, Mycobacterium tuberculosis, Bordetella pertussis, bacterial lipopolysaccharides, aminoalkyl glucosamine phosphate compounds (AGP), or derivatives or analogs thereof, which are available from Corixa (Hamilton, MT), and which are described in U.S. Patent Number 6,113,918; one such AGP is 2-[(R)-3-Tetradecanoyloxytetradecanoylamino]ethyl 2-Deoxy-4-O-phosphono-3-O-[(R)-3-tetradecanoyoxytetradecanoyl]-2-[(R)-3-tetradecanoyoxytetradecanoylamino]-b-D-glucopyranoside, which is also known as 529 (formerly known as RC529), which is formulated as an aqueous form or as a stable emulsion, MPL™ (3-O-deacylatcd monophosphoryl lipid A) (Corixa) described in U.S. Patent No. 4,912,094, synthetic polynucleotides such as oligonucleotides containing a CpG motif (U.S. Patent No. 6,207,646), polypeptides, saponins such as Quil A or STIMULON™ QS-21 (Antigenics, Framingham, Massachusetts), described in U.S. Patent No. 5,057,540, a pertussis toxin (PT), or an E. coli heat-labile toxin (LT), particularly LT-K63, LT-R72, CT-S109, PT-K9/G129; see, e.g., International Patent Publication Nos. WO 93/13302 and WO 92/19265, cholera toxin (either in a wild-type or mutant form, e.g., wherein the glutamic acid at amino acid position 29 is replaced by another amino acid, preferably a histidine, in accordance with published International Patent Application number WO 00/18434). Various cytokines and lymphokines are suitable for use as adjuvants. One such adjuvant is granulocyte-macrophage colony stimulating factor (GM-CSF), which has a nucleotide sequence as described in U.S. Patent No. 5,078,996. A plasmid containing GM-CSF cDNA has been transformed into E. coli and has been deposited with the American Type Culture Collection (ATCC), 1081 University Boulevard, Manassas, VA 20110-2209, under Accession Number 39900. The cytokine IL-12 is another adjuvant which is described in U.S. Patent Number 5,723,127. Other cytokines or lymphokines have been shown to have immune modulating activity, including, but not limited to, the interleukins 1-alpha, 1-beta, 2, 4, 5,6, 7, 8, 10, 13, 14, 15, 16, 17 and 18, the interferons-alpha, beta and gamma, granulocyte colony stimulating factor, and the tumor necrosis factors alpha and beta, and are suitable for use as adjuvants.

Any vaccine provided herein may be prepared using well known methods that result in a combination of antigen, immune response enhancer and a suitable carrier or excipient. The compositions described herein may be administered as part of a sustained release formulation (i.e., a formulation such as a capsule, sponge or gel (composed of polysaccharides, for example) that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane.

Carriers for use within such formulations are biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core (e.g., a cross-linked polysaccharide or oligosaccharide) and, optionally, an external layer comprising an amphiphilic compound, such as a phospholipid (see e.g., U.S. Patent No. 5,151,254 and PCT applications WO 94/20078, WO 94/23701 and WO 96/06638). The amount of active compound contained within a sustained release formulation depends upon the site of implantation, the rate and expected duration of release and the nature of the condition to be treated or prevented.

Any of a variety of delivery vehicles may be employed within vaccines to facilitate production of an antigen-specific immune response that targets cancer cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-influenza effects per se and/or to be immunologically compatible with the receiver (i.e., matched HLA haplotype). APCs may generally be isolated from any of a variety of biological fluids and organs, and may be autologous, allogeneic, syngeneic or xenogenic cells.

Vaccines may be presented in unit-dose or multi-dose containers, such as sealed ampoules or vials. Such containers are preferably hermetically scaled to preserve sterility of the formulation until use. In general, formulations may be stored as suspensions, solutions or emulsions in oily or aqueous vehicles. Alternatively, a vaccine may be stored in a freezedricd condition requiring only the addition of a sterile liquid carrier immediately prior to use.

### Screening: Methods

Also described are methods (also referred to herein as "screening assays") for identifying modulators, i.e., candidate or test compounds or agents comprising therapeutic moieties (e.g., peptides, peptidomimetics, peptoids, polynucleotides, small molecules or other drugs) which (a) bind to an IRGPP, or (b) have a modulatory (e.g., stimulatory or inhibitory) effect on the activity of an IRGPP or, more specifically, (c) have a modulatory effect on the interactions of the IRGPP with one or more of its natural substrates (e.g., peptide, protein, hormone, co-factor, or polynucleotide), or (d) have a modulatory effect on the expression of the IRGPPs. Such assays typically comprise a reaction between the IRGPP and one or more assay components. The other components may be either the test compound itself, or a combination of the test compound and a binding partner of the IRGPP.

To screen for compounds which interfere with binding of two proteins e.g., an IRGPP and its binding partner, a Scintillation Proximity Assay can be used. In this assay, the IRGPP is labeled with an isotope such as ¹²⁵I. The binding partner is labeled with a scintillant, which emits light when proximal to radioactive decay (i.e., when the IRGPP is bound to its binding partner). A reduction in light emission will indicate that a compound has interfered with the binding of the two proteins.

Alternatively a Fluorescence Energy Transfer (FRET) assay could be used. In a FRET assay as described herein, a fluorescence energy donor is comprised on one protein (e.g., an IRGPP) and a fluorescence energy acceptor is comprised on a second protein (e.g., a binding partner of the IRGPP). If the absorption spectrum of the acceptor molecule overlaps with the emission spectrum of the donor fluorophore, the fluorescent light emitted by the donor is absorbed by the acceptor. The donor molecule can be a fluorescent residue on the protein (e.g., intrinsic fluorescence such as a tryptophan or tyrosine residue), or a fluorophore which is covalcntly conjugated to the protein (e.g., fluorescein isothiocyanate, FITC). An appropriate donor molecule is then selected with the above acceptor/donor spectral requirements in mind.

Thus, in this example, an IRGPP is labeled with a fluorescent molecule (i.e., a donor fluorophore) and its binding partner is labeled with a quenching molecule (i.e., an acceptor). When the IRGPP and its binding partner are bound, fluorescence emission will be quenched or reduced relative the IRGPP alone. Similarly, a compound which can dissociate the interaction of the IRGPP-partner complex, will result in an increase in fluorescence emission, which indicates the compound has interfered with the binding of the IRGPP to its binding partner.

Another assay to detect binding or dissociation of two proteins is fluorescence polarization or anisotropy. In this assay, the investigated protein (e.g., an IRGPP) is labeled with a fluorophore with an appropriate fluorescence lifetime. The protein sample is then excited with vertically polarized light. The value of anisotropy is then calculated by determining the intensity of the horizontally and vertically polarized emission light. Next, the labeled protein (IRGPP) is mixed with an IRGPP binding partner and the anisotropy measured again. Because fluorescence anisotropy intensity is related to the rotational freedom of the labeled protein, the more rapidly a protein rotates in solution, the smaller the anisotropy value. Thus, if the labeled IRGPP is part of a complex (e.g., IRGPP-partner), the IRGPP rotates more slowly in solution (relative to free, unbound IRGPP) and the anisotropy intensity increases. Subsequently, a compound which can dissociate the interaction of the IRGPP-partner complex, will result in a decrease in anisotropy (i.e., the labeled IRGPP rotates more rapidly), which indicates the compound has interfered with the binding of IRGPP to its binding partner.

A more traditional assay would involve labeling the IRGPP binding partner with an isotope such as ¹²⁵I, incubating with the IRGPP, then immunoprecipitating of the IRGPP. Compounds that increase the free IRGPP will decrease the precipitated counts. To avoid using radioactivity, the IRGPP binding partner could be labeled with an enzyme-conjugated antibody instead.

Alternatively, the IRGPP binding partner could be immobilized on the surface of an assay plate and the IRGPP could be labeled with a radioactive tag. A rise in the number of counts would identify compounds that had interfered with binding of the IRGPP and its binding partner.

Evaluation of binding interactions may further be performed using Biacore technology, wherein the IRGPP or its binding partner is bound to a micro chip, either directly by chemical modification or tethered via antibody-epitope association (e.g., antibody to the IRGPP), antibody directed to an epitope tag (e.g., His tagged) or fusion protein (e.g., GST). A second protein or proteins is/are then applied via flow over the "chip" and the change in signal is detected. Finally, test compounds are applied via flow over the "chip" and the change in signal is detected.

Once a series of potential compounds has been identified for a combination of IRGPP and IRGPP binding partner, a bioassay can be used to select the most promising candidates. For example, a cellular assay that measures cell proliferation in presence of the IRGPP and the IRGPP binding partner was described above. This assay could be modified to test the effectiveness of small molecules that interfere with binding of an IRGPP and its binding partner in enhancing cellular proliferation. An increase in cell proliferation would correlate with a compound's potency.

The test compounds described herein are generally either small molecules or biomolecules. Small molecules include, but are not limited to, inorganic molecules and small organic molecules. Biomolecules include, but are not limited to, naturally-occurring and synthetic compounds that have a bioactivity in mammals, such as lipids, steroids, polypeptides, polysaccharides, and polynucleotides. In one preferred embodiment, the test compound is a small molecule. In another preferred embodiment, the test compound is a biomolecule. One skilled in the art will appreciate that the nature of the test compound may vary depending on the nature of the IRGPP. For example, if the IRGPP is an orphan receptor having an unknown ligand, the test compound may be any of a number of biomolecules which may act as cognate ligand, including but not limited to, cytokines, lipid-derived mediators, small biogenic amines, hormones, neuropeptides, or proteases.

The test compounds described herein may be obtained from any available source, including systematic libraries of natural and/or synthetic compounds. Test compounds may also be obtained by any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds. As used herein, the term "binding partner" refers to a molecule which serves as either a substrate for an IRGPP, or alternatively, as a ligand having binding affinity to the IRGPP.

### High-Throushput Screening Assays

Described are methods of conducting high-throughput screening for test compounds capable of inhibiting activity or expression of an IRGPP described herein. It In one embodiment, the method of high-throughput screening involves combining test compounds and the IRGPP and detecting the effect of the test compound on the IRGPP.

A variety of high-throughput functional assays well-known in the art may be used in combination to screen and/or study the reactivity of different types of activating test compounds. Since the coupling system is often difficult to predict, a number of assays may need to be configured to detect a wide range of coupling mechanisms. A variety of fluorescence-based techniques are well-known in the art and are capable of high-throughput and ultra high throughput screening for activity, including but not limited to BRET^{®} or FRET^{®} (both by Packard Instrument Co., Meriden, CT). The ability to screen a large volume and a variety of test compounds with great sensitivity permits analysis of the therapeutic targets described herein to further provide potential inhibitors of influenza. For example, where the IRG encodes an orphan receptor with an unidentified ligand, high-throughput assays may be utilized to identify the ligand, and to further identify test compounds which prevent binding of the receptor to the ligand. The BIACORE^{®} system may also be manipulated to detect binding of test compounds with individual components of the therapeutic target, to detect binding to either the encoded protein or to the ligand.

By combining test compounds with IRGPPs described herein and determining the binding activity between them, diagnostic analysis can be performed to elucidate the coupling systems. Generic assays using cytoscnsor microphysiometer may also be used to measure metabolic activation, while changes in calcium mobilization can be detected by using the fluorescence-based techniques such as FLIPR^{®} (Molecular Devices Corp, Sunnyvale, CA). In addition, the presence of apoptotic cells may be determined by TUNEL assay, which utilizes flow cytometry to detect free 3-OH termini resulting from cleavage of genomic DNA during apoptosis. As mentioned above, a variety of functional assays well-known in the art may be used in combination to screen and/or study the reactivity of different types of activating test compounds. Preferably, the high-throughput screening assay described herein utilizes label-free plasmon resonance technology as provided by BIACORE^{®} systems (Biacore International AB, Uppsala, Sweden). Plasmon free resonance occurs when surface plasmon waves are excited at a metal/liquid interface. By reflecting directed light from the surface as a result of contact with a sample, the surface plasmon resonance causes a change in the refractive index at the surface layer. The refractive index change for a given change of mass concentration at the surface layer is similar for many bioactive agents (including proteins, peptides, lipids and polynucleotides), and since the BIACORE^{®} sensor surface can be functionalized to bind a variety of these bioactive agents, detection of a wide selection of test compounds can thus be accomplished.

Therefore, the disclosure provides for high-throughput screening of test compounds for the ability to inhibit activity of a protein encoded by the IRGs listed in Table 3, by combining the test compounds and the protein in high-throughput assays such as BIACORE^{®}, or in fluorescence-based assays such as BRET^{®}. In addition, high-throughput assays may be utilized to identify specific factors which bind to the encoded proteins, or alternatively, to identify test compounds which prevent binding of the receptor to the binding partner. In the case of orphan receptors, the binding partner may be the natural ligand for the receptor. Moreover, the high-throughput screening assays may be modified to determine whether test compounds can bind to either the encoded protein or to the binding partner (e.g., substrate or ligand) which binds to the protein.

### Detection Methods

Detection and measurement of the relative amount of an IRG product (polynucleotide or polypeptide) described herein can be by any method known in the art. Typical methodologies for detection of a transcribed polynucleotide include RNA extraction from a cell or tissue sample, followed by hybridization of a labeled probe (i.e., a complementary polynucleotide molecule) specific for the target RNA to the extracted RNA and detection of the probe (i.e., Northern blotting).

Typical methodologies for peptide detection include protein extraction from a cell or tissue sample, followed by binding of an antibody specific for the target protein to the protein sample, and detection of the antibody. For example, detection of desmin may be accomplished using polyclonal antibody anti-desmin. Antibodies are generally detected by the use of a labeled secondary antibody. The label can be a radioisotope, a fluorescent compound, an enzyme, an enzyme co-factor, or ligand. Such methods are well understood in the art.

Detection of specific polynucleotide molecules may also be assessed by gel electrophoresis, column chromatography, or direct sequencing, quantitative PCR (in the case of polynucleotide molecules), RT-PCR, or nested-PCR among many other techniques well known to those skilled in the art.

Detection of the presence or number of copies of all or a part of an IRG described herein may be performed using any method known in the art. Typically, it is convenient to assess the presence and/or quantity of a DNA or cDNA by Southern analysis, in which total DNA from a cell or tissue sample is extracted and hybridized with a labeled probe (i.e., a complementary DNA molecules). The probe is then detected and quantified. The label group can be a radioisotope, a fluorescent compound, an enzyme, or an enzyme co-factor. Other useful methods of DNA detection and/or quantification include direct sequencing, gel electrophoresis, column chromatography, and quantitative PCR, as is known by one skilled in the art.

Detection of specific polypeptide molecules may be assessed by gel electrophoresis, Western blot, column chromatography, or direct sequencing, among many other techniques well known to those skilled in the art.

An exemplary method for detecting the presence or absence of an IRGPP or IRGPN in a biological sample involves contacting a biological sample with a compound or an agent capable of detecting the IRGPP or IRGPN (e.g., mRNA, genomic DNA). A preferred agent, for detecting mRNA or genomic DNA corresponding to an IRG or IRGPP described herein is a labeled polynucleotide probe capable of hybridizing to a mRNA or genomic DNA of the invention. In a most preferred embodiment, the polynucleotides to be screened are arranged on a GeneChip®. Suitable probes for use in the diagnostic assays described herein are described herein.

A preferred agent for detecting an IRGPP is an antibody capable of binding to the IRGPP, preferably an antibody with a detectable label. Antibodies can be polyclonal or more preferably, monoclonal. An intact antibody, or a fragment thereof (e.g., Fab or F(ab')₂) can be used. The term "labeled," with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method described herein can be used to detect IRG mRNA, protein or genomic DNA in a biological sample *in vitro* as well as *in vivo.* For example, *in vitro* techniques for detection of IRG mRNA include Northern hybridizations and *in situ* hybridizations. *In vitro* techniques for detection of IRGPP include enzyme linked immunosorbent assays (ELISAs), Western blots, immunoprecipitations and immunofluorescence. *In vitro* techniques for detection of IRG genomic DNA include Southern hybridizations. Furthermore, *in vivo* techniques for detection of IRGPP include introducing into a subject a labeled anti-IRGPP antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a tissue or serum sample isolated by conventional means from a subject, e.g., a biopsy or blood draw.

### Detection of genetic alterations

The methods described herein can also be used to detect genetic alterations in an IRG, thereby determining if a subject with the altered gene is at risk for damage characterized by aberrant regulation in IRG expression or activity. In preferred embodiments, the methods include detecting, in a sample of cells from the subject, the presence or absence of a genetic alteration characterized by at least one alteration affecting the integrity of an IRG, or the aberrant expression of the IRG. For example, such genetic alterations can be detected by ascertaining the existence of at least one of the following: 1) deletion of one or more nucleotides from an IRG; 2) addition of one or more nucleotides to an IRG; 3) substitution of one or more nucleotides of an IRG, 4) a chromosomal rearrangement of an IRG; 5) alteration in the level of a messenger RNA transcript of an IRG, 6) aberrant modification of an IRG, such as of the methylation pattern of the genomic DNA, 7) the presence of a non-wild type splicing pattern of a messenger RNA transcript of an IRG, 8) non-wild type level of an IRGPP, 9) allelic loss of an IRG, and 10) inappropriate post-translational modification of an IRGPP. As described herein, there are a large number of assays known in the art, which can be used for detecting alterations in an IRG or an IRG product. A preferred biological sample is a blood sample isolated by conventional means from a subject.

In certain embodiments, detection of the alteration involves the use of a probe/primer in a polymerase chain reaction (PCR), such as anchor PCR or RACE PCR, or, alternatively, in a ligation chain reaction (LCR), the latter of which can be particularly useful for detecting point mutations in the IRG. This method can include the steps of collecting a sample of cells from a subject, isolating a polynucleotide sample (e.g., genomic, mRNA or both) from the cells of the sample, contacting the polynucleotide sample with one or more primers which specifically hybridize to an IRG under conditions such that hybridization and amplification of the IRG (if present) occurs, and detecting the presence or absence of an amplification product, or detecting the size of the amplification product and comparing the length to a control sample. It is understood that PCR and/or LCR may be desirable to be used as a preliminary amplification step in conjunction with any of the techniques used for detecting mutations described herein.

Alternative amplification methods include: self-sustained sequence replication, transcriptional amplification system, Q-Beta Replicase, or any other polynucleotide amplification method, followed by the detection of the amplified molecules using techniques well known to those of skill in the art. These detection schemes are especially useful for the detection of polynucleotide molecules if such molecules are present in very low numbers.

In an alternative embodiment, mutations in an IRG from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicate mutations in the sample DNA. Moreover, sequence specific ribozymes (see, for example, U.S. Patent No. 5,498,531) can be used to score for the presence of specific mutations by development or loss of a ribozyme cleavage site.

In other embodiments, genetic mutations in an IRG can be identified by hybridizing sample and control polynucleotides, e.g., DNA or RNA, to high density arrays containing hundreds or thousands of oligonucleotides probes. For example, genetic mutations in an IRG can be identified in two dimensional arrays containing light generated DNA probes. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the IRG and detect mutations by comparing the sequence of the sample IRG with the corresponding wild-type (control) sequence. It is also contemplated that any of a variety of automated sequencing procedures can be utilized when performing the diagnostic assays, including sequencing by mass spectrometry.

Other methods for detecting mutations in an IRG include methods in which protection from cleavage agents is used to detect mismatched bases in RNA/RNA or RNA/DNA heteroduplexes. In general, the art technique of "mismatch cleavage" starts by providing heteroduplexes by hybridizing (labeled) RNA or DNA containing the wild-type IRG sequence with potentially mutant RNA or DNA obtained from a tissue sample. The double-stranded duplexes are treated with an agent which cleaves single-stranded regions of the duplex, which will exist due to basepair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in IRG cDNAs obtained from samples of cells. For example, the mutY enzyme of E. coli cleaves A at G/A mismatches and the thymidine DNA glycosylase from HeLa cells cleaves T at G/T mismatches. According to an exemplary embodiment, a probe based on an IRG sequence, e.g., a wild-type IRG sequence, is hybridized to cDNA or other DNA product from a test cell(s). The duplex is treated with a DNA mismatch repair enzyme, and the cleavage products, if any, can be detected from, e.g., electrophoresis protocols. See for example, U.S. Patent No. 5,459,039.

In other embodiments, alterations in electrophoretic mobility will be used to identify mutations in IRGs. For example, single-strand conformation polymorphism (SSCP) may be used to detect differences in electrophoretic mobility between mutant and wild type polynucleotides. Single-stranded DNA fragments of sample and control IRG polynucleotides will be denatured and allowed to renature. The secondary structure of single-stranded polynucleotides varies according to sequence. The resulting alteration in electrophoretic mobility enables the detection of even a single base change. The DNA fragments may be labeled or detected with labeled probes. The sensitivity of the assay may be enhanced by using RNA (rather than DNA) in which the secondary structure is more sensitive to a change in sequence. In a preferred embodiment, the subject method utilizes heteroduplex analysis to separate double-stranded heteroduplex molecules on the basis of changes in electrophoretic mobility (Keen et al. Trends Genet 7:5, 1991).

In yet another embodiment the movement of mutant or wild-type fragments in polyacrylamide gels containing a gradient of denaturant is assayed using denaturing gradient gel electrophoresis (DGGE). When DGGE is used as the method of analysis, DNA will be modified to insure that it does not completely denature, for example, by adding a GC clamp of approximately 40bp of high-melting GC-rich DNA by PCR. In a further embodiment a temperature gradient is used in place of a denaturing gradient to identify differences in the mobility of control and sample DNA (Rosenbaum and Reissner Biophys Chem 265:12753, 1987).

Examples of other techniques for detecting point mutations include, but are not limited to, selective oligonucleotide hybridization, selective amplification, and selective primer extension. For example, oligonucleotide primers may be prepared in which the known mutation is placed centrally and then hybridized to target DNA under conditions which permit hybridization only if a perfect match is found (Saiki et al. Proc. Natl. Acad. Sci USA 86:6230, 1989). Such allele specific oligonucleotides are hybridized to PCR amplified target or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) or at the extreme 3' end of one primer where, under appropriate conditions, mismatch can prevent or reduce polymerase extension. In addition, it may be desirable to introduce a novel restriction site in the region of the mutation to create cleavage-based detection. It is anticipated that, in certain embodiments, amplification may also be performed using Taq ligase for amplification. In such cases, ligation will occur only if there is a perfect match at the 3' end of the 5' sequence, thus making it possible to detect the presence of a known mutation at a specific site by looking for the presence or absence of amplification.

### Monitoring Effects During Clinical Trials

Monitoring the influence of agents (e.g., drugs, small molecules, proteins, nucleotides) on the expression of an IRG or activity of an IRGPP can be applied not only in basic drug screening, but also in clinical trials. For example, the effectiveness of an agent determined by a screening assay, as described herein to decrease an IRGPP activity, can be monitored in clinical trials of subjects exhibiting increased IRGPP activity. In such clinical trials, the activity of the IRGPP can be used as a "read-out" of the phenotype of a particular tissue.

For example, and not by way of limitation, IRGs that are modulated in tissues by treatment with an agent can be identified. Thus, to study the effect of agents on the IRGPP in a clinical trial, cells can be isolated and RNA prepared and analyzed for the levels of expression of an IRG. The levels of gene expression or a gene expression pattern can be quantified by Northern blot analysis, RT-PCR or GeneChip® as described herein, or alternatively by measuring the amount of protein produced, by one of the methods as described herein, or by measuring the levels of activity of IRGPP. In this way, the gene expression pattern can serve as a read-out, indicative of the physiological response of the cells to the agent. Accordingly, this response state may be determined before treatment and at various points during treatment of the individual with the agent.

In a preferred embodiment, the present disclosure provides a method for monitoring the effectiveness of treatment of a subject with an agent (e.g., an agonist, antagonist, peptidomimetic, protein, peptide, polynucleotide, small molecule, or other drug candidate identified by the screening assays described herein) including the steps of (i) obtaining a pre-administration sample from a subject prior to administration of the agent; (ii) detecting the level of expression of an IRG protein or mRNA in the pre-administration sample; (iii) obtaining one or more post-administration samples from the subject; (iv) detecting the level of expression or activity of the IRG protein or mRNA in the post-administration samples; (v) comparing the level of expression or activity of the IRG protein or mRNA in the pre-administration sample with the IRG protein or mRNA the post administration sample or samples; and (vi) altering the administration of the agent to the subject accordingly. According to such an embodiment, IRG expression or activity may be used as an indicator of the effectiveness of an agent, even in the absence of an observable phenotypic response.

### Methods of Treatment

The present invention provides an antibody for use in both prophylactic and therapeutic methods of treating a subject at risk for, susceptible to or diagnosed with influenza.
In one aspect, the invention provides an antibody which binds to a polypeptide comprising the amino acid sequence of SEQ ID NO: 17 (PTCH) for use in a method for enhancing the resistance of a mammal to infection by an influenza virus or a method to treat influenza virus infection in a mammal. Also provided is a composition for use in a method of enhancing the resistance of a mammal to influenza virus, wherein said composition comprises an antibody suspended in a pharmaceutically acceptable carrier, which antibody binds to a polypeptide comprising the amino acid sequence of SEQ ID NO: 17 (PTCH). Also described herein is a method for preventing influenza in a subject by administering to the subject an IRG product or an agent which modulates IRG protein expression or activity.

Administration of a prophylactic agent can occur prior to the manifestation of symptoms characteristic of the differential IRG protein expression, such that influenza is prevented or, alternatively, delayed in its progression. Depending on the type of IRG aberrancy (e.g., typically a modulation outside the normal standard deviation), for example, an IRG product, IRG agonist or antagonist agent can be used for treating the subject. The appropriate agent can be determined based on screening assays described herein.

Described herein are methods of modulating IRG protein expression or activity for therapeutic purposes. Accordingly, in an exemplary embodiment, the modulatory method described herein involves contacting a cell with an agent that modulates one or more of the activities of a IRG product activity associated with the cell. An agent that modulates IRG product activity can be an agent as described herein, such as a polynucleotide (e.g., an antisense molecule) or a polypeptide (e.g., a dominant-negative mutant of an IRGPP), a naturally-occurring target molecule of an IRGPP (e.g., an IRGPP substrate), an anti-TRGPP antibody, an IRG modulator (e.g., agonist or antagonist), a peptidomimetic of an IRG protein agonist or antagonist, or other small molecules.

Also described are methods of modulating a level of expression of an IRG described herein, comprising administration to a subject having influenza, a variety of compositions which correspond to the IRGs of Table 3, including proteins or antisense oligonucleotides. The protein may be provided by further providing a vector comprising a polynucleotide encoding the protein to the cells. Alternatively, the expression levels of the IRGs described herein may be modulated by providing an antibody, a plurality of antibodies or an antibody conjugated to a therapeutic moiety.

### Determining Efficacy of a Test Compound or Therapy

Also described are methods of assessing the efficacy of a test compound or therapy for inhibiting influenza in a subject. These methods involve isolating samples from a subject suffering from influenza, who is undergoing treatment or therapy, and detecting the presence, quantity, and/or activity of one or more IRGs described herein in the first sample relative to a second sample. Where the efficacy of a test compound is determined, the first and second samples arc preferably sub-portions of a single sample taken from the subject, wherein the first portion is exposed to the test compound and the second portion is not. In one aspect of this embodiment, the IRG is expressed at a substantially decreased level in the first sample, relative to the second. Most preferably, the level of expression in the first sample approximates (i.e., is less than the standard deviation for normal samples) the level of expression in a third control sample, taken from a control sample of normal tissue. This result suggests that the test compound inhibits the expression of the IRG in the sample. In another aspect of this embodiment, the IRG is expressed at a substantially increased level in the first sample, relative to the second. Most preferably, the level of expression in the first sample approximates (i.e., is less than the standard deviation for normal samples) the level of expression in a third control sample, taken from a control sample of normal tissue. This result suggests that the test compound augments the expression of the IRG in the sample.

Where the efficacy of a therapy is being assessed, the first sample obtained from the subject is preferably obtained prior to provision of at least a portion of the therapy, whereas the second sample is obtained following provision of the portion of the therapy. The levels of IRG product in the samples are compared, preferably against a third control sample as well, and correlated with the presence, or risk of presence, of influenza. Most preferably, the level of IRG product in the second sample approximates the level of expression of a third control sample. A substantially decreased level of expression of an IRG indicates that the therapy is efficacious for treating influenza.

### Pharmaceutical Compositions

The invention is further directed to pharmaceutical compositions for use in a method of enhancing the resistance of a mammal to influenza virus, wherein said composition comprises an antibody suspended in a pharmaceutically acceptable carrier, which antibody binds to a polypeptide comprising the amino acid sequence of SEQ ID NO: 17 (PTCH). Also described are pharmaceutical compositions comprising the test compound, or bioactive agent, or an IRG modulator (i.e., agonist or antagonist), which may further include an IRG product, and can be formulated as described herein. Alternatively, these compositions may include an antibody which specifically binds to an IRG protein described herein and/or an antisense polynucleotide molecule which is complementary to an IRGPN described herein and can be formulated as described herein.

One or more of the IRGs described herein, fragments of IRGs, IRG products, fragments of IRG products, IRG modulators, or anti-IRGPP antibodies can be incorporated into pharmaceutical compositions suitable for administration. In particular, the anti-IRGPP antibody of the invention is an antibody which binds to a polypeptide comprising the amino acid sequence of SEQ ID NO: 17 (PTCH).

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

Also described are methods for preparing pharmaceutical compositions for modulating the expression or activity of a polypeptide or polynucleotide corresponding to an IRG described herein. Such methods comprise formulating a pharmaceutically acceptable carrier with an agent which modulates expression or activity of an IRG. Such compositions can further include additional active agents. Thus, also described are methods for preparing a pharmaceutical composition by formulating a pharmaceutically acceptable carrier with an agent which modulates expression or activity of an IRG and one or more additional bioactive agents.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), intraperitoneal, transmucosal, and rectal administration. Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersion. For intravenous administration, suitable carriers include physiological saline, bacteriostatic water, Cremophor EL^{TM} (BASF, Parsippany, NJ) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid and thimerosal. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a fragment of an IRGPP or an anti-IRGPP antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The, for example, tablets, pills, capsules and troches can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the bioactive compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the therapeutic moieties, which may contain a bioactive compound, are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form, as used herein, includes physically discrete units suited as unitary dosages for the subject to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that includes the ED50 with little or no toxicity. The dosage may vary within this range depending upon the dosage form employed and the route of administration utilized. For any compound used in the method described herein,

the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The IRGs described hereincan be inserted into gene delivery vectors and used as gene therapy vectors. Gene therapy vectors can be delivered to a subject by, for example, intravenous administration, intraportal administration, intrabiliary administration, intraarterial administration, direct injection into the liver parenchyma, by intramusclular injection, by inhalation, by perfusion, or by stereotactic injection. The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Kits

Also described are kits for detecting the presence of an IRG product in a biological sample, the kit comprising reagents for assessing expression of the IRGs described herein.

Preferably, the reagents may be an antibody or fragment thereof, wherein the antibody or fragment thereof specifically binds with a protein corresponding to an IRG from Table 3. For example, antibodies of interest may be prepared by methods known in the art. Optionally, the kits may comprise a polynucleotide probe wherein the probe specifically binds with a transcribed polynucleotide corresponding to an IRG selected from the group consisting of the IRGs listed in Table 3. The kits may also include an array of IRGs arranged on a biochip, such as, for example, a GeneChip^{®}. The kit may contain means for determining the amount of the IRG protein or mRNA in the sample; and means for comparing the amount of the IRG protein or mRNA in the sample with a control or standard. The compound or agent can be packaged in a suitable container. The kit can further comprise instructions for using the kit to detect IRG protein or polynucleotide

Also described are kits for assessing the suitability of each of a plurality of compounds for inhibiting influenza in a subject. Such kits include a plurality of compounds to be tested, and a reagent (i.e., antibody specific to corresponding proteins, or a probe or primer specific to corresponding polynucleotides) for assessing expression of an IRG listed in Table 3.

### Arrays and Biochips

Also described is an array comprising a panel of IRGs described herein.

The array can be used to assay expression of one or more genes in the array.

It will be appreciated by one skilled in the art that the panels of IRGs described herein may conveniently be provided on solid supports, such as a biochip. For example, polynucleotides may be coupled to an array (e.g., a biochip using GeneChip^{®} for hybridization analysis), to a resin (e.g., a resin which can be packed into a column for column chromatography), or a matrix (e.g., a nitrocellulose matrix for Northern blot analysis). The immobilization of molecules complementary to the IRG(s), either covalently or noncovalently, permits a discrete analysis of the presence or activity of each IRG in a sample. In an array, for example, polynucleotides complementary to each member of a panel of IRGs may individually be attached to different, known locations on the array. The array may be hybridized with, for example, polynucleotides extracted from a blood or colon sample from a subject. The hybridization of polynucleotides from the sample with the array at any location on the array can be detected, and thus the presence or quantity of the IRG and IRG transcripts in the sample can be ascertained. In a preferred embodiment, an array based on a biochip is employed. Similarly, Western analyses may be performed on immobilized antibodies specific for IRGPPs hybridized to a protein sample from a subject.

It will also be apparent to one skilled in the art that the entire IRG product (protein or polynucleotide) molecule need not be conjugated to the biochip support; a portion of the IRG product or sufficient length for detection purposes (i.e., for hybridization), for example a portion of the IRG product which is 7, 10, 15, 20, 25, 30, 35, 40, 45, 50, 55, 60, 65, 70, 75, 100 or more nucleotides or amino acids in length may be sufficient for detection purposes.

In addition to such qualitative determination, the disclosure allows the quantitation of gene expression in the biochip. Thus, not only tissue specificity, but also the level of expression of a battery of IRGs in the tissue is ascertainable. Thus, IRGs can be grouped on the basis of their tissue expression per se and level of expression in that tissue. As used herein, a "normal level of expression" refers to the level of expression of a gene provided in a control sample, typically the control is taken from either a non-diseased animal or from a subject who has not suffered from influenza. The determination of normal levels of expression is useful, for example, in ascertaining the relationship of gene expression between or among tissues. Thus, one tissue or cell type can be perturbed and the effect on gene expression in a second tissue or cell type can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the disclosure provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the arrays can be used to monitor the time course of expression of one or more genes in the array. This can occur in various biological contexts, as disclosed herein, for example development and differentiation, disease progression, *in vitro* processes, such as cellular transformation and activation.

The array is also useful for ascertaining the effect of the expression of a gene on the expression of other genes in the same cell or in different cells. This provides, for example, for a selection of alternate molecular targets for therapeutic intervention if the ultimate or downstream target cannot be regulated.

Importantly, described are arrays useful for ascertaining differential expression patterns of one or more genes identified in diseased tissue versus non-diseased tissue. This provides a battery of genes that serve as a molecular target for diagnosis or therapeutic intervention. In particular, biochips can be made comprising arrays not only of the IRGs listed in Table 3, but of IRGs specific to subjects suffering from specific manifestations or stages of the disease.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. As will be appreciated by those in the art, the number of possible substrates are very large, and include, but are not limited to, glass and modified or functionalized glass, plastics (including, e.g., acrylics, polystyrene and copolymers of styrene and other materials, polypropylene, polyethylene, polybutylene, polyurethanes and TeflonJ), polysaccharides, nylon or nitrocellulose, resins, silica or silica-based materials including silicon and modified silicon, carbon, metals, inorganic glasses and plastics.

Generally the substrate is planar, although as will be appreciated by those in the art, other configurations of substrates may be used as well. For example, the probes may be placed on the inside surface of a tube, for flow-through sample analysis to minimize sample volume. Similarly, the substrate may be flexible, such as a flexible foam, including closed cell foams made of particular plastics.

In a preferred embodiment, the surface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups. Linkers, such as homo-or hetero-bifunctional linkers, may also be used.

In an embodiment, the oligonucleotides are synthesized as is known in the art, and then attached to the surface of the solid support. As will be appreciated by those skilled in the art, either the 5' or 3' terminus maybe attached to the solid support, or attachment maybe via an internal nucleoside.

In an additional embodiment, the immobilization to the solid support may be very strong, yet non-covalent. For example, biotinylatcd oligonucleotides can be made, which bind to surfaces covalently coated with streptavidin, resulting in attachment.

Alternatively, the oligonucleotides may be synthesized on the surface, as is known in the art. For example, photoactivation techniques utilizing photopolymerization compounds and techniques are used. In a preferred embodiment, the nucleic acids can be synthesized *in situ*, using well known photolithographic techniques.

Modifications to the above-described compositions and methods according to standard techniques, will be readily apparent to one skilled in the art.

This invention is further illustrated by the following examples which should not be construed as limiting.

### Host cells

Also described are host cells into which a polynucleotide molecule describe herein, e.g., an IRG of Table 3 or homolog thereof, is introduced within an expression vector, a gene delivery vector, or a polynucleotide molecule described herein containing sequences which allow it to homologously recombine into a specific site of the host cell's genome. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, an IRG can be expressed in bacterial cells such as E. coli, insect cells, yeast or mammalian cells (such as Chinese hamster ovary cells (CHO), COS cells, Fischer 344 rat cells, HLA-B27 rat cells, HeLa cells, A549 cells, or 293 cells. Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign polynucleotide (e.g., DNA) into a host cell, including calcium phosphate or calcium chloride co-precipitation, DAKD-dextran-mediated transfection, lipofection, or electoporation.

For stable transfection of mammalian cells, it is known that, depending upon the expression vector and transfection technique used, only a small fraction of cells may integrate the foreign DNA into their genome. In order to identify and select these integrants, a gene that encodes a selectable flag (*e.g.*, resistance to antibiotics) is generally introduced into the host cells along with the gene of interest. Preferred selectable flags include those which confer resistance to drugs, such as G418, hygromycin and methotrexate. Polynucleotide encoding a selectable flag can be introduced into a host cell on the same vector as that encoding STK3P23 or can be introduced on a separate vector. Cells stably transfected with the introduced polynucleotide can be identified by drug selection (e.g., cells that have incorporated the selectable flag gene will survive, while the other cells die).

A host cell described herein, such as a prokaryotic or eukaryotic host cell in culture, can be used to produce (i.e., express) an IRG product. Accordingly, also described are methods for producing an IRG product using the host cells described herein. In one embodiment, the method comprises culturing the host cell described herein (onto which a recombinant expression vector encoding an IRG has been introduced) in a suitable medium such that an IRG product is produced. In another embodiment, the method further comprises isolating the IRG product from the medium or the host cell.

### Transgenic and knockout animals

The host cells described herein can also be used to produce non-human transgenic animals. For example, in one embodiment, a host cell described herein is a fertilized oocyte or an embryonic stem cell into which an IRG sequence has been introduced. Such host cells can then be used to create non-human transgenic animals in which an exogenous sequence encoding an IRG has been introduced into their genome or homologous recombinant animals in which an endogenous sequence encoding an IRG has been altered. Such animals are useful for studying the function and/or activity of the IRG and for identifying and/or evaluating modulators of the IRG activity. As used herein, a "transgenic animal" is a non-human animal, preferably a mammal, more preferably a rodent such as a rat or mouse, in which one or more of the cells of the animal includes a transgene. Other examples of transgenic animals include, for example, non-human primates, sheep, dogs, cows, goats, chickens and amphibians. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous IRG has been altered by homologous recombination between the endogenous gene and an exogenous DNA molecule introduced into a cell of the animal, e.g., an embryonic cell of the animal, prior to development of the animal.

A transgenic animal described herein can be created by introducing an IRG-encoding polynucleotide into the mate pronuclei of a fertilized oocyte, e.g., by microinjection or retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to a transgene to direct expression of an IRG to particular cells. Methods for generating transgenic animals via embryo manipulation and microinjection, particularly animals such as mice, have become conventional in the art. Similar methods are used for production of other transgenic animals. A transgenic founder animal can be identified based upon the presence of a transgene described herein in its genome and/or expression of mRNA corresponding to a gene described herein in tissues or cells of the animals. A transgenic founder animal can then be used to breed additional animals carrying the transgene. Moreover, transgenic animals carrying an IRG can further be bred to other transgenic animals carrying other transgenes.

To create a homologous recombinant animal (knockout animal), a vector is prepared which contains at least a portion of a gene described herein into which a deletion, addition or substitution has been introduced to thereby alter, e.g., functionally disrupt, the gene. The gene can be a human gene, but more preferably, is a non-human homolog of a human gene of the invention (e.g., a homolog of an IRG). For example, a mouse gene can be used to construct a homologous recombination polynucleotide molecule, e.g., a vector, suitable for altering an endogenous gene described herein in the mouse genome. In a preferred embodiment, the homologous recombination polynucleotide molecule is designed such that, upon homologous recombination, the endogenous gene described herein is functionally disrupted (i.e., no longer encodes a functional protein; also referred to as a "knockout" vector). Alternatively, the homologous recombination polynucleotide molecule can be designed such that, upon homologous recombination, the endogenous gene is mutated or otherwise altered but still encodes functional protein (e.g., the upstream regulatory region can be altered to thereby alter the expression of the endogenous IRG). In the homologous recombination polynucleotide molecule, the altered portion of the gene described herein is flanked at its 5' and 3' ends by additional polynucleotide sequence of the gene described herein to allow for homologous recombination to occur between the exogenous gene carried by the homologous recombination polynucleotide molecule and an endogenous gene in a cell, e.g., an embryonic stem cell. The additional flanking polynucleotide sequence is of sufficient length for successful homologous recombination with the endogenous gene.

Typically, several kilobases of flanking DNA (both at the 5' and 3' ends) are included in the homologous recombination polynucleotide molecule (see, e.g., Thomas, K.R. and Capecchi, M.R. (1987) Cell 51:503 for a description of homologous recombination vectors). The homologous recombination polynucleotide molecule is introduced into a cell, e.g., an embryonic stem cell line (e.g., by electroporation) and cells in which the introduced gene has homologously recombined with the endogenous gene are selected. The selected cells can then be injected into a blastocyst of an animal (e.g., a mouse) to form aggregation chimeras (see e.g., Bradley, S A. in Teratocarcirtomas and Embryonic Stem Cells: A Practical Approach, E.J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination polynucleotide molecules, e.g., vectors, or homologous recombinant animals are described further in Bradley, A. (1991) Current Opinion in Biotechnology 2:823-829 and in PCT International Publication Nos.: WO 90/11354 by Le Moucllcc et al.; WO 91/01140 by Smithies et al.; WO 92/0968 by Zijlstra et al.; and WO 93/04169 by Berns et al.

In another embodiment, transgenic non-human animals can be produced which contain selected systems which allow for regulated expression of the transgene. One example of such a system is the cre/loxP recombinase system of bacteriophage P1. For a description of the crc/loxP recombinase system, see, e.g., Laksa ct al. (1992) Proc. Natl. Acad. Sci. USA 89:6232-6236. Another example of a recombinase system is the FLP recombinase system of Saccharomyces cerevisiae (O'Gorman et al. (1991) Science 251:1351-1355. If a crc/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase.

Clones of the non-human transgenic animals described herein can also be produced according to the methods described in Wilmut, I. et al. (1997) Nature 385:810-813 and PCT International Publication Nos. WO 97/07668 and WO 97/07669. In brief, a cell, e.g., a somatic cell, from the transgenic animal can be isolated and induced to exit the growth cycle and enter Go phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocytc and then transferred to pseudopregnant female foster animal. The offspring borne of this female foster animal will be a clone of the animal from which the cell, *e.g.,* the somatic cell, is isolated.

Modifications to the above-described compositions and methods, according to standard techniques, will be readily apparent to one skilled in the art.

This invention is further illustrated by the following examples

### EXAMPLES

### Example 1: Construction of RHKO vectors and screening of influenza resistant clones

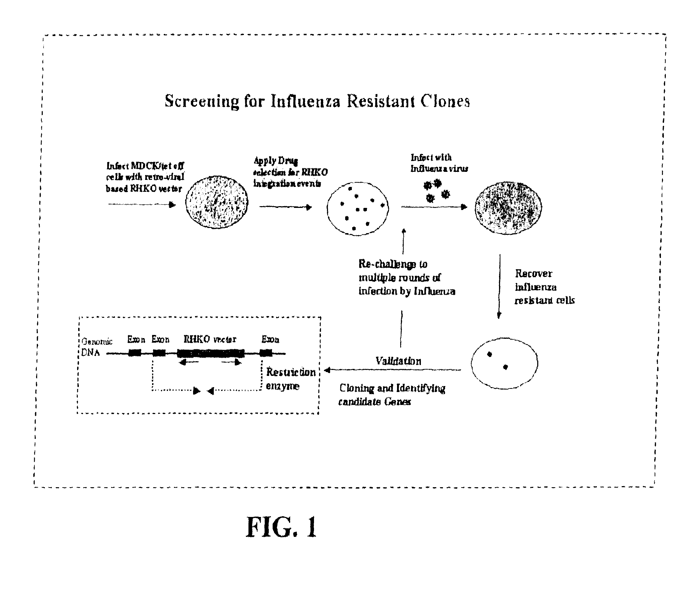

RHKO vectors were constructed as described by Li et al. (Li et al. Cell, 85: 319-329, 1996). The procedure for screening influenza resistant clones is depicted in Figure 1. Briefly, Madin Darby Canine Kidney (MDCK) cells were infected with a retro-viral based random homozygous knock-out (RHKO) vector. Cells containing the stably integrated vector were selected and subjected to influenza infection using the MOI which would result in 100% killing of parental cells between 48 to 72 hour. The influenza resistant cells were expanded and subject to additional rounds of influenza infection with higher multiplicity of infection (MOI). The resistant clones that survived multiple rounds of influenza infection were recovered. The influenza resistant phenotype was validated by testing the clones' resistance to multiple strains of influenza virus and by correlation of the phenotype with RHKO integration. The RHKO integration sites in the resistant cells were then cloned and identified as described in Example 2.

### Example 2: Identification of influenza resistant genes

The RHKO integration sites in the resistant cells were cloned and the sequences flanking the RHKO integration site were determined. The affected genes were identified by aligning the flanking sequences at the integration site to the Genebank database.

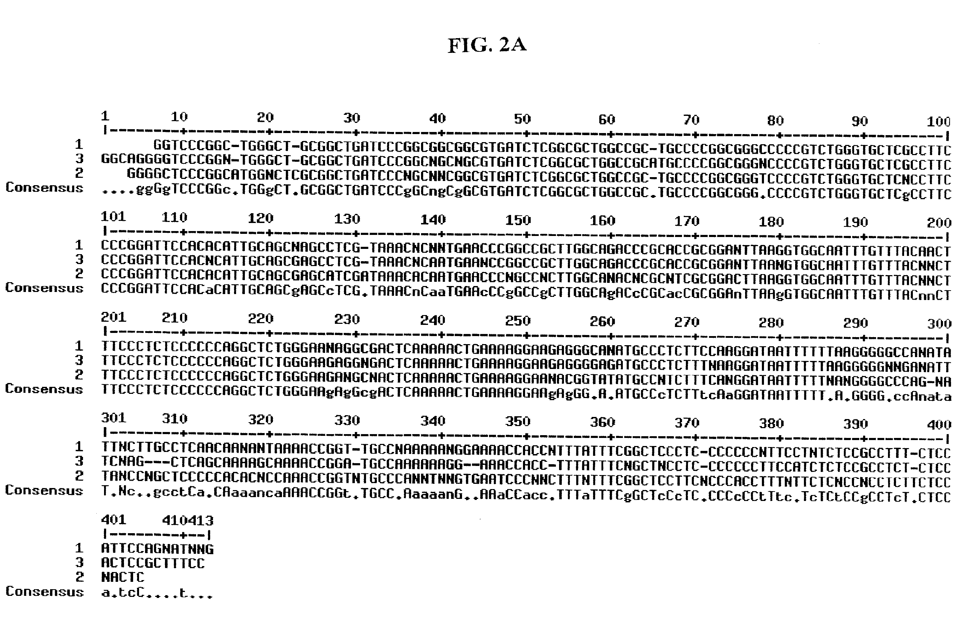

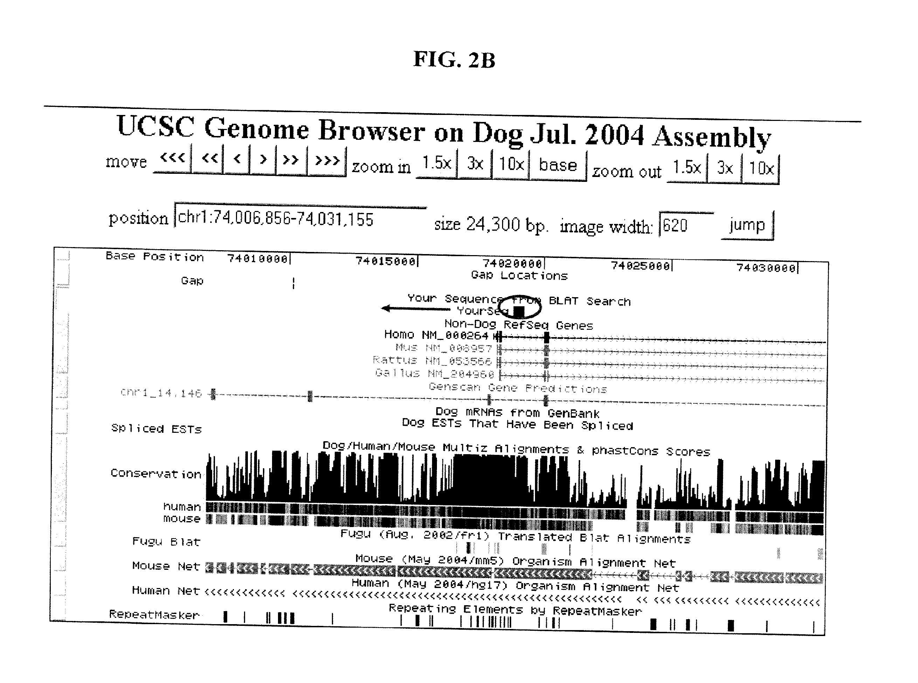

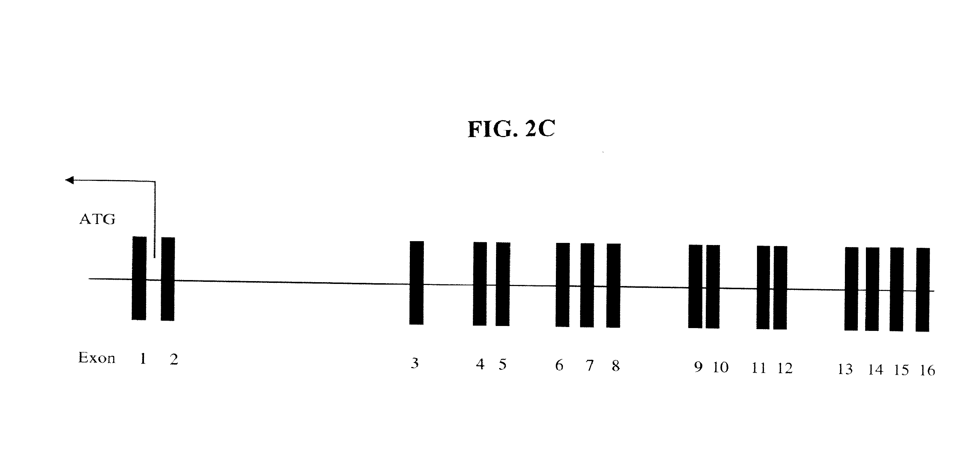

Figure 2A shows the alignment of the 5'-end flanking sequences obtained from three subclones of influenza resistant clone 26-8-7. The consensus sequence derived from the alignment (SEQ ID NO:1) was used to identify the affected gene PTCH (SEQ ID NOS: 9 and 17). Figure 2B depicts the genomic site of RHKO integration. As shown in Figure 2C, the position of the RHKO integration indicate that the PTCH gene is likely to be inactivated by the antisense expression from the RHKO construct.

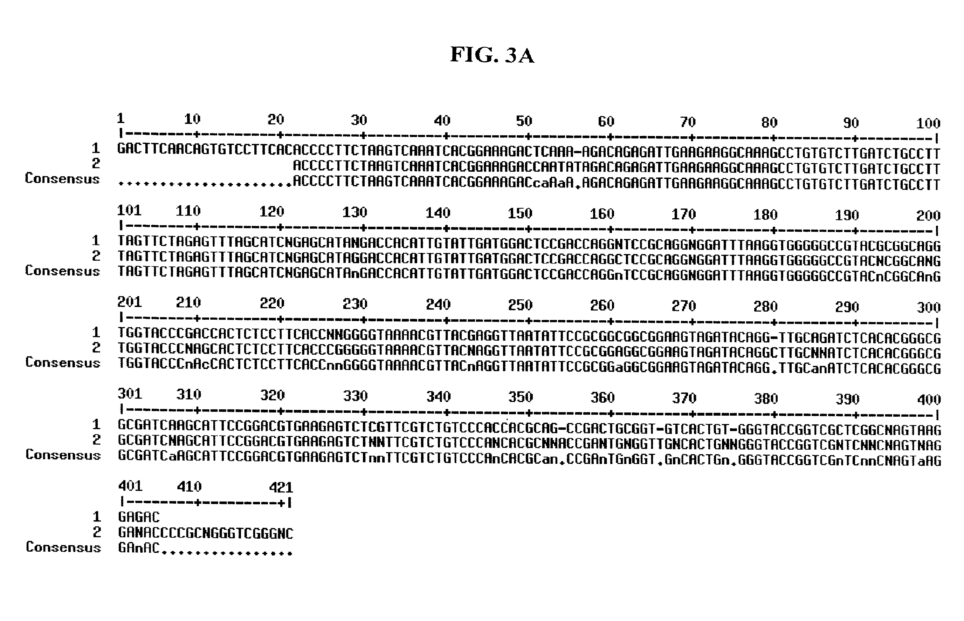

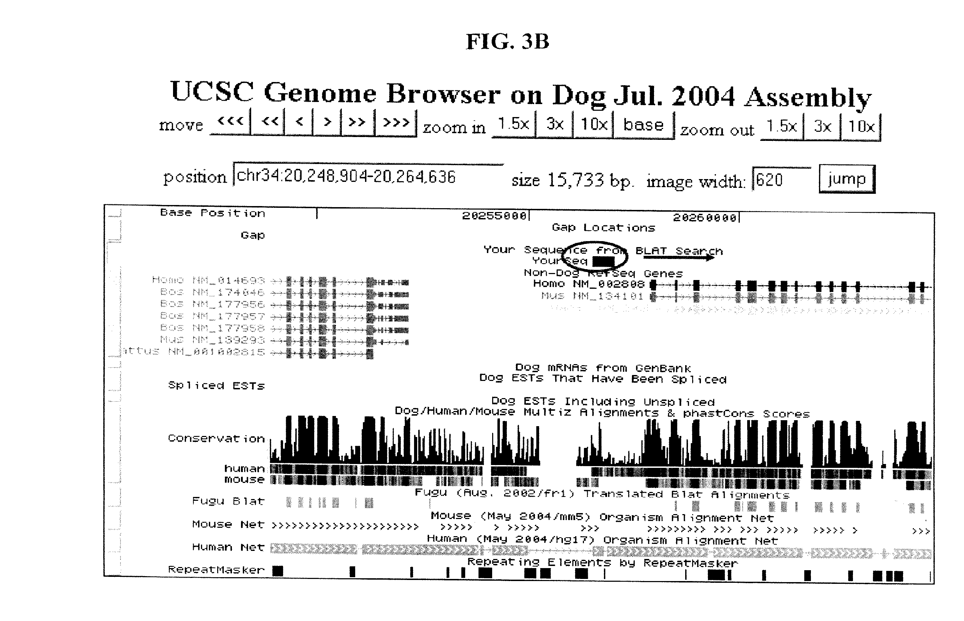

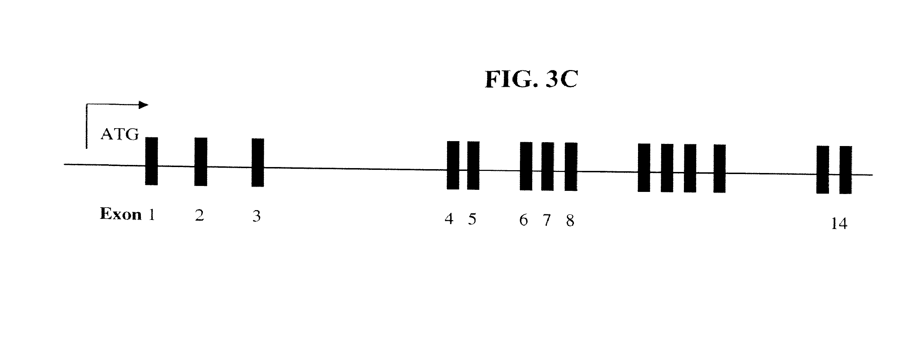

Figure 3A shows the alignment of the 5'-end flanking sequences obtained from two subclones of influenza resistant clone R18-6. The consensus sequence derived from the alignment (SEQ ID NO:2) was used to identify the affected gene PSMD2 (SEQ ID NOS: 10 and 18). Figure 3B depicts the genomic site of RHKO integration. As shown in Figure 3C, the position of the RHKO integration indicate that the PSMD2 gene is likely to be overexpressed due to activation by the RHKO construct.

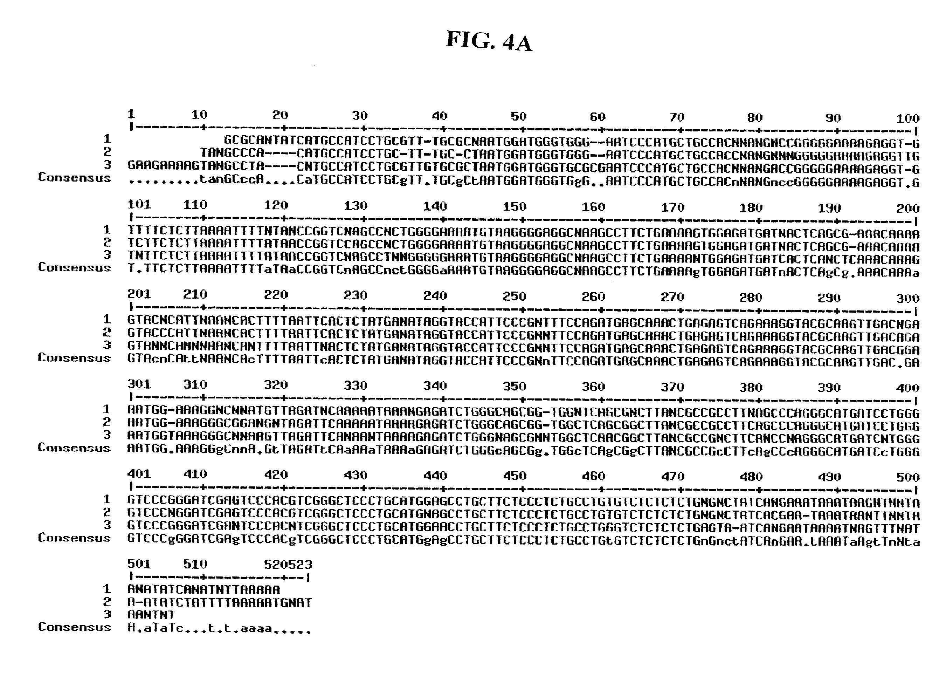

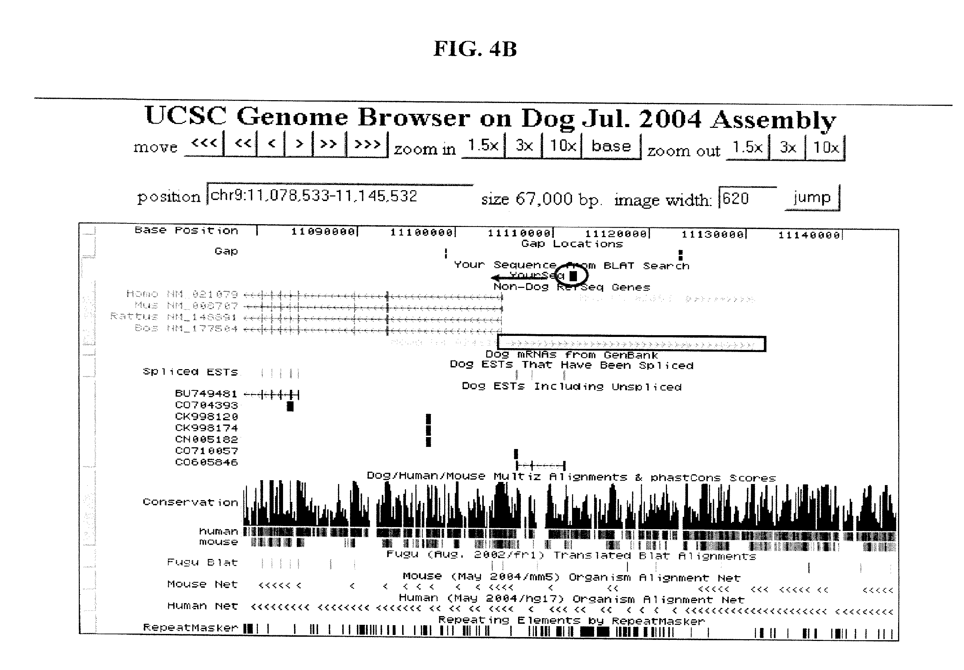

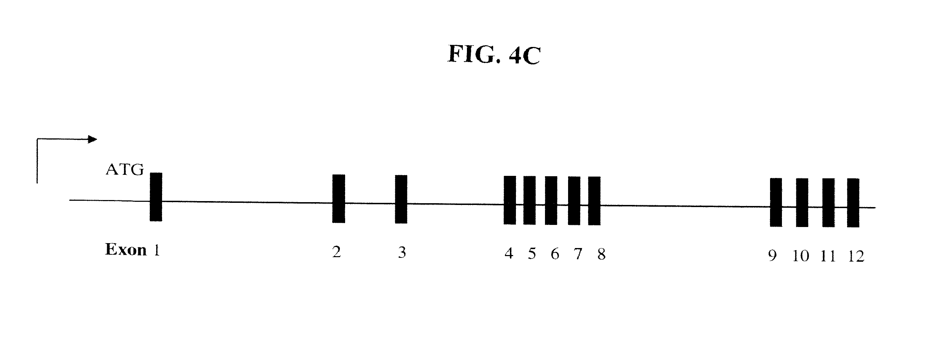

Figure 4A shows the alignment of the 5'-end flanking sequences obtained from three subclones of influenza resistant clone R26-8-11. The consensus sequence derived from the alignment (SEQ ID NO:3) was used to identify the affected gene NMT1 (SEQ ID NOS: 11 and 19). Figure 4B depicts the genomic site of RHKO integration. As shown in Figure 4C, the position of the RHKO integration indicate that the NMT1 gene is likely to be inactivated by the disruption of promoter by the RHKO construct.

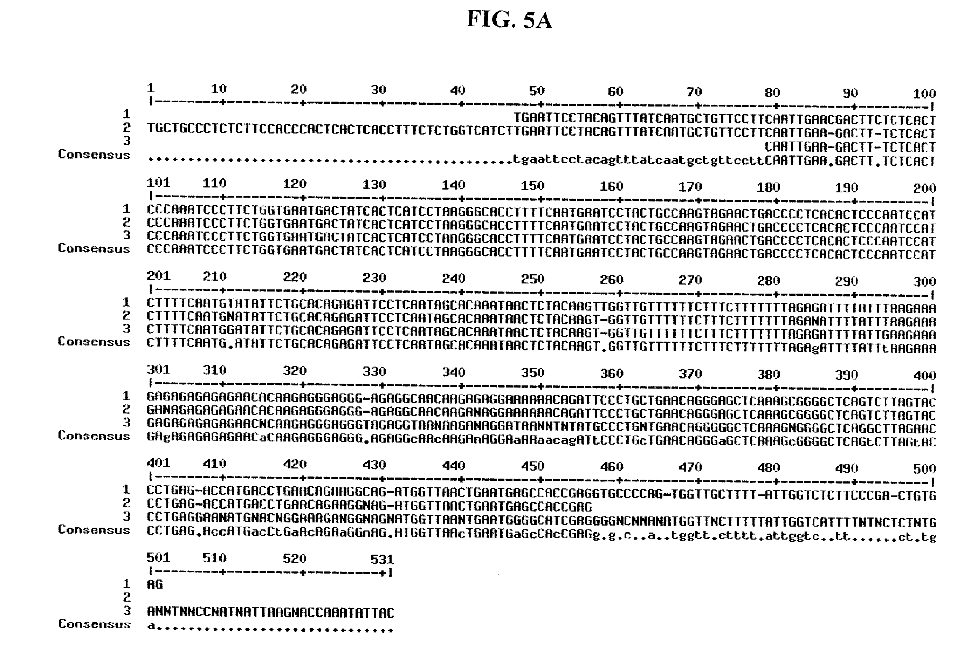

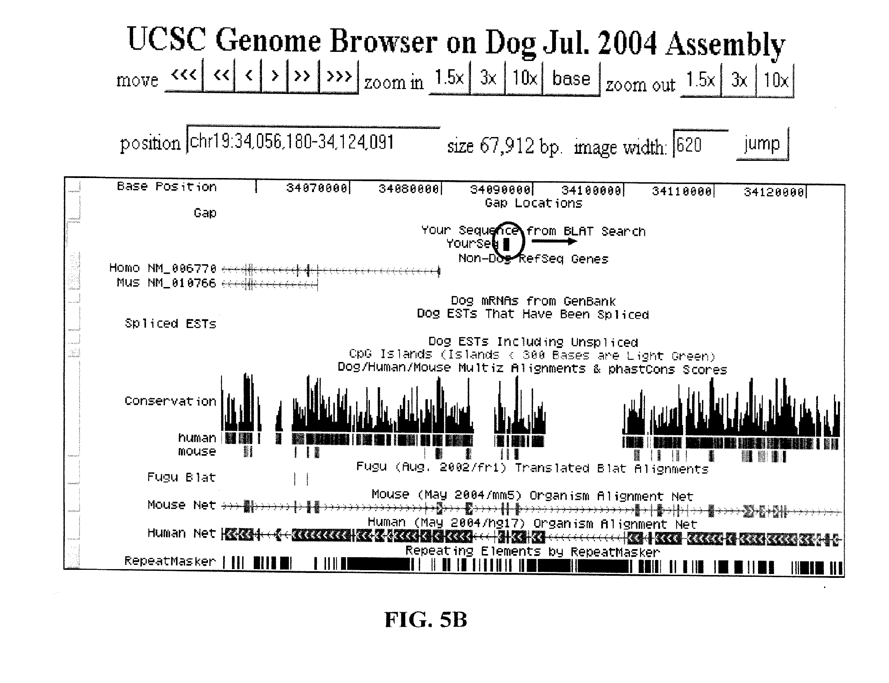

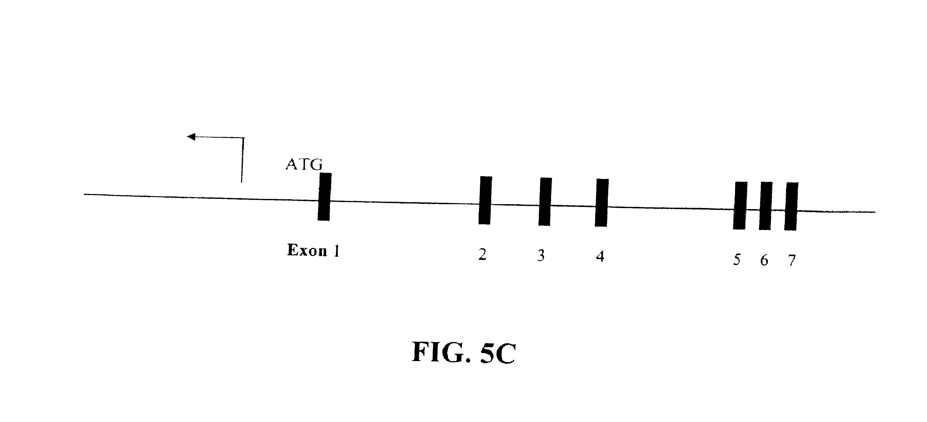

Figure 5A shows the alignment of the 5'-end flanking sequences obtained from three subclones of influenza resistant clone 26-8-11. The consensus sequence derived from the alignment (SEQ ID NO:4) was used to identify the affected gene MACRO (SEQ ID NOS: 12 and 20). Figure 5B depicts the genomic site of RHKO integration. As shown in Figure 5C, the position of the RHKO integration indicate that the MACRO gene is likely to be overexpressed due to the integration of the RHKO construct.

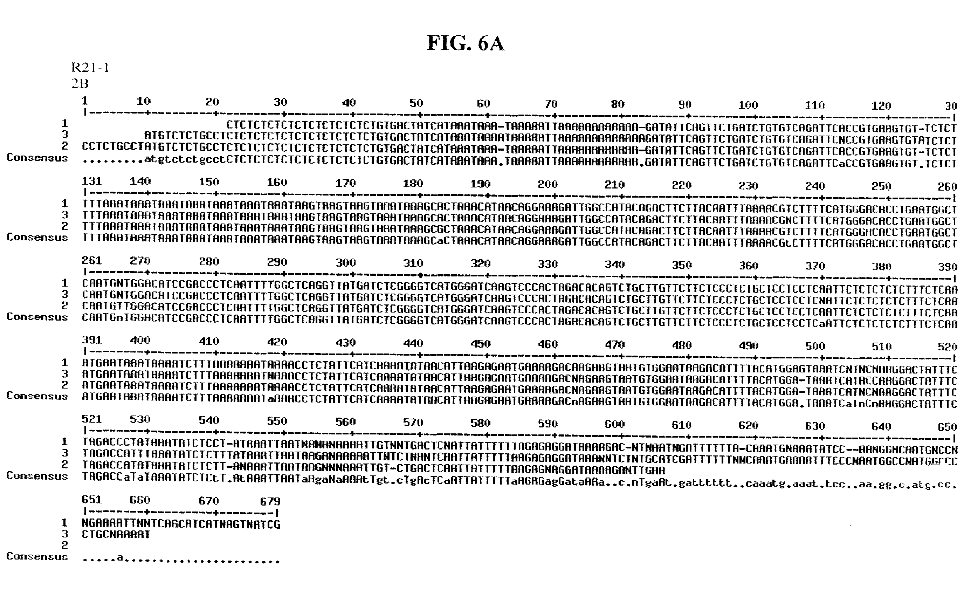

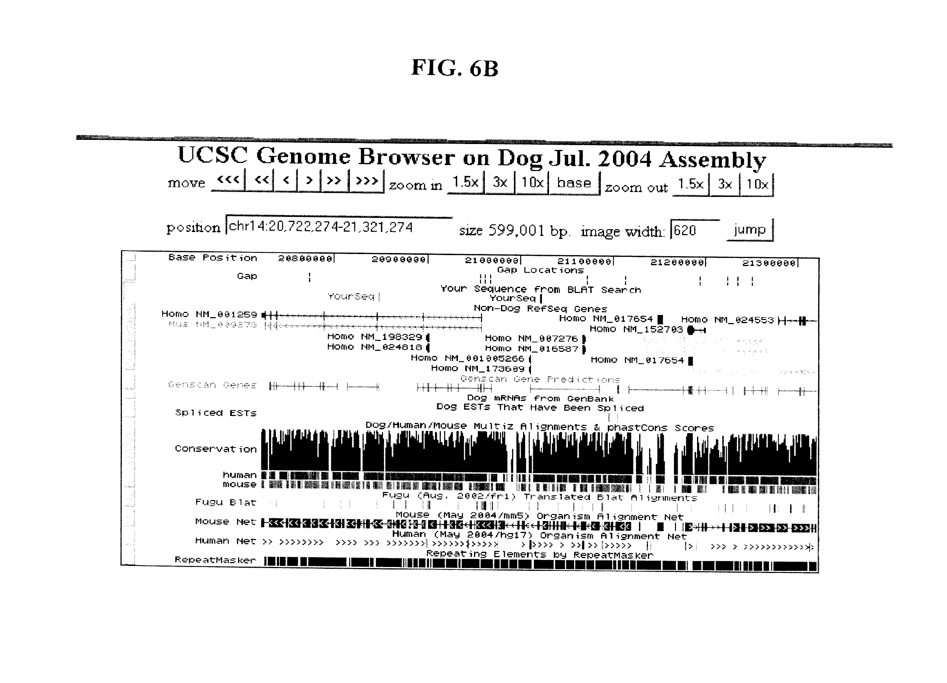

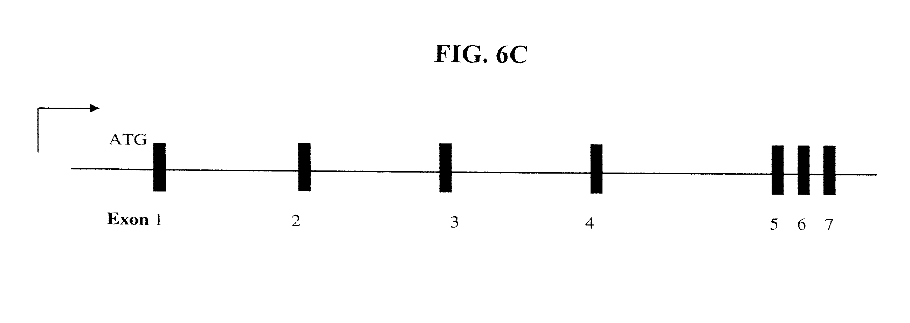

Figure 6A shows the alignment of the 5'-end flanking sequences obtained from three subclones of influenza resistant clone R21-1. The consensus sequence derived from the alignment (SEQ ID NO:5) was used to identify the affected gene CDK6 (SEQ ID NOS: 13 and 21). Figure 6B depicts the genomic site of RHKO integration. As shown in Figure 6C, the position of the RHKO integration indicate that the CDK6 gene is likely to be inactivated by the integration of the RHKO construct due to the disruption of promoter.

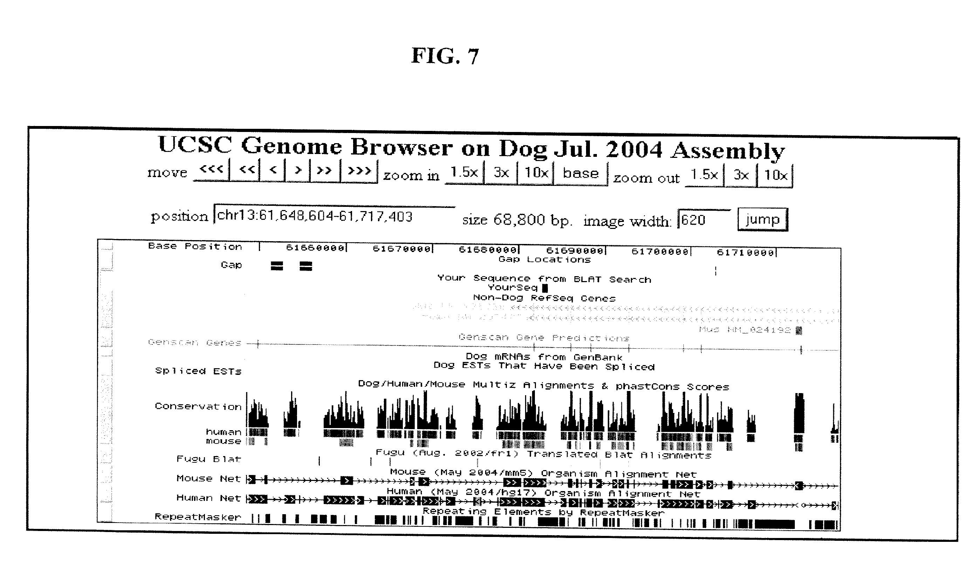

The 5'-end flanking sequence (SEQ ID NO:6) obtained from influenza resistant clone R27-32 was used to identify the affected gene FLJ16046 (SEQ ID NOS: 14 and 22). Figure 7 depicts the genomic site of RHKO integration. The position of the RHKO integration indicate that the FLJ1604 gene is likely to be overexpressed due to the integration of the RHKO construct.

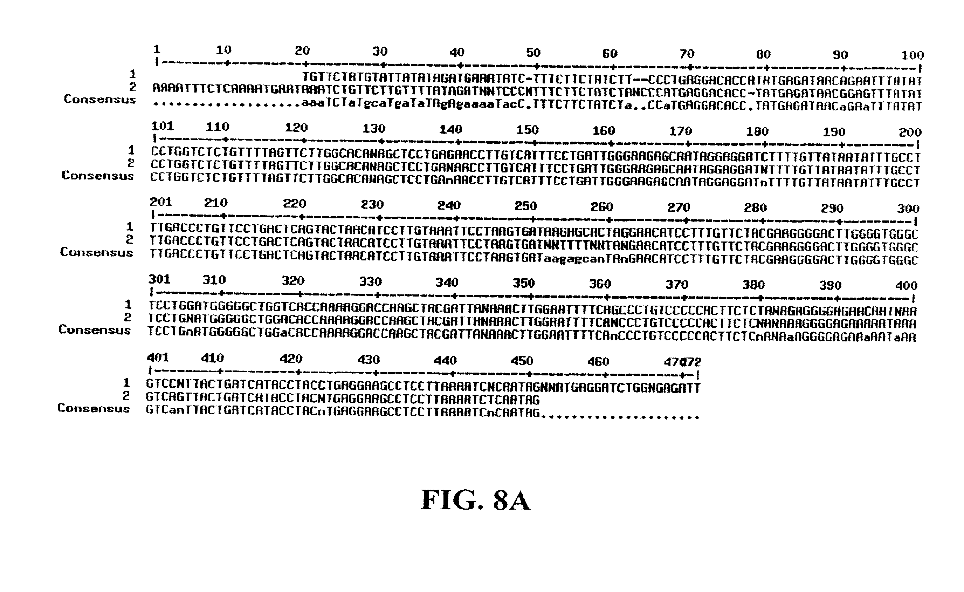

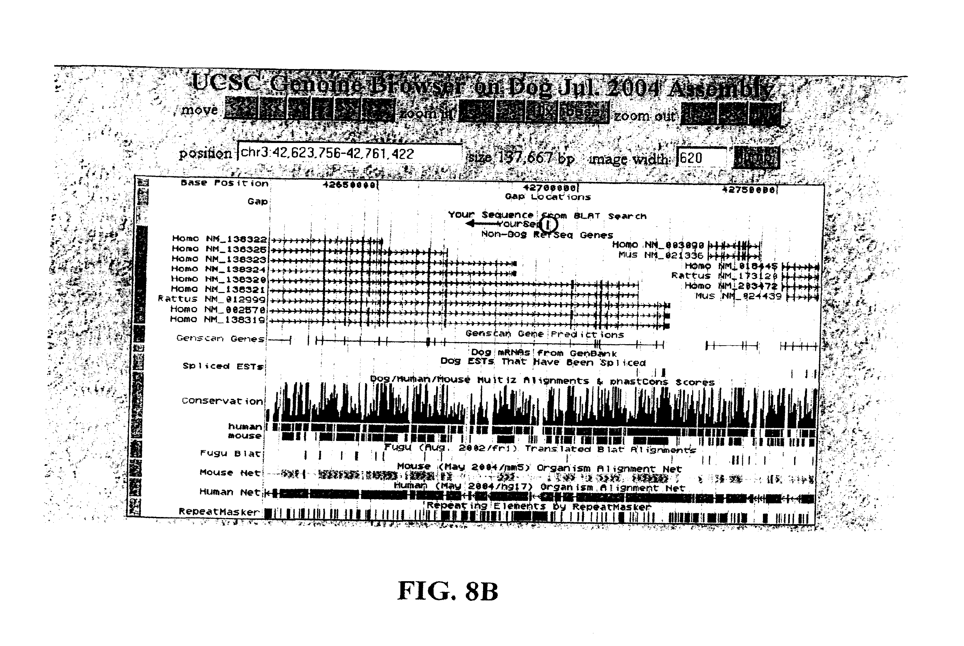

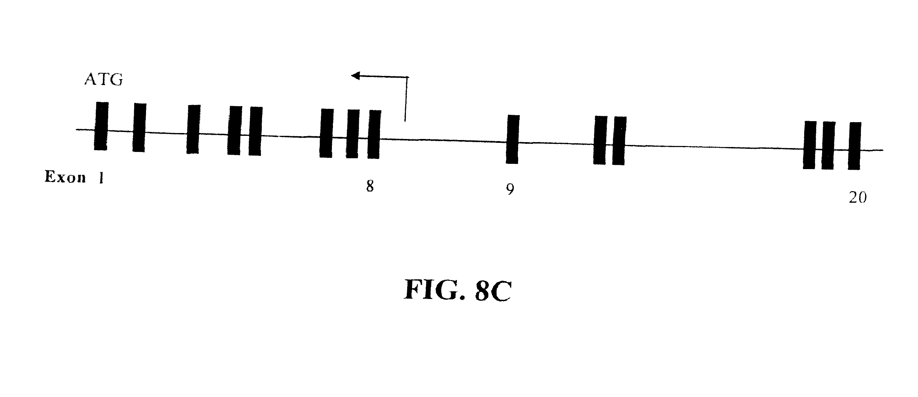

Figure 8A shows the alignment of the 5'-end flanking sequences obtained from two subclones of influenza resistant clone R27-3-33. The consensus sequence derived from the alignment (SEQ ID NO:7) was used to identify the affected gene PCSK6 (SEQ ID NOS: 15 and 23). Figure 8B depicts the genomic site of RHKO integration. As shown in Figure 8C, the position of the RHKO integration indicate that the PCSK6 gene is likely to be inactivated by the antisense transcription from the RHKO construct.

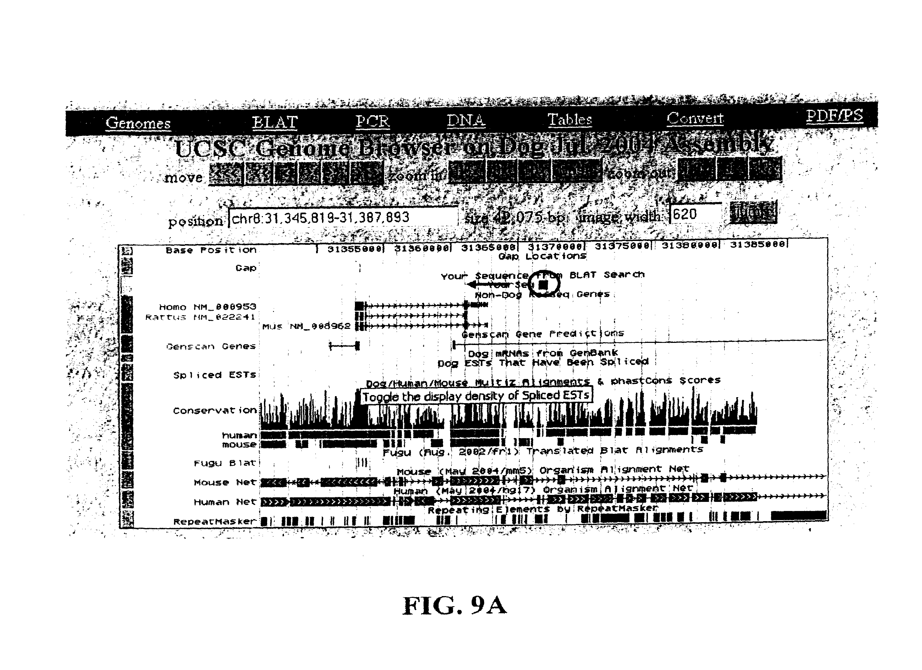

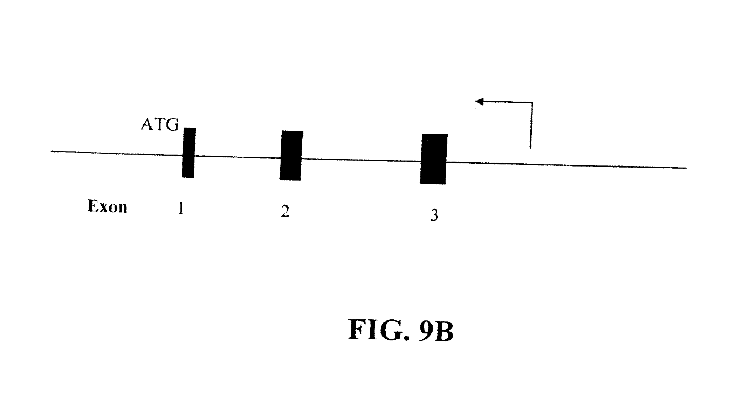

The 5'-end flanking sequence (SEQ ID NO:8) obtained from influenza resistant clone R27-3-35 was used to identify the affected gene PTGDR (SEQ ID NOS: 16 and 24). Figure 9A depicts the genomic site of RHKO integration. As shown in Figure 9B, the position of the RHKO integration indicate that the PTGDR gene is likely to be inactivated by the antisense transcription from the RHKO construct.
SEQ ID NO:1 PTCH flanking 
TAAACGTAAAAAGTAGCCAAGCGCACGGGGGAAGGGCCCCGGCCGGCGCAGGC
AGGGGTC
CCGGNTGGGCTGCGGCTGATCCCGGCNGCNGCGTGATCTCGGCGCTGGCCGCATG
CCCCG
GCGGGNCCCCGTCTGGGTGCTCGCCTTCCCCGGATTCCACNCATTGCAGCGAGCC
TCGTA
AACNCAATGAANCCGGCCGCTTGGCAGACCCGCACCGCGGANTTAANGTGGCAA
TTTGTT
TACNNCTTTCCCTCTCCCCCCAGGCTCTGGGAAGAGGNGACTCAAAAACTGAAAA
GGAAG
AGGGGAGATGCCCTCTTTNAAGGATAATTTTTAAGGGGGNNGANATTTCNAGCTC
AGCAA
AAGCAAAACCGGATGCCAAAAAAGGAAACCACCTTTATTTCNGCTNCCTCCCCCC
CTTCC

ATCTCTCCGCCTCTCTCCACTCCGCTTTCCNCCCTCAAAAGATGTTAAAAAAATGT
GGCA
GCATTTCNCGGGNNTTGGGACNGCAAANTAAGGNGCCAAGGGGCTANGNCCATC
TGGGGT
 
TCTCCNNGGGCNCGGGTNTNCCGGGTCGNTGACCTCGCGGACTGTNTGGCNNTCN
TAGNA
TGGCNCCCGCANAANCGCTNTNCANTNNTCTGTNAAAAGGNATNNCTTTTAANCN
TCCTT
ACNACCCNTCCNACCNCACCCAAATNANNTTTNTTCTTGNATATGCTGATNNATC
NCTTG
CCGATTTCTTAANCNTCTTNCCTACCCNTGNNNCAAGGGNAGGTATANNT
SEQ ID NO:9, PTCH cDNA
GCGCCCGCCGTGTGAGCAGCAGCAGCGGCTGGTCTGTCAACCGGAGCCCGAGCC
CGAGCA
GCCTGCGGCCAGCAGCGTCCTCGCAAGCCGAGCGCCCAGGCGCGCCAGGAGCCC
GCAGCA
GCGGCAGCAGCGCGCCGGGCCGCCCGGGAAGCCTCCGTCCCCGCGGCGGCGG
GCGGCG
GCGGCAACATGGCCTCGGCTGGTAACGCCGCCGAGCCCCAGGACCGCGGCGG
GCGGCA
GCGGCTGTATCGGTGCCCCGGGACGGCCGGCTGGAGGCGGAGGCGCAGACGGA
CGGGGG
GGCTGCGCCGTGCTGCCGCGCCGGACCGGGACTATCTGCACCGGCCCAGCTACTG
CGACG
CCGCCTTCGCTCTGGAGCAGATTTCCAAGGGGAAGGCTACTGGCCGGAAAGCGCC
GCTGT
GGCTGAGAGCGAAGTTTCAGAGACTCTTATTTAAACTGGGTTGTTACATTCAAAA
AAACT
GCGGCAAGTTCTTGGTTGTGGGCCTCCTCATATTTGGGGCCTTCGCGGTGGGATTA
AAAG
CAGCGAACCTCGAGACCAACGTGGAGGAGCTGTGGGTGGAAGTTGGAGGACGAG
TAAGTC
 
GTGAATTAAATTATACTCGCCAGAAGATTGGAGAAGAGGCTATGTTTAATCCTCA
ACTCA
TGATACAGACCCCTAAAGAAGAAGGTGCTAATGTCCTGACCACAGAAGCGCTCCT
ACAAC
ACCTGGACTCGGCACTCCAGGCCAGCCGTGTCCATGTATACATGTACAACAGGCA
GTGGA
AATTGGAACATTTGTGTTACAAATCAGGAGAGCTTATCACAGAAACAGGTTACAT
GGATC
AGATAATAGAATATCTTTACCCTTGTTTGATTATTACACCTTTGGACTGCTTCTGG
GAAG
GGGCGAAATTACAGTCTGGGACAGCATACCTCCTAGGTAAACCTCCTTTGCGGTG
GACAA
ACTTCGACCCTTTGGAATTCCTGGAAGAGTTAAAGAAAATAAACTATCAAGTGGA
CAGCT
GGGAGGAAATGCTGAATAAGGCTGAGGTTGGTCATGGTTACATGGACCGCCCCT
GCCTCA
ATCCGGCCGATCCAGACTGCCCCGCCACAGCCCCCAACAAAAATTCAACCAAACC
TCTTG
ATATGGCCCTTGTTTTGAATGGTGGATGTCATGGCTTATCCAGAAAGTATATGCA
CTGGC
AGGAGGAGTTGATTGTGGGTGGCACAGTCAAGAACAGCACTGGAAAACTCGTCA
GCGCCC
ATGCCCTGCAGACCATGTTCCAGTTAATGACTCCCAAGCAAATGTACGAGCACTT
CAAGG
GGTACGAGTATGTCTCACACATCAACTGGAACGAGGACAAAGCGGCAGCCATCC
TGGAGG
CCTGGCAGAGGACATATGTGGAGGTGGTTCATCAGAGTGTCGCACAGAACTCCAC
TCAAA
 
AGGTGCTTTCCTTCACCACCACGACCCTGGACGACATCCTGAAATCCTTCTCTGAC
GTCA
GTGTCATCCGCGTGGCCAGCGGCTACTTACTCATGCTCGCCTATGCCTGTCTAACC
ATGC
TGCGCTGGGACTGCTCCAAGTCCCAGGGTGCCGTGGGGCTGGCTGGCGTCCTGCT
GGTTG
CACTGTCAGTGGCTGCAGGACTGGGCCTGTGCTCATTGATCGGAATTTCCTTTAAC
GCTG
CAACAACTCAGGTTTTGCCATTTCTCGCTCTTGGTGTTGGTGTGGATGATGTTTTT
CTTC
TGGCCCACGCCTTCAGTGAAACAGGACAGAATAAAAGAATCCCTTTTGAGGACA
GGACCG
GGGAGTGCCTGAAGCGCACAGGAGCCAGCGTGGCCCTCACGTCCATCAGCAATG
TCACAG
CCTTCTTCATGGCCGCGTTAATCCCAATTCCCGCTCTGCGGGCGTTCTCCCTCCAG
GCAG
CGGTAGTAGTGGTGTTCAATTTTGCCATGGTTCTGCTCATTTTTCCTGCAATTCTC
AGCA
TGGATTTATATCGACGCGAGGACAGGAGACTGGATATTTCTGCTGTTTTACAAG
CCCCT
GCGTCAGCAGAGTGATTCAGGTTGAACCTCAGGCCTACACCGACACACACGACA
ATACCC
GCTACAGCCCCCCACCTCCCTACAGCAGCCACAGCTTTGCCCATGAAACGCAGAT
TACCA
TGCAGTCCACTGTCCAGCTCCGCACGGAGTACGACCCCCACACGCACGTGTACTA
CACCA
CCGCTGAGCCGCGCTCCGAGATCTCTGTGCAGCCCGTCACCGTGACACAGGACAC
CCTCA
 
GCTGCCAGAGCCCAGAGAGCACCAGCTCCACAAGGGACCTGCTCTCCCAGTTCTC
CGACT
CCAGCCTCCACTGCCTCGAGCCCCCCTGTACGAAGTGGACACTCTCATCTTTTGCT
GAGA
AGCACTATGCTCCTTTCCTCTTGAAACCAAAAGCCAAGGTAGTGGTGATCTTCCTT
TTTC
TGGGCTTGCTGGGGGTCAGCCTTTATGGCACCACCCGAGTGAGAGACGGGCTGGA
CCTTA
CGGACATTGTACCTCGGGAAACCAGAGAATATGACTTTATTGCTGCACAATTCAA
ATACT
TTTCTTTCTACAACATGTATATAGTCACCCAGAAAGCAGACTACCCGAATATCCA
GCACT
TACTTTACGACCTACACAGGAGTTTCAGTAACGTGAAGTATGTCATGTTGGAAGA
AAACA
AACAGCTTCCCAAAATGTGGCTGCACTACTTCAGAGACTGGCTTCAGGGACTTCA
GGATG
CATTTGACAGTGACTGGGAAACCGGGAAAATCATGCCAAACAATTACAAGAATG
GATCAG
ACGATGGAGTCCTTGCCTACAAACTCCTGGTGCAAACCGGCAGCCGCGATAAGCC
CATCG
ACATCAGCCAGTTGACTAAACAGCGTCTGGTGGATGCAGATGGCATCATTAATCC
CAGCG
CTTTCTACATCTACCTGACGGCTTGGGTCAGCAACGACCCCGTCGCGTATGCTGCC
TCCC
AGGCCAACATCCGGCCACACCGACCAGAATGGGTCCACGACAAAGCCGACTACA
TGCCTG
AAACAAGGCTGAGAATCCCGGCAGCAGAGCCCATCGAGTATGCCCAGTTCCCTTT
CTACC
 
TCAACGGCTTGCGGACACCTCAGACTTTGTGGAGGCAATTGAAAAGTAAGGA
CCATCT
GCAGCAACTATACGAGCCTGGGGCTGTCCAGTTACCCCAACGGCTACCCCTTCCT
CTTCT
GGGAGCAGTACATCGGCCTCCGCCACTGGCTGCTGCTGTTCATCAGCGTGGTGTT
GGCCT
GCACATTCCTCGTGTGCGCTGTCTTCCTTCTGAACCCCTGGACGGCCGGGATCATT
GTGA
TGGTCCTGGCGCTGATGACGGTCGAGCTGTTCGGCATGATGGGCCTCATCGGAAT
CAAGC
TCAGTGCCGTGCCCGTGGTCATCCTGATCGCTTCTGTTGGCATAGGAGTGGAGTTC
ACCG
TTCACGTTGCTTTGGCCTTTCTGACGGCCATCGGCGACAAGAACCGCAGGGCTGT
GCTTG
CCCTGGAGCACATGTTTGCACCCGTCCTGGATGGCGCCGTGTCCACTCTGCTGGG
AGTGC
TGATGCTGGCGGGATCTGAGTTCGACTTCATTGTCAGGTATTTCTTTGCTGTGCTG
GCGA
TCCTCACCATCCTCGGCGTTCTCAATGGGCTGGTTTTGCTTCCCGTGCTTTTGTCTT
TCT
TTGGACCATATCCTGAGGTGTCTCCAGCCAACGGCTTGAACCGCCTGCCCACACC
CTCCC
CTGAGCCACCCCCCAGCGTGGTCCGCTTCGCCATGCCGCCCGGCCACACGCACAG
CGGGT
CTGATTCCTCCGACTCGGAGTATAGTTCCCAGACGACAGTGTCAGGCCTCAGCGA
GGAGC
TTCGGCACTACGAGGCCCAGCAGGGCGCGGGAGGCCCTGCCCACCAAGTGATCG
TGGAAG
 
CCACAGAAAACCCCGTCTTCGCCCACTCCACTGTGGTCCATCCCGAATCCAGGCA
TCACC
CACCCTCGAACCCGAGACAGCAGCCCACCTGGACTCAGGGTCCCTGCCTCCCGG
ACGGC
AAGGCCAGCAGCCCCGCAGGGACCCCCCCAGAGAAGGCTTGTGGCCACCCCTCT
ACAGAC
CGCGCAGAGACGCTTTTGAAATTTCTACTGAAGGGCATTCTGGCCCTAGCAATAG
GGCCC
GCTGGGGCCCTCGCGGGGCCCGTTCTCACAACCCTCGGAACCCAGCGTCCACTGC
CATGG
GCAGCTCCGTGCCCGGCTACTGCCAGCCCATCACCACTGTGACGGCTTCTGCCTC
CGTGA
CTGTCGCCGTGCACCCGCCGCCTGTCCCTGGGCCTGGGCGGAACCCCCGAGGGGG
ACTCT
GCCCAGGCTACCCTGAGACTGACCACGGCCTGTTTGAGGACCCCCACGTGCCTTT
CCACG
TCCGGTGTGAGAGGAGGGATTCGAAGGTGGAAGTCATTGAGCTGCAGGACGTGG
AATGCG
AGGAGAGGCCCCGGGGAAGCAGCTCCAACTGAGGGTGATTAAAATCTGAAGCAA
AGAGGC
CAAAGATTGGAAACCCCCCACCCCCACCTCTTTCCAGAACTGCTTGAAGAGAACT
GGTTG
GAGTTATGGAAAAGATGCCCTGTGCCAGGACAGCAGTTCATTGTTACTGTAACCG
ATTGT
ATTATTTTGTTAAATATTTCTATAAATATTTAAGAGATGTACACATGTGTAATATA
GGAA
GGAAGGATGTAAAGTGGTATGATCTGGGGCTTCTCCACTCCTGCCCCAGAGTGTG
GAGGC
 
CACAGTGGGGCCTCTCCGTATTTGTGCATTGGGCTCCGTGCCACAACCAAGCTTC
ATTAG
TCTTAAATTTCAGCATATGTTGCTGCTGCTTAAATATTGTATAATTTACTTGTATA
ATTC
TATGCAAATATTGCTTATGTAATAGGATTATTTTGTAAAGGTTTCTGTTTAAAATA
TTTT
AAATTTGCATATCACAACCCTGTGGTAGTATGAAATGTTACTGTTAACTTTCAAAC
ACGC
TATGCGTGATAATTTTTTTGTTTAATGAGCAGATATGAAGAAAGCACGTTAATCCT
GGTG
GCTTCTCTAGGTGTCGTTGTGTGCGGTCCTCTTGTTTGGCTGTGCGTGTGAACACG
TGTG
TGAGTTCACCATGTACTGTACTGTGATTTTTTTTTGTCTTGTTTTGTTTCTCTACA
CTG
TCTGTAACCTGTAGTAGGCTCTGACCTAGTCAGGCTGGAAGCGTCAGGATATCTT
TTCTT
CGTGCTGGTGAGGGCTGGCCCTAAACATCCACCTAATCCTTTCAAATCAGCCCGG
CAAAA
GCTAGACTCTCCTCGTGTCTACGGCATCTCTTATGATCATTGGCTGCCATCCAGGA
CCCC
AATTTGTGCTTCAGGGGGATAATCTCCTTCTCTCGGATCATTGTGATGGATGCTGG
AACC
TCAGGGTATGGAGCTCACATCAGTTCATCATGGTGGGTGTTAGAGAATTCGGTGA
CATGC
CTAGTGCTGAGCCTTGGCTGGGCCATGAGAGTCTGTATACTCTAAAAAGCATGCA
GCATG
GTGCCCCTCTTCTGACCAACACACACACGACCCCTCCCCCAACACCCCCAAATTC
AAGAG
 
TGGATGTGGCCCTGTCACAGGTAGAAAAACCTATTTAGTTAATTCTTTCTTGGCCC
ACAG
TCTCCCAGAAATGATGTTTTGAGTCCCTATAGTTTAAACTCCCTCTCTTAAATGGA
GCAG
CTGGTTGAGGCTTTCTAGATCTGTTTGCATCTTCTTTAAAACTAAGTGGTGAGCAT
GCAT
TGTGGTGTAGAGGCAGGCATTATGTAGGATAAGAGCTCCGGGGGGATTCTTCATG
CACCA
GTGTTTAGGGTACGTGCTTCCTAAGTAAATCCAAACATTGTCTCCATCCTCCCCGT
CATT
AGTGCTCTTTCAATGTGATGTGGGAAAGCAGGAGGATGGACACACCCCACTGAA
AGATGT
AGGCAGGGGCAGGTCTCTCAACCAGGCATATTTTTAAAAGTTGCTTCTGTACTGG
TTCTC
TTCTTTTGCTCTGAGGTGTGGGCTCCCTCATCTCGTAACCAGAGACCAGCACATGT
CAGG
GAAGCACCCAGTGTCGGCTCCCCATCCAAATCCACACCAGCACCTTGTTACAGAC
AAGAA
GTCAGAGGAAAGGGCGGGGTCCCTGCAGGGCTGAAGCCTAAGCTACTGTGAGGC
GCTCAC
GAGTGGCAGCTCCTGTTACTCCCTTTTAAATTACCTGGGAAATCTTAACAGAAAG
GTAAT
GGGCCCCCAGAAATACCCACAGCATAGTGACCTCAGACCCTGATACTCACCACAA
AACTT
TTAAGATGCTGATTGGGAGCCGCTTGTGGCTGCTGGGTGTGTGTGTGTGTGTGTG
CGTGC
GTGCGTGTGTGTGTGTCTCTGCTGGGGACCCTGGCCACCCCCCTGCTGCTGTCTTG
GTGC
 
CTGTCACCCACATGGTCTGCCATCCTAACACCCAGCTCTGCTCAGAAAACGTCCT
GCGTG
GAGGAGGGATGATGCAGAATTCTGAAGTCGACTTCCCTCTGGCTCCTGGCGTGCC
CTCGC
TCCCTTCCTGAGCCCAGCTCGTGTTGCGCCGGAGGCTGCGCGGCCCCTGATTTCTG
CATG
GTGTAGAACTTTCTCCAATAGTCACATTGGCAAAGGGAGAACTGGGGTGGGCGG
GGGGTG
GGGCTGGCAGGGAATTAGAATTTCTCTCTCTCTTTTAATAGTTTTATTTTGTCTGTC
CTG
TTTGTTCATTTGGATGTTTTAATTTTTAAAAAAAAAAAAAAAAAA
SEQ ID NO:17, PTCH protein 
MASAGNAAEPQDRGGGGSGCIGAPGRPAGGGRRRRTGGLRRAAAPDRDYL
HRPSYCDAAFALEQISKGKATGRKAPLWLRAKFQRLLFKLGCYIQKNCGK
FLVVGLLIFGAFAVGLKAANLETNVEELWVEVGGRVSRELNYTRQKIGEE
AMFNPQLMIQTPKEEGANVLTTEALLQHLDSALQASRVHVYMYNRQWKLE
HLCYKSGELITETGYMDQIIEYLYPCLIITPLDCFWEGAKLQSGTAYLLG
KPPLRWTNFDPLEFLEELKKINYQVDSWEEMLNKAEVGHGYMDRPCLNPA
DPDCPATAPNKNSTKPLDMALVLNGGCHGLSRKYMHWQEELIVGGTVKS
TGKLVSAHALQTMFQLMTPKQMYEHFKGYEYVSHINWNEDKAAAILEAWQ
RTYVEVVHQSVAQNSTQKVLSFTTTTLDDILKSFSDVSVIRVASGYLLML
AYACLTMLRWDCSKSQGAVGLAGVLLVALSVAAGLGLCSLIGISFNAATT
QVLPFLALGVGVDDVFLLAHAFSETGQNKRIPFEDRTGECLKRTGASVAL
TSISNVTAFFMAALIPIPALRAFSLQAAVVVVFNFAMVLLIFPAILSMDL
YRREDRRLDIFCCFTSPCVSRVIQVEPQAYTDTHDNTRYSPPPPYSSHSF
AHETQITMQSTVQLRTEYDPHTHVYYTTAEPRSEISVQPVTVTQDTLSCQ
SPESTSSTRDLLSQFSDSSLHCLEPPCTKWTLSSFAEKHYAPFLLKPKAK
VVVIFLFLGLLGVSLYGTTRVRDGLDLTDIVPRETREYDFIAAQFKYFSF
 
YNMYIVTQKADYPNIQHLLYDLHRSFSNVKYVMLEENKQLPKMWLHYFRD
WLQGLQDAFDSDWETGKIMPNNYKNGSDDGVLAYKLLVQTGSRDKPIDIS
QLTKQRLVDADGIINPSAFYIYLTAWVSNDPVAYAASQANIRPHRPEWVH
DKADYMPETRLRIPAAEPIEYAQFPFYLNGLRDTSDFVEAIEKVRTICSN
YTSLGLSSYPNGYPFLFWEQYIGLRHWLLLFISVVLACTFLVCAVFLLNP
WTAGIIVMVLALMTVELFGMMGLIGIKLSAVPVVILIASVGIGVEFTVHV
ALAFLTAIGDKNRRAVLALEHMFAPVLDGAVSTLLGVLMLAGSEFDFIVR
YFFAVLAILTILGVLNGLVLLPVLLSFFGPYPEVSPANGLNRLPTPSPEP
PPSVVRFAMPPGHTHSGSDSSDSEYSSQTTVSGLSEELRHYEAQQGAGGP
AHQVIVEATENPVFAHSTVVHPESRHHPPSNPRQQPHLDSGSLPPGRQGQ
QPRRDPPREGLWPPLYRPRRDAFEISTEGHSGPSNRARWGPRGARSHNPR
NPASTAMGSSVPGYCQPITTVTASASVTVAVHPPPVPGPGRNPRGGLCPG
YPETDHGLFEDPHVPFHVRCERRDSKVEVIELQDVECEERPRGSSSN
SEQ ID NO:2 PSMD2-flanking 
CTTCTTCNTGACTCCTGGATTTCCTCTGTTCNCAACGGACACAGCCTTACCAAAT
TCAA
ACGGCCGAGAGGACGTTATGTATCATCTAGAACTAATCCTGACTTCAACAGTGTC
CTTCA
CACCCCTTCTAAGTCAAATCACGGAAAGACTCAAAAGACAGAGATTGAAGAAGG
CAAAGC
CTGTGTCTTGATCTGCCTTTAGTTCTAGAGTTTAGCATCNGAGCATANGACCACAT
TGTA
TTGATGGACTCCGACCAGGNTCCGCAGGNGGATTTAAGGTGGGGGCCGTACGCG
GCAGGT
GGTACCCGACCACTCTCCTTCACCNNGGGGTAAAACGTTACGAGGTTAATATTCC
GCGGC
GGCGGAAGTAGATACAGGTTGCAGATCTCACACGGGCGGCGATCAAGCATTCCG
GACGTG
 
AAGAGTCTCGTTCGTCTGTCCCACCACGCAGCCGACTGCGGTGTCACTGTGGGTA
CCGGT
CGCTCGGCNAGTAAGGAGACCCCGCGGGCGGNCCCTCGGNTCGCGGCTCTTCATC
TCCTA
CCGCAGCCAGCGGACTCGGATCNCAGACTGCACGGCCNCATGGCCTTCCGGAAA
CTCCCG
GTCCGAGCCGGGGCGGCGCCTGGGGCGNATNAACNGTTAGAACTTGCAGTTTTG
GGGGCG
GNCTCCGAGGGNGGGGTCCAGGGCCCGGGCCTCNCGAAA
SEQ ID NO:10, PSMD2-cDNA 
TGCGCGCGCAGCGGGCCGGCAGTGGCGGCGGAGATGGAGGAGGGAGGCCGGGA
CAAGGCG
CCGGTGCAGCCCCAGCAGTCTCCAGCGGCGGCCCCCGGCGGCACGGACGAGAAG
CCGAGC
GGCAAGGAGCGGCGGGATGCCGGGGACAAGGACAAAGAACAGGAGCTGTCTGA
AGAGGAT
AAACAGCTTCAAGATGAACTGGAGATGCTCGTGGAACGACTAGGGGAGAAGGAT
ACATCC
CTGTATCGACCAGCGCTGGAGGAATTGCGAAGGCAGATTCGTTCTTCTACAACTT
CCATG
ACTTCAGTGCCCAAGCCTCTCAAATTTCTGCGTCCACACTATGGCAAACTGAAGG
AAATC
TATGAGAACATGGCCCCTGGGGAGAATAAGCGTTTTGCTGCTGACATCATCTCCG
TTTTG
GCCATGACCATGAGTGGGGAGCGTGAGTGCCTCAAGTATCGGCTAGTGGGCTCCC
AGGAG
GAATTGGCATCATGGGGTCATGAGTATGTCAGGCATCTGGCAGGAGAAGTGGCT
AAGGAG
 
TGGCAGGAGCTGGATGACGCAGAGAAGGTCCAGCGGGAGCCTCTGCTCACTCTG
GTGAAG
GAAATCGTCCCCTATAACATGGCCCACAATGCAGAGCATGAGGCTTGCGACCTGC
TTATG
GAAATTGAGCAGGTGGACATGCTGGAGAAGGACATTGATGAAAATGCATATGCA
AAGGTC
TGCCTTTATCTCACCAGTTGTGTGAATTACGTGCCTGAGCCTGAGAACTCAGCCCT
ACTG
CGTTGTGCCCTGGGTGTGTTCCGAAAGTTTAGCCGCTTCCCTGAAGCTCTGAGATT
GGCA
TTGATGCTCAATGACATGGAGTTGGTAGAAGACATCTTCACCTCCTGCAAGGATG
TGGTA
GTACAGAAACAGATGGCATTCATGCTAGGCCGGCATGGGGTGTTCCTGGAGCTGA
GTGAA
GATGTCGAGGAGTATGAGGACCTGACAGAGATCATGTCCAATGTACAGCTCAAC
AGCAAC
TTCTTGGCCTTAGCTCGGGAGCTGGACATCATGGAGCCCAAGGTGCCTGATGACA
TCTAC
AAAACCCACCTAGAGAACAACAGGTTTGGGGGCAGTGGCTCTCAGGTGGACTCT
GCCCGC
ATGAACCTGGCCTCCTCTTTTGTGAATGGCTTTGTGAATGCAGCTTTTGGCCAAGA
CAAG
CTGCTAACAGATGATGGCAACAAATGGCTTTACAAGAACAAGGACCACGGAATG
TTGAGT
GCAGCTGCATCTCTTGGGATGATTCTGCTGTGGGATGTGGATGGTGGCCTCACCC
AGATT
GACAAGTACCTGTACTCCTCTGAGGACTACATTAAGTCAGGAGCTCTTCTTGCCT
GTGGC
 
ATAGTGAACTCTGGGGTCCGGAATGAGTGTGACCCTGCTCTGGCACTGCTCTCAG
ACTAT
GTTCTCCACAACAGCAACACCATGAGACTTGGTTCCATCTTTGGGCTAGGCTTGG
CTTAT
GCTGGCTCAAATCGTGAAGATGTCCTAACACTGCTGCTGCCTGTGATGGGAGATT
CAAAG
TCCAGCATGGAGGTGGCAGGTGTCACAGCTTTAGCCTGTGGAATGATAGCAGTAG
GGTCC
TGCAATGGAGATGTAACTTCCACTATCCTTCAGACCATCATGGAGAAGTCAGAGA
CTGAG
CTCAAGGATACTTATGCTCGTTGGCTTCCTCTTGGACTGGGTCTCAACCACCTGGG
GAAG
GGTGAGGCCATCGAGGCAATCCTGGCTGCACTGGAGGTTGTGTCAGAGCCATTCC
GCAGT
TTTGCCAACACACTGGTGGATGTGTGTGCATATGCAGGCTCTGGGAATGTGCTGA
AGGTG
CAGCAGCTGCTCCACATTTGTAGCGAACACTTTGACTCCAAAGAGAAGGAGGAA
GACAAA
GACAAGAAGGAAAAGAAAGACAAGGACAAGAAGGAAGCCCCTGCTGACATGGG
AGCACAT
CAGGGAGTGGCTGTTCTGGGGATTGCCCTTATTGCTATGGGGGAGGAGATTGGTG
CAGAG
ATGGCATTACGAACCTTTGGCCACTTGCTGAGATATGGGGAGCCTACACTCCGGA
GGGCT
GTACCTTTAGCACTGGCCCTCATCTCTGTTTCAAATCCACGACTCAACATCCTGGA
TACC
CTAAGCAAATTCTCTCATGATGCTGATCCAGAAGTTTCCTATAACTCCATTTTTGC
CATG
 
GGCATGGTGGGCAGTGGTACCAATAATGCCCGTCTGGCTGCAATGCTGCGCCAGT
TAGCT
CAATATCATGCCAAGGACCCAAACAACCTCTTCATGGTGCGCTTGGCACAGGGCC
TGACA
CATTTAGGGAAGGGCACCCTTACCCTCTGCCCCTACCACAGCGACCGGCAGCTTA
TGAGC
CAGGTGGCCGTGGCTGGACTGCTCACTGTGCTTGTCTCTTTCCTGGATGTTCGAAA
CATT
ATTCTAGGCAAATCACACTATGTATTGTATGGGCTGGTGGCTGCCATGCAGCCCC
GAATG
CTGGTTACGTTTGATGAGGAGCTGCGGCCATTGCCAGTGTCTGTCCGTGTGGGCC
AGGCA
GTGGATGTGGTGGGCCAGGCTGGCAAGCCGAAGACTATCACAGGGTTCCAGACG
CATACA
ACCCCAGTGTTGTTGGCCCACGGGGAACGGGCAGAATTGGCCACTGAGGAGTTTC
TTCCT
GTTACCCCCATTCTGGAAGGTTTTGTTATCCTTCGGAAGAACCCCAATTATGATCT
CTAA
GTGACCACCAGGGGCTCTGAACTGCAGCTGATGTTATCAGCAGGCCATGCATCCT
GCTGC
CAAGGGTGGACACGGCTGCAGACTTCTGGGGGAATTGTCGCCTCCTGCTCTTTTG
TTACT
GAGTGAGATAAGGTTGTTCAATAAAGACTTTTATCCCCAAGGAAAAAAAAAAAA
AAAAAA
AAAAAAAAAAAAAAAAAAAAAAAAAAAAAAAAAAAAAAAAAAAAAAAAAAAA
SEQ ID NO; 18, PSDM2 protein 
MEEGGRDKAPVQPQQSPAAAPGGTDEKPSGKERRDAGDKDKEQELSEEDK
QLQDELEMLVERLGEKDTSLYRPALEELRRQIRSSTTSMTSVPKPLKFLR
 
PHYGKLKEIYENMAPGENKRFAADIISVLAMTMSGERECLKYRLVGSQEE
LASWGHEYVRHLAGEVAKEWQELDDAEKVQREPLLTLVKEIVPYNMAHNA
EHEACDLLMEIEQVDMLEKDIDENAYAKVCLYLTSCVNYVPEPENSALLR
CALGVFRKFSRFPEALRLALMLNDMELVEDIFTSCKDVVVQKQMAFMLGR
HGVFLELSEDVEEYEDLTEIMSNVQLNSNFLALARELDIMEPKVPDDIYK
THLENNRFGGSGSQVDSARMNLASSFVNGFVNAAFGQDKLLTDDGNKWLY
KNKDHGMLSAAASLGMILLWDVDGGLTQIDKYLYSSEDYIKSGALLACGI
VNSGVRNECDPALALLSDYVLHNSNTMRLGSIFGLGLAYAGSNREDVLTL
LLPVMGDSKSSMEVAGVTALACGMIAVGSCNGDVTSTILQTIMEKSETEL
KDTYARWLPLGLGLNHLGKGEAIEAILAALEVVSEPFRSFANTLVDVCAY
AGSGNVLKVQQLLHICSEHFDSKEKEEDKDKKEKKDKDKKEAPADMGAHQ
GVAVLGIALIAMGEEIGAEMALRTFGHLLRYGEPTLRRAVPLALALISVS
NPRLNILDTLSKFSHDADPEVSYNSIFAMGMVGSGTNNARLAAMLRQLAQ
YHAKDPNNLFMVRLAQGLTHLGKGTLTLCPYHSDRQLMSQVAVAGLLTVL
VSFLDVRNIILGKSHYVLYGLVAAMQPRMLVTFDEELRPLPVSVRVGQAV
DVVGQAGKPKTITGFQTHTTPVLLAHGERAELATEEFLPVTPILEGFVIL
RKNPNYDL
SEQ ID NO:3, NMT1 flanking 
GTCTCCAGTTTAGGGAACCATGGGGGAAGGAAGAAAAGTCGCGCANTATCATGC
CATCCT
GCGTTTGCGCNAATGGATGGGTGGGAATCCCATGCTGCCACNNANGNCCGGGGG
AAAAGA
GGTGTTTCTCTTAAAATTTTNTANCCGGTCNAGCCNCTGGGGAAAATGTAAGGG
GAGGC
NAAGCCTTCTGAAAAGTGGAGATGATNACTCAGCGAAACAAAAGTACNCATTNA
ANCACT
TTTAATTCACTCTATGANATAGGTACCATTCCCGNTTTCCAGATGAGCAAACTGA
GAGTC
 
AGAAAGGTACGCAAGTTGACNGAAATGGAAAGGNCNNATGTTAGATNCAAAAAT
AAANGA
GATCTGGGCAGCGGTGGNTCAGCGNCTTANCGCCGCCTTNAGCCCAGGGCATGAT
CCTGG
GGTCCCGGGATCGAGTCCCACGTCGGGCTCCCTGCATGGAGCCTGCTTCTCCCTCT
GCCT
GTGTCTCTCTCTGNGNCTATCANGAAATAAATAAGNTNNTAANATATCANATNTT
AAAAA
AATNNTCTCCCTCAGNATCTGCCCCCNNAGTTTCTTGAGTCCTAGNGGNCTTTTG
GNAC
TGGAACCTGCCTGTATCTTCAACCCACCTTTCTCAAATCNNNAGNTGNAAANNAG
GNAAN
GGAACNCCTNCCTNAACCGGGTGCCNTTNAGGGCTGATGACCCACNGTATTCCAG
GCNNT
TTTACCCANGGGNTTGNNTCCAAANATCCNTGCTCCAACAATTNNANTNAAAGGN
TTGAA
SEQ ID NO:11. (NMT1) cDNA 
CTGCTCTCGCAACTCAAGATGGCGGACGAGAGTGAGACAGCAGTGAAGCCGCCG
GCACCT
CCGCTGCCGCAGATGATGGAAGGGAACGGAACGGCCATGAGCACTGCAGCGAT
TGCGAG
AATGAGGAGGACAACAGCTACAACCGGGGTGGTTTGAGTCCAGCCAATGACACT
GGAGCC
AAAAAGAAGAAAAAGAAACAAAAAAAGAAGAAAGAAAAAGGCAGTGAGACAG
ATTCAGCC
CAGGATCAGCCTGTGAAGATGAACTCTTTGCCAGCAGAGAGGATCCAGGAAATA
CAGAAG
 
GCCATTGAGCTGTTCTCAGTGGGTCAGGGACCTGCCAAAACCATGGAGGAGGCTA
GCAAG
CGAAGCTACCAGTTCTGGGATACGCAGCCCGTCCCCAAGCTGGGCGAAGTGGTG
AACACC
CATGGCCCCGTGGAGCCTGACAAGGACAATATCCGCCAGGAGCCCTACACCCTGC
CCCAG
GGCTTCACCTGGGATGCTTTGGACTTGGGCGATCGTGGTGTGCTAAAAGAACTGT
ACACC
CTCCTGAATGAGAACTATGTGGAAGATGATGACAACATGTTCCGATTTGATTATT
CCCCG
GAGTTTCTTTTGTGGGCTCTCCGGCCACCCGGCTGGCTCCCCCAGTGGCACTGTGG
GGTT
CGAGTGGTCTCAAGTCGGAAATTGGTTGGGTTCATTAGCGCCATCCCAGCAAACA
TCCAT
ATCTATGACACAGAGAAGAAGATGGTAGAGATCAACTTCCTGTGTGTCCACAAG
AAGCTG
CGTTCCAAGAGGGTTGCTCCAGTTCTGATCCGAGAGATCACCAGGCGGGTTCACC
TGGAG
GGCATCTTCCAAGCAGTTTACACTGCCGGGGTGGTACTACCAAAGCCCGTTGGCA
CCTGC
AGGTATTGGCATCGGTCCCTAAACCCACGGAAGCTGATTGAAGTGAAGTTCTCCC
ACCTG
AGCAGAAATATGACCATGCAGCGCACCATGAAGCTCTACCGACTGCCAGAGACT
CCCAAG
ACAGCTGGGCTGCGACCAATGGAAACAAAGGACATTCCAGTAGTGCACCAGCTC
CTCACC
AGGTACTTGAAGCAATTTCACCTTACGCCCGTCATGAGCCAGGAGGAGGTGGAGC
ACTGG
 
TTCTACCCCCAGGAGAATATCATCGACACTTTCGTGGTGGAGAACGCAAACGGAG
AGGTG
ACAGATTTCCTGAGCTTTTATACGCTGCCCTCCACCATCATGAACCATCCAACCCA
CAAG
AGTCTCAAAGCTGCTTATTCTTTCTACAACGTTCACACCCAGACCCCTCTTCTAGA
CCTC
ATGAGCGACGCCCTTGTCCTCGCCAAAATGAAAGGGTTTGATGTGTTCAATGCAC
TGGAT
CTCATGGAGAACAAAACCTTCCTGGAGAAGCTCAAGTTTGGCATAGGGGACGGC
AACCTG
CAGTATTACCTTTACAATTGGAAATGCCCCAGCATGGGGGCAGAGAAGGTTGGAC
TGGTG
CTACAATAACCAGTCACCAGTGCGATTCTGGATAAAGCCACTGAAAATTCGAACC
AGGAA
ATGGAACCCCACCACTGTTGGTCCAATTTTCACACACGTGAGAATCCCTGGCAAA
GGGAG
CAGAACTGAACCGGCTTTACCAAACCGCCAGCGAACTTGACAATTGTATTGCGAT
GGCGT
GGGCTGCGTGACGTCACCTCCGGTCGTGTCTCTGGTCTCCGTGTTTTCCAGTTAAT
TACA
TCCTCATGCAGCCGTGATCAAGGGAATGTAACTGCTGAAAACTAGCTCGTGATTG
GCATA
TAATGGAGTTAACGGGTGAATAATAAAGTATATATATATATTATATATATATATAA
ATATT
TTAAATATCTTTCATGTTCCAAATGTACAAGGATGTTTGGTCTTTAATGAAAAGCT
GAAT
CTAGATCATTCCTCAGAATGAGGACCCGAGGACAGTGGCAGACAGACGCGTTGG
CACAGT
 
TCATGGTTTCCTCCAGAGGAGACATTGGCTTATCATGGGGAAAAAGAGGATCTGG
AGAAC
CTCATCCAGCTCCCCTTCTGAATCAGCTGGGATGACTGGCTTTGAGAAGGAAGGG
AAGAT
GGAACAGGCTCAGATCTCATGGGATAGCACGTGGAGCTCTTGGCTGGGGCTGACC
CTGGG
CAGGGACTTTCCTGCAGGGCCAGACCTGCCTGCATTCTGAGACAAAGCAATGGAC
GGTCC
GCAGAAGCAGACCTCATTGATTGAGTCCTTTCTTCCATCCCCTTGGCCTGCTCCCT
GTAG
GAAGTCATCCTGCCAACTGATTTAAAAGGGCTCTTTAGCCAGTTGTTGCCAACCTT
ATAG
GGATGAGTCCCCTGTGAGATTTTGCTTTTCCACTGCCTGGGATGATGCAGTTTGAA
GAGG
CCCTTGGACCTCCTTGTAACATCAGGGACCTTTGGAGACCATTATCAGTGTAAGC
CCTGC
TTAGCTCATCTTAGAGCAAAGAGCCAGCACCCTGATGTCCCTGGGGTGGCTAGGC
AGGAG
TGGCGTGGGGCCAATACCCAGACCCCTTCAGCCACCAGCCCCTGGCCTGTGCCTT
CCAAC
CCATTAGCCATTTCTTGTTGTGCCCCTTTCCAAGATACAGCCTGCAAGTGGTAGCA
AGAA
GTGATTAGAGGCAGATCTGGACTTGGCAACAGAAGTGGTTTCCCATCTCCATTGT
CTGAG
TCTGATTTTCGCTGATGCTGTTTTGTGGATTTTTGTGGTAGTGATGGTTGTCAGTG
CTGC
CAGTTTCCCAAAACGTAATCAAGCCTCTGGTCACATGGCTGTCGATGTAGGCATT
CTGGA
 
GTGGTGTTCAGCCAAGTGACCGGGCAAAATTGGGCTGTGAAATTGTACTTCCAGG
CTTGG
ATGTAATTTTTGCTCTAGAGAGAAGCAAGTGGTGGGAAGGAGGTAGCATGACGT
GTGGTG
TGCGGGTTTCCTTGCTGCCGTCACCTCTCCGCTCATACAGGAATGAAGCCTTAGCC
AGGA
GGCCAGGCTCAGCCCTGTGCCACTCACCGAAGCCACTTTCTACAGGCCAGCAGGG
GCTTG
TTGCAGGCTGTGGGTTTTGGTGTGGTTTGTCAGAGGCTAATTCTGCAGAGTTTCCA
AAAC
CAGAAGACATCGTATGCTTGGGATGGGGGCCGTGCCACCCGTGGGAATGCTGCCC
GCTCT
GCAGACTGCTGCTAGAGCCAGCAACTCCACTAAGGTGGATTTTCATCAGGGGCCT
GCAGG
GCCCTCCCTTTTCCCATTGTTCCTGCGCTGCAAATTGCAGGCCCCAGCAATCGTGA
CTGA
CGTTTGCTCCTTGACTCCAAGAAACTGAGACCAAAGAAGCTGCTGTTCTTAGCAA
GATGC
GCACTGCATTCCACAGGTGGGAGGAGTCGGAGAGGCAGGGGCTTGCTTTGCAGC
CCCACA
GACAACAGTTGCACAGTGCCTCAAGCCCCAGAGTGGCTCACCCTGTCCAGACCTT
TGAGG
ATATCAAAGGACAAAGTGCCCAAGTCTTTCCTACCTTGGGGGAACCTGGAACTTG
GAAAG
GCTCCCTGTCCTAGTCTTGATCTGTTCTGGGCCAGGTCCCAGCTTGAGCTGCCTCT
GAGA
TTTGGGCTGTGCGGATCTCTGGAGTGAGCTCTGTTTCGGTTGACCCAGGTCATGG
AATGG
 
AAACGGTGAGGCCCCAGTGGCTGTTCTGGAAGAAACAGATCTCCTGGCAAAGGC
CCCAGC
ATCTCCCTCACTGAAACCAGGTGGCCGGCTCCTCGGACTCTGCTTTATGTTGCGGT
GAGA
ACTCTGCCCAGGTGTGCAGGGTTTGGCTTGTGGGCTGCTTGCTGCTCATCTGATTT
TTGT
CCCAGTAGTCCCTGCGTTCTTCATTCAACCCCTTCTGGGACTTCAGCTCAGAGAGC
ACCA
TCCCGGGGGTCAGGGCCTCCCCACAGGAGCCCTGCAGTGTGGTAGCGCCATGGCT
GTCTC
AAACCAAGCAAAGGAAGGACCCTGAGGCCTTCACGCTAACCATCCTCGAGCAAC
TGCTGT
TGGAAGGCCTCCCTGGGCCTGGCCCCACCCTCTGCCACCCAGTCCTCCCAGCTG
CCATG
TTTCAAAGACGACCTTTACCTCCTGCCTTTGGATTGACTCTGCATTTGACCACGGA
CTCC
AGTCTGTGTGTAGGGAGAGAGCTGAGTAGGAGGCCTCCACTCCGGATCGAGGCC
TGTATA
GGGCTCGTTTCCCCACACATGCCTATTTCTGAAGAGGCTTCTGTCTTATTTGAAGG
CCAG
CCCACACCCAGCTACTTTAACACCAGGTTTATGGAAAATGTCAGGCCTTCCCCAC
AACTC
CTGTCTAACTGCTGTCGCCCCCCTACTTGCTGGCTCTCAGAAGCCTAGGGGAGTCC
CTGT
GGTCCTGAATTCTTTCCCCAAAGACGACCAGCATTTAACCAACCTAAGGGCCCAA
AGGCC
TTGGACAACTGCATGGAGCTGCACTCTAGGAGAAGGAGGGGAACCAGATGTTAG
ATCAGG
 
GGAGGGAGCAGGAGTGTCCCTCCCGTCAGTGCCTACCCACCTGTGAGGCAGCCTT
CTGAT
GGCCTGGCCCACCTTCCCCAGAACCAGGGGAGGCCTGAGGCTTCAGTTTACTCT
GCTGC
AAAATGAAGGCGGGCCTGCAAGCCGACTACACCTACGGAGGCTGTTGAGGACAA
TTTCAT
TCCATTAAATTAAAAAATACTGACTGGCTGGCAGGCAGGTGCCATGTCTGGGAAC
AGGGA
CGGGGGAGCTTCACCTTTTTGTCTTGGCTTTTCTTTGGGCTGTGGGGGGCATCCA
TTTC
CAGGGTCGGGGAGGAAATACCAAATGCATTGTTGTTCTGCTCAATACATCTCACT
TGTTT
CTAATAAAGAAAGCAGCTGAACAAAAAAAAAAAAAAAAAAAA
NO:19 protein (NMT1) 
MADESETAVKPPAPPLPQMMEGNGNGHEHCSDCENEEDNSYNRGGLSPAN
DTGAKKKKKKQKKKKEKGSETDSAQDQPVKMNSLPAERIQEIQKAIELFS
VGQGPAKTMEEASKRSYQFWDTQPVPKLGEVVNTHGPVEPDKDNIRQEPY
TLPQGFTWDALDLGDRGVLKELYTLLNENYVEDDDNMFRFDYSPEFLLWA
LRPPGWLPQWHCGVRVVSSRKLVGFISAIPANIHIYDTEKKMVEINFLCV
HKKLRSKRVAPVLIREITRRVHLEGIFQAVYTAGVVLPKPVGTCRYWHRS
LNPRKLIEVKFSHLSRNMTMQRTMKLYRLPETPKTAGLRPMETKDIPVVH
QLLTRYLKQFHLTPVMSQEEVEHWFYPQENIIDTFVVENANGEVTDFLSF
YTLPSTIMNHPTHKSLKAAYSFYNVHTQTPLLDLMSDALVLAKMKGFDVF
NALDLMENKTFLEKLKFGIGDGNLQYYLYNWKCPSMGAEKVGLVLQ
NO:4, Macro flanking 
CTGGTGCTGCCCTCTCTTCCACCCACTCACTCACCTTTCTCTGGTCATCTTGAATTC
CTA
 
CAGTTTATCAATGCTGTTCCTTCAATTGAACGACTTCTCTCACTCCCAAATCCCTT
CTGG
TGAATGACTATCACTCATCCTAAGGGCACCTTTTCAATGAATCCTACTGCCAAGT
AGAAC
TGACCCCTCACACTCCCAATCCATCTTTTCAATGTATATTCTGCACAGAGATTCCT
CAAT
AGCACAAATAACTCTACAAGTTGGTTGTTTTTCTTTCTTTTTTAGAGATTTTATT
TAA
GAAAGAGAGAGAGAACACAAGAGGGAGGGAGAGGCAACAAGAGAGGAAAA
AACAGATT
CCCTGCTGAACAGGGAGCTCAAAGCGGGGCTCAGTCTTAGTACCCTGAGACCATG
ACCTG
AACAGAAGGCAGATGGTTAACTGAATGAGCCACCGAGGTGCCCCAGTGGTTGCT
TTTATT
GGTCTCTTCCCGACTGTGAGTTCCCCAAGAGCAGGAACCACACATTACATTGCTT
AAACC
TCAGTTCAAGCAGGAATAAAGAAGNGAAAGGATGATGGNAATTATCCAAACNCT
GAGGAG
CAAACCCCACGCANCATGCC
NO:12 MACRO cDNA 
GGGGGCCAAAGGGAAGTGCTGCGAGGTTTACAACCAGCTGCAGTGGTTCGATGG
GAAGGA
TCTTTCTCCAAGTGGTTCCTCTTGAGGGGAGCATTTCTGCTGGCTCCAGGACTTTG
GCCA
TCTATAAAGCTTGGCAATGAGAAATAAGAAAATTCTCAAGGAGGACGAGCTCTT
GAGTGA
GACCCAACAAGCTGCTTTTCACCAAATTGCAATGGAGCCTTTCGAAATCAATGTT
CCAAA
 
GCCCAAGAGGAGAAATGGGGTGAACTTCTCCCTAGCTGTGGTGGTCATCTACCTG
ATCCT
GCTCACCGCTGGCGCTGGGCTGCTGGTGGTCCAAGTTCTGAATCTGCAGGCGCGG
CTCCG
GGTCCTGGAGATGTATTTCCTCAATGACACTCTGGCGGCTGAGGACAGCCCGTCC
TTCTC
CTTGCTGCAGTCAGCACACCCTGGAGAACACCTGGCTCAGGGTGCATCGAGGCTG
CAAGT
CCTGCAGGCCCAACTCACCTGGGTCCGCGTCAGCCATGAGCACTTGCTGCAGCGG
GTAGA
CAACTTCACTCAGAACCCAGGGATGTTCAGAATCAAAGGTGAACAAGGCGCCCC
AGGTCT
TCAAGGCCACAAGGGGGCCATGGGCATGCCTGGTGCCCCTGGCCCGCCGGGACC
ACCTGC
TGAGAAGGGAGCCAAGGGGGCTATGGGACGAGATGGAGCAACAGGCCCCTCGG
GACCCCA
AGGCCCACCGGGAGTCAAGGGAGAGGCGGGCCTCCAAGGACCCCAGGGTGCTCC
AGGGAA
GCAAGGAGCCACTGGCACCCCAGGACCCCAAGGAGAGAAGGGCAGCAAAGGCG
ATGGGGG
TCTCATTGGCCCAAAAGGGGAAACTGGAACTAAGGGAGAGAAAGGAGACCTGGG
TCTCCC
AGGAAGCAAAGGGGACAGGGGCATGAAAGGAGATGCAGGGGTCATGGGGCCTC
CTGGAGC
CCAGGGGAGTAAAGGTGACTTCGGGAGGCCAGGCCCACCAGGTTTGGCTGGTTTT
CCTGG
AGCTAAAGGAGATCAAGGACAACCTGGACTGCAGGGTGTTCCGGGCCCTCCTGG
TGCAGT
 
GGGACACCCAGGTGCCAAGGGTGAGCCTGGCAGTGCTGGCTCCCCTGGGCGAGC
AGGACT
TCCAGGGAGCCCCGGGAGTCCAGGAGCCACAGGCCTGAAAGGAAGCAAAGGGG
ACACAGG
ACTTCAAGGACAGCAAGGAAGAAAAGGAGAATCAGGAGTTCCAGGCCCTGCAGG
TGTGAA
GGGAGAACAGGGGAGCCCAGGGCTGGCAGGTCCCAAGGGAGCCCCTGGACAAG
CTGGCCA
GAAGGGAGACCAGGGAGTGAAAGGATCTTCTGGGGAGCAAGGAGTAAAGGGAG
AAAAAGG
TGAAAGAGGTGAAAACTCAGTGTCCGTCAGGATTGTCGGCAGTAGTAACCGAGG
CCGGGC
TGAAGTTTACTACAGTGGTACCTGGGGGACAATTTGCGATGACGAGTGGCAAAAT
TCTGA
TGCCATTGTCTTCTGCCGCATGCTGGGTTACTCCAAAGGAAGGGCCCTGTACAAA
GTGGG
AGCTGGCACTGGGCAGATCTGGCTGGATAATGTTCAGTGTCGGGGCACGGAGAG
TACCCT
GTGGAGCTGCACCAAGAATAGCTGGGGCCATCATGACTGCAGCCACGAGGAGGA
CGCAGG
CGTGGAGTGCAGCGTCTGACCCGGAAACCCTTTCACTTCTCTGCTCCCGAGGTGT
CCTCG
GGCTCATATGTGGGAAGGCAGAGGATCTCTGAGGAGTTCCCTGGGGACAACTGA
GCAGCC
TCTGGAGAGGGGCCATTAATAAAGCTCAACATCAAAAAAAAAAAAGAAAAAAA
AAAAAAA
AAA
NO:20, MACRO protein 
MRNKKILKEDELLSETQQAAFHQIAMEPFEINVPKPKRRNGVNFSLAVVV
IYLILLTAGAGLLVVQVLNLQARLRVLEMYFLNDTLAAEDSPSFSLLQSA
HPGEHLAQGASRLQVLQAQLTWVRVSHEHLLQRVDNFTQNPGMFRIKGEQ
GAPGLQGHKGAMGMPGAPGPPGPPAEKGAKGAMGRDGATGPSGPQGPPGV
KGEAGLQGPQGAPGKQGATGTPGPQGEKGSKGDGGLIGPKGETGTKGEKG
DLGLPGSKGDRGMKGDAGVMGPPGAQGSKGDFGRPGPPGLAGFPGAKGDQ
GQPGLQGVPGPPGAVGHPGAKGEPGSAGSPGRAGLPGSPGSPGATGLKGS
KGDTGLQGQQGRKGESGVPGPAGVKGEQGSPGLAGPKGAPGQAGQKGDQG
VKGSSGEQGVKGEKGERGENSVSVRIVGSSNRGRAEVYYSGTWGTICDDE
WQNSDAIVFCRMLGYSKGRALYKVGAGTGQIWLDNVQCRGTESTLWSCTK
NSWGHHDCSHEEDAGVECSV
SEQ ID NO:5, CDK6 planking 
CCTCTGCCTATGTCTCTGCCTCTCTCTCTCTCTCTCTCTCTGTGACTATCATAAA
TAA
ATAAAAATTAAAAAAAAAAAAGATATTCAGTTCTGATCTGTGTCAGATTCACCGT
GAAGT
GTTCTCTTTTAAATAAATAAATAAATAAATAAATAAATAAGTAAGTAAGTAAATA
AAGCG
CTAAACATAACAGGAAAGATTGGCCATACAGACTTCTTACAATTTAAAACGTCTT
TTCAT
GGGACACCTGAATGGCTCAATGTTGGACATCCGACCCTCAATTTTGGCTCAGGTT
ATGAT
CTCGGGGTCATGGGATCAAGTCCCACTAGACACAGTCTGCTTGTTCTTCTCCCTCT
GCTC
CTCCTCAATTCTCTCTCTCTTTCTCAAATGAATAAATAAAATCTTTAAAAAAATAA
AACC
TCTATTCATCAAAATATAACATTAAGAGAATGAAAAGACNAGAAGTAATGTGGA
ATAAGA
 
CATTTTACATGGATAAATCATNCNAAGGACTATTTCTAGACCATATAAATATCTCT
TANA
AATTAATAAGNNNAAATTGTCTGACTCAATTATTTTAAGAGNAGGATAAAAGAN
TTGAA
TAGATTTTTNCAAATGAAAATATCCCAATGGNCCAATGNCCATGAAAATATNNT
CCNNC
CNCNAAAGNTATCCGGAAAATGCNAGNNGGAAATTAAACN
SEQ ID NO:13, CDK6 CDNA 
GGCTTCAGCCCTGCAGGGAAAGAAAAGTGCAATGATTCTGGACTGAGACGCGCT
TGGGCA
GAGGCTATGTAATCGTGTCTGTGTTGAGGACTTCGCTTCGAGGAGGGAAGAGGAG
GGATC
GGCTCGCTCCTCCGGCGGCGGCGGCGGCGACTCTGCAGGCGGAGTTTCGCGG
CGGCG
GCACCAGGGTTACGCCAGCCCCGCGGGGAGGTCTCTCCATCCAGCTTCTGCAGCG
GCGAA
AGCCCCAGCGCCCGAGCGCCTGAGCCGGCGGGGAGCAAGTAAAGCTAGACCGAT
CTCCGG
GGAGCCCCGGAGTAGGCGAGCGGCGGCCGCCAGCTAGTTGAGCGCACCCCCCGC
CCGCCC
CAGCGGCGCCGCGGCGGGCGGCGTCCAGGCGGCATGGAGAAGGACGGCCTGTGC
CGCGCT
GACCAGCAGTACGAATGCGTGGCGGAGATCGGGGAGGGCGCCTATGGGAAGGTG
TTCAAG
GCCCGCGACTTGAAGAACGGAGGCCGTTTCGTGGCGTTGAAGCGCGTGCGGGTG
CAGACC
GGCGAGGAGGGCATGCCGCTCTCCACCATCCGCGAGGTGGCGGTGCTGAGGCAC
CTGGAG
 
ACCTTCGAGCACCCCAACGTGGTCAGGTTGTTTGATGTGTGCACAGTGTCACGAA
CAGAC
AGAGAAACCAAACTAACTTTAGTGTTTGAACATGTCGATCAAGACTTGACCACTT
ACTTG
GATAAAGTTCCAGAGCCTGGAGTGCCCACTGAAACCATAAAGGATATGATGTTTC
AGCTT
CTCCGAGGTCTGGACTTTCTTCATTCACACCGAGTAGTGCATCGCGATCTAAAAC
CACAG
AACATTCTGGTGACCAGCAGCGGACAAATAAAACTCGCTGACTTCGGCCTTGCCC
GCATC
TATAGTTTCCAGATGGCTCTAACCTCAGTGGTCGTCACGCTGTGGTACAGAGCAC
CCGAA
GTCTTGCTCCAGTCCAGCTACGCCACCCCCGTGGATCTCTGGAGTGTTGGCTGCAT
ATTT
GCAGAAATGTTTCGTAGAAAGCCTCTTTTTCGTGGAAGTTCAGATGTTGATCAAC
TAGGA
AAAATCTTGGACGTGATTGGACTCCCAGGAGAAGAAGACTGGCCTAGAGATGTT
GCCCTT
CCCAGGCAGGCTTTTCATTCAAAATCTGCCCAACCAATTGAGAAGTTTGTAACAG
ATATC
GATGAACTAGGCAAAGACCTACTTCTGAAGTGTTTGACATTTAACCCAGCCAAAA
GAATA
TCTGCCTACAGTGCCCTGTCTCACCCATACTTCCAGGACCTGGAAAGGTGCAAAG
AAAAC
CTGGATTCCCACCTGCCGCCCAGCCAGAACACCTCGGAGCTGAATACAGCCTGA1
372GGCCTC
AGCAGCCGCCTTAAGCTGATCCTGCGGAGAACACCCTTGGTGGCTTATGGGTCCC
CCTCA
 
GCAAGCCCTACAGAGCTGTGGAGGATTGCTATCTGGAGGCCTTCCAGCTGCTGTC
TTCTG
GACAGGCTCTGCTTCTCCAAGGAAACCGCCTAGTTTACTGTTTGAAATCAATGC
AAGAG
TGATTGCAGCTTTATGTTCATTTGTTTGTTTGTTTGTCTGTTTGTTTCAAGAACCTG
GAA
AAATTCCAGAAGAAGAGAAGCTGCTGACCAATTGTGCTGCCATTTGATTTTTCTA
ACCTT
GAATGCTGCCAGTGTGGAGTGGGTAATCCAGGCACAGCTGAGTTATGATGTAATC
TCTCT
GCAGCTGCCGGGCCTGATTTGGTACTTTTGAGTGTGTGTGTGCATGTGTGTGTG
TGTG
TGTGTGTGTGTGTGTGTATGTGAGAGATTCTGTGATCTTTTAAAGTGTTACTTTTT
GTAA
ACGACAAGAATAATTCAATTTTAAAGACTCAAGGTGGTCAGTAAATAACAGGCA
TTTGTT
CACTGAAGGTGATTCACCAAAATAGTCTTCTCAAATTAGAAAGTTAACCCCATGT
CCTCA
GCATTTCTTTTCTGGCCAAAAGCAGTAAATTTGCTAGCAGTAAAAGATGAAGTTT
TATAC
ACACAGCAAAAAGGAGAAAAAATTCTAG
TATATTTTAAGAGATGTGCATGCATTCTATTT
AGTCTTCAGAATGCTGAATTTACTTGTTGTAAGTCTATTTTAACCTTCTGTATGAC
ATCA
TGCTTTATCATTTCTTTTGGAAAATAGCCTGTAAGCTTTTTATTACTTGCTATAGGT
TTAGGGAGTGTACCTCAGATAGATTTTAAAAAAAAGAATAGAAAGCCTTTATTTC
CTGGTTTG
 
AAATTCCTTTCTTCCCTTTTTTTGTTGTTGTTATTGTTGTTTGTTGTTGTTATTTTGT
TT
TTGTTTTTAGGAATTTGTCAGAAACTCTTTCCTGTTTTGGTTTGGAGAGTAGTTCTC
TCT
AACTAGAGACAGGAGTGGCCTTGAAATTTTCCTCATCTATTACACTGTACTTTCTG
CCAC

ACACTGCCTTGTTGGCAAAGTATCCATCTTGTCTATCTCCCGGCACTTCTGAAATA
TATT
GCTACCATTGTATAACTAATAACAGATTGCTTAAGCTGTTCCCATGCACCACCTGT
TTGC
TTGCTTTCAATGAACCTTTCATAAATTCGCAGTCTCAGCTTATGGTTTATGGCCTC
GATT
CTGCAAACCTAACAGGGTCACATATGTTCTCTAATGCAGTCCTTCTACCTGGTGTT
TACT
TTTGCTACCCAAATAATGAGTAGGATCTTGTTTTCGTATACCCCCACCACTCCCAT
TGCT
ACCAACTGTCACCTTGTGCACTCCTTTTTTATAGAAGATATTTTCAGTGTCTTTAC
CTGA
GGGTATGTCTTTAGCTATGTTTTAGGGCCATACATTTACTCTATCAAATGATCTTT
TCTC
CATCCCCCAGGCTGTGCTTATTTCTAGTGCCTTGTGCTCACTCCTGCTCTCTACAG
AGCC
AGCCTGGCCTGGGCATTGTAAACAGCTTTTCCTTTTTCTCTTACTGTTTCTCTACA
GTC
CTTTATATTTCATACCATCTCTGCCTTATAAGTGGTTTAGTGCTCAGTTGGCTCTA
GTAA
 
CCAGAGGACACAGAAAGTATCTTTTGGAAAGTTTAGCCACCTGTGCTTTCTGACT
CAGAG
TGCATGCAACAGTTAGATCATGCAACAGTTAGATTATGTTTAGGGTTAGGATTTT
CAAAG
AATGGAGGTTGCTGCACTCAGAAAATAATTCAGATCATGTTTATGCATTATTAAG
TTGTA
CTGAATTCTTTGCAGCTTAATGTGATATATGACTATCTTGAACAAGAGAAAAAAC
TAGGA
GATGTTTCTCCTGAAGAGCTTTTGGGGTTGGGAACTATTCTTTTTTAATTGCTGTA
CTAC
TTAACATTGTTCTAATTCAGTAGCTTGAGGAACAGGAACATTGTTTTCTAGAGCA
AGATA
ATAAAGGAGATGGGCCATACAAATGTTTTCTACTTTCGTTGTGACAACATTGATT
AGGTG
TTGTCAGTACTATAAATGCTTGAGATATAATGAATCCACAGCATTCAAGGTCAGG
TCTAC
TCAAAGTCTCACATGGAAAAGTGAGTTCTGCCTTTCCTTTGATCGAGGGTCAAAA
TACAA
AGACATTTTTGCTAGGGCCTACAAATTGAATTTAAAAACTCACTGCACTGATTCA
TCTGA
GCTTTTTGGTTAGTATTCATGGCTAGAGTGAACATAGCTTTAGTTTTGCTGTTGT
AAAA
GTGTTTTCATAAGTTCACTCAAGAAAAATGCAGCTGTTCTGAACTGGAATTTTTCA
GCAT
TCTTTAGAATTTTAAATGAGTAGAGAGCTCAACTTTTATTCCTAGCATCTGCTTTT
GACT
CATTTCTAGGCAGTGCTTATGAAGAAAAATTAAAGCACAAACATTCTGGCATTCA
ATCGT
 
TGGCAGATTATCTTCTGATGACACAGAATGAAAGGGCATCTCAGCCTCTCTGAAC
TTTGT
AAAAATCTGTCCCCAGTTCTTCCATCGGTGTAGTTGTTGCATTTGAGTGAATACTC
TCTT
GATTTATGTATTTTATGTCCAGATTCGCCATTTCTGAAATCCAGATCCAACACAAG
CAGT
CTTGCCGTTAGGGCATTTTGAAGCAGATAGTAGAGTAAGAACTTAGTGACTACAG
CTTAT
TCTTCTGTAACATATGGTTTCAAACATCTTTGCCAAAAGCTAAGCAGTGGTGAAC
TGAAA
AGGGCATATTGCCCCAAGGTTACACTGAAGCAGCTCATAGCAAGTTAAAATATTG
TGACA
GATTTGAAATCATGTTTGAATTTCATAGTAGGACCAGTACAAGAATGTCCCTGCT
AGTTT
CTGTTTGATGTTTGGTTCTGGCGGCTCAGGCATTTTGGGAACTGTTGCACAGGGTG
CAGT
CAAAACAACCTACATATAAAAATTACATAAAAGAACCTTGTCCATTTAGCTTTCA
TAAGA
AATCCCATGGCAAAGAGTAATAAAAAGGACCTAATCTTAAAAATACAATTTCTAA
GCACT
TGTAAGAACCCAGTGGGTTGGAGCCTCCCACTTTGTCCCTCCTTTGAAGTGGATG
GGAAC
TCAAGGTGCAAAGAACCTGTTTTGGAAGAAAGCTTGGGGCCATTTCAGCCCCCTG
TATTC
TCATGATTTTCTCTCAGGAAGCACACACTGTGAATGGCAGACTTTTCATTTAGCCC
CAGG
TGACTTACTAAAAATAGTTGAAAATTATTCACCTAAGAATAGAATCTCAGCATTG
TGTTA
 
AATAAAAATGAAAGCTTTAGAAGGCATGAGATGTTCCTATCTTAAATAAAGCATG
TTTCT
TTTCTATAGAGAAATGTATAGTTTGACTCTCCAGAATGTACTATCCATCTTGATGA
GAAA
ACTCTTAAATAGTACCAAACATTTTGAACTTTAAATTATGTATTTAAAGTGAGTGT
TTAA
GAAACTGTAGCTGCTTCTTTTACAAGTGGTGCCTATTAAAGTCAGTAATGGCCATT
ATTG
TTCCATTGTGGAAATTAAATTATGTAAGCTTCCTAATATCATAAACATATTAAAAT
TCTT
CTAAAATATTGCTTTTCTTTTAAGTGACAATTTGACTATTCTTATGATAAGCACAT
GAGA
GTGTCTTACATTTTCCAAAAGCAGGCTTTAATTGCATAGTTGAGTCTAGGAAAAA
ATAAT
GTTAAAAGTGAATATGCCACCATAATTACTTAATTATGTTAGTATAGAAACTACA
GAATA
TTTACCCTGGAAAGAAAATATTGGAATGTTATTATAAACTCTTAGATATTTATATA
ATTC
AAAAGAATGCATGTTTCACATTGTGACAGATAAAGATGTATGATTTCTAAGGCTT
TAAAA
ATTATTCATAAAACAGTGGGCAATAGATAAAGGAAATTCTGGAGAAAATGAAGG
TATTTA
AAGGGTAGTTTCAAAGCTATATATATTTTGAAGGATATATTCTTTATGAACAAAT
ATATT
GTAAAAATTTATACTAAGGTCATCTGGTAACTGTGGGATTAATATGGTCGAAAAC
AAATG
TTATGGAGAAGCTGTCCCAAGCAAACTAAATTACCTGTACTTTTTTCCCATTTCAA
GGGA
 
AGAGGCAACCACATGAAGCAATACTTCTTACACATGCCTAAGAACGTTCATTGAA
AAAAT
AAATTTTTAAAAGGCATGTGTTTCCTATGCCACCAATACTTTTGAAAAATTGTGAA
CCTT
ACCCAAAACCATTTATCATGTCCATTAAGTATATTTGGGTATATAATTAGGAAGA
TATTT
ACATGTTCCATCTCCACAGTGGAAAAACTTATTGAGGCTACCAAAGTGTGCCAAG
AAATG
TAAGTCCTTAGAGTAATTAGAAATGCTGTTTTCCTCAAAAGCATGAGAAACTAGC
ATTTT
CATTTCTTATTTACTCCCTTTCTATATCAATGCAATTCACAACCCAATTTTAATACA
TCC
CTATATCTCAAGCATTTCTATCTTGTACTTTTTCAGAAAATAAACCAAAAATAATC
CTTT
GGTCTCTCTATCTTCTGACCTTTGTAAGCAACAGAAATGTAAAAACAGAAGGGGT
CCAAT
TTTTACACGTTTTTTTCTCAAGTAGCCTTTCTGGGGATTTTTATTTCTTAATGAAG
TGC
CAATCAGCTTTTCAAAATGTTTTCTATTTCTCAGCATTTCCAGGAAGTGATAACGT
TTAG
CTAAATGAGTAGAAGTGGACTTCCTTCAACATATTGTTACCTTGTCTAGCCTTAGG
AAGA
AAACAAGAGCCACCTGAAAATAAATACAGGCTCTTTTCGAGCATCTGCTGAAATA
CTGTT
ACAGCAATTTGAAGTTGATGTGGTAGGAAAGGAAGGTGACTTTTCTTGCAAAAGT
CTTTC
TAAACATTCACACTGTCCTAAGAGATGAGCTTTCTTGTTTTATTCCGGTATATTCC
ACAA
 
GGTGGCACTTTTAGAGAAAAACAAATCTGATGAAGACTAAAGAGGTACTTCTAA
AAGAGA
TTTCATTCTAACTTTATTTTTCTGCGCATATTTAACTCTTTCCTAGCACTTGTTTTTT
GG
GATGATTAATAGTCTCTATAATGTTCTGTAACTTCAATATTTTACTTGTTACCTAG
GTTC
TGAACAATTGTCTGCAAATAAATTGTTCTTAAGGATGGATAATACACCCATTTTG
ATCAT
TTAAGTAAAGAAAGCCTAGTCATTCATTCAGTCAAGAAAAAATTTTTGAAGTACC
CAGTT
ACCTTACTTTTCTAGATTAAAACAGGCTTAGTTACTAAAAAGGCAGTCCTCATCTG
TGAA
CAGGATAGTTTCGTTAGAAGTATAAAACTCCTTTAGTGGCCCCAGTTAAAACACA
CATAC
CCTCTCTGCTGCTTTCAAATTCCCTAGCATGGTGGCCTTTCAACATTGATTAAATT
TTAA
AATCCTAATTTAAAGATCAGGTGAGCAAAATGAGTAGCACATCAGTAATTCAGTA
GACAA
AACTTTTGTCTGAAAAATTGCTGTATTGAAACAGAGCCCTAAAATACCAAAAGAC
CAGGT
AATTTTAACATTTGTGGAATCACAAATGTAAATTCATAAGAAGCTCTAATTAAAA
AAAAA
AAGTCTGAAGTATATGAGCATAACAACTTAGGAGTGTGTCTACATACTTAACTTT
TGAAG
TTTTTTGGCAACTTTATATACTTTTTTTAAATTTACAAGTCTACTTAAAGACTTCTT
ATA
CCCCAAATGATTAAGTTAATTTTAGAGGTCACCTTTCTCACAGCAGTGTCACTTGA
AATT
 
TAGTAGGGAAGGATATTGCAGTATTTTTCAGTTTCCTTAGCACAGCACCACAGAA
AGCAG
CTTATTCCTTTTGAGTGGCAGACACTCGACGGTGCCTGCCCAACTTTCCTCCTGAG
TGGC
AAGCAGATGAGTCTCAGTAATTCATACTGAACCAAAATGCCACATACACTAGGG
GCAGTC
AGAAACTGGCTGAGAAATCCCCCGCCTCATTCGCCCCTCTGCTCCCAGGAACTAG
AGTCC
AGTTAAAGCCCCTATGCGAAAGGCCGAATTCCACCCCAGGGTTTGTTATAACAGT
GGCCA
GTCTGAACCCCATTTGCTCGTGCTCAAAACTTGATTCCCACTTGAAAGCCTTCCGG
GCGC
GCTGCCTCGTTGCCCCGCCCCTTTGGCAGGAGAGGCAGTGGGCGAGGCCGGG
CTGGGG
CCCCGCCTCCCACTCACCTGCCGGTGCCTGAAATTATGTGCGGCCCCGCGGGCTG
CTTTC
CGAGGTCAGAGTGCCCTGCTGCTGTCTCAGAGGCATCTGTTCTGCAAATCTTAGG
AAGAA
AAATGTCCCTAGTAGCAAACGGGTGTCTTCTGTGCATAAATAAGTACAACACAAT
TCTCC
GAAAGTTCGGGTAAAAAGAGATGCGGTAGCAGCTGCCCTGTGTGAAGCTGTCTA
CCCCGC
ATCTCTCAGGCGCTAAGCTCAGTTTTTGTTTTTGTTTTTGTTTTTTTAAAGAAAAGA
TGT
ATAATTGCAGGAATTTTTTTTTATTTTTTTATTTTCCATCATTCTATATATGTGATG
GTG
AAAGATATGCCTGGAAAAGTTTTGTTTTGAAAAGTTTATTTCTGCTTCGTCTTCA
GTTG
 
GCAAAAGCTCTCAATTCTTTAGCTTCCAGTTTCTTTTCTCTCTTTTTCTTTGTTAGG
TAA
TTAAAGGTATGTAAACAAATTATCTCATGTAGCAGGGGATTTTCATGTTGAGAGG
AATCT
TCCGTGTGAGTTGTTTGGTCACACAAATAACCCTTTCTCAATTTTAGGAGTTTGGA
TTGT
CAAATGTAGGTTTTTCTCAAAGGGGGCATATAACTACATATTGACTGCCAAGAAC
TATGA
CTGTAGCACTAATCAGCACACATAGAGCCACACAATTATTTAATTTCTAACTCTCT
GTGG
TCCCTAGAAAAATTCCGTTGATGTGCTTAGGTTAAAGTTCTGAAGATACCCGTTGT
ACCC
TTACTTGAAAGTTTCTAATCTTAAGTTTTATGAAATGCAATAATATGTATCAGCTA
GCAA
TATTTCTGTGATCACCAACAACTCTCAGTTTGATCTTAAAGTCTGAATAATAAAAC
AAAT
CCCAGCAGTAATACATTTCTTAAACCTCACAGTGCATGATATATCTTTTCATTCTG
ATCC
TGTGTTTGCAAAAATATACACATGTATATCATAGTTCCTCACTTTTTATTCATTTGT
TTT
CCTATTACCTGTAGTAAATATATTAGTTAGTACATGGAATTTATAGCATCAGCTAC
CCCC
AGGAACAGCACCTGACAGGCGGGGGATTTTTTTCAAGTTGTTCTACATTTGCAT
AAATT
ATTTCTATTATTATTCATGTATGTTATTTATTTCTGAATCACACTAGTCCTGTGAAA
GTA
CAACTGAAGGCAGAAAGTGTTAGGATTTTGCATCTAATGTTCATTATCATGGTAT
TGATG
 
GACCTAAGAAAATAAAAATTAGACTAAGCCCCCAAATAAGCTGCATGCATTTGTA
ACATG
ATTAGTAGATTTGAATATATAGATGTAGTATTTTGGGTATCTAGGTGTTTTATCAT
TATG
TAAAGGAATTAAAGTAAAGGACTTTGTAGTTGTTTTTATTAAATATGCATATAGT
AGAGT
GCAAAAATATAGCAAAAATAAAAACTAAAGGTAGAAAAGCATTTTAGATATGCC
TTAATT
TAGAAACTGTGCCAGGTGGCCCTCGGAATAGATGCCAGGCAGAGACCAGTGCCT
GGGTGG
TGCCTCCTCTTGTCTGCCCTCATGAAGAAGCTTCCCTCACGTGATGTAGTGCCCTC
GTAG
GTGTCATGTGGAGTAGTGGGAACAGGCAGTACTGTTGAGAGGAGAGCAGTGTGA
GAGTTT
TTCTGTAGAAGCAGAACTGTCAGCTTGTGCCTTGAGGCTTCCAGAACGTGTCAGA
TGGAG
AAGTCCAAGTTTCCATGCTTCAGGCAACTTAGCTGTGTACAGAAGCAATCCAGTG
TGGTA
ATAAAAAGCAAGGATTGCCTGTATAATTTATTATAAAATAAAAGGGATTTTAACA
ACCAA
CAATTCCCAACACCTCAAAAGCTTGTTGCATTTTTTGGTATTTGAGGTTTTTATCT
GAAG
GTTAAAGGGCAAGTGTTTGGTATAGAAGAGCAGTATGTGTTAAGAAAAGAAAAA
TATTGG
TCACGTAGAGTGCAAATTAGAACTAGAAAGTTTTATACGATTATCATTTTGAGAT
GTGT
TAAAGTAGGTTTTCACTGTAAAATGTATTAGTGTTTCTGCATTGCCATAGGGCCTG
GTTA
 
AAACTTTCTCTTAGGTTTCAGGAAGACTGTCACATACAGTAAGCTTTTTTCCTTCT
GACT
TATAATAGAAAATGTTTTGAAAGTAAAAAAAAAAATCTAATTTGGAAATTTGAC
TTGTT
AGTTTCTGTGTTTGAAATCATGGTTCTAGAAATGTAGAAATTGTGTATATCAGATA
CTCA
TCTAGGCTGTGTGAACCAGCCCAAGATGACCAACATCCCCACACCTCTACATCTC
TGTCC
CCTGTATCTCTTCCTTTCTACCACTAAAGTGTTCCCTGCTACCATCCTGGCTTGTCC
ACA
TGGTGCTCTCCATCTTCCTCCACATCATGGACCACAGGTGTGCCTGTCTAGGCCTG
GCCA
CCACTCCCAACTTGACCTAGCCACATTCATCTAGAGATGGTTCCTGATGCTGGGC
ACAGA
CTGTGCTCATGGCACCCATTAGAAATGCCTCTAGCATCTTTGTATGCATCTTGATT
TTTA
AACCAAGTCATTGTACAGAGCATTCAGTTTTGGCTGTGGTACCAAGAGAAAAACT
AATCA
AGAATATAAACCACATTCCAGGCTGCTGTTTTCTCTCCATCTACAGGCCACACTTT
TACT
GTATTTCTTCATACTTGAAATTCATTCTGCTATTTTCATATCAGGGTACAGACTTA
TAAG
GGTGCATGTTCCTTAAAGGTGCATAATTATTCTTATTCCGTTTGCTTATATTGCTA
CAGA
ATGCTCTGTTTTGGTGCTTTGAGTTCTGCAGACCCAAGAAGCAGTGTGGAAATTC
ACTGC
CTGGGACACAGTCTTATAAGAATGTTGGCAGGTGACTTTGTATCAGATGTTGCTT
CTCTT
 
TTCTCTGTACACAGATTGAGAGTTACCACAGTGGCCTGTCGGGTCCACCCTGTGG
GTGCA
GCACAGCTCTCTGAAAGCAAGAACCTTCCTACCTATTCTAACGTTTTGCCCTCTA
AGAA
AAATGGCCTCAGGTATGGTATAGACATAGCAAGAGGGGAAGGGCTGTCTCACTC
TAGCAA
CCATCCCTCCATTACACACAGAAAGCCCTCTTGAAGCAAAAGAAGAAAGAA
AGAAAG
CTTATCTCTAAGGCTACTGTCTTCAGAATGCTCTGAGCTGAATGCTCTTGCTCCTT
TCCC
AAGAGGCAGATGAAAATATAGCCAGTTTATCTATACCCTTCCTATCTGAGGAGGA
GAATA
GAAAAGTAGGGTAAATATGTAACGTAAAATATGTCATTCAAGGACCACCAAAAC
TTTAAG
TACCCTATCATTAAAAATCTGGTTTTAAAAGTAGCTCAAGTAAGGATGCTTTGT
GACCC
AGGGTTTCTGAAGTCAGATAGCCATTCTTACCTGCCCCTTACTCTGACTTATTGGG
AAAG
GAGAACTGCAGTGGTGTTTCTGTTGCAGTGGCAAAGGTAACATGTCAGAAAATTC
AGAG
GGTTGCATACCAATAATCCTTTGGAAACTGGATGTCTTACTGGGTGCTAGAATGA
AAATG
TAGGTATTTATTGTCAGATGATGAAGTTCATTGTTTTTTCAAAATTGGTGTTGAA
ATAT
CACTGTCCAATGTGTTCACTTATGTGAAAGCTAAATTGAATGAGGCAAAAAGAGC
AAATA
GTTTGTATATTTGTAATACCTTTTGTATTTCTTACAATAAAAATATTGGTAGCAAA
TAAA
 
AATAATAAAAACAATAACTTTAAACTGCTTTCTGGAGATGAATTACTCTCCTGGC
TATTT
TCTTTTTTACTTTAATGTAAAATGAGTATAACTGTAGTGAGTAAAATTCATTAAAT
TCCA
AGTTTTAGCAGAAAAAAAAAAAAAAAAAAA
NO:21, CDK6. Protein: 
MEKDGLCRADQQYECVAEIGEGAYGKVFKARDLKNGGRFVALKRVRVQTG
EEGMPLSTIREVAVLRHLETFEHPNVVRLFDVCTVSRTDRETKLTLVFEH
VDQDLTTYLDKVPEPGVPTETIKDMMFQLLRGLDFLHSHRVVHRDLKPQN
ILVTSSGQIKLADFGLARIYSFQMALTSVVVTLWYRAPEVLLQSSYATPV
DLWSVGCIFAEMFRRKPLFRGSSDVDQLGKILDVIGLPGEEDWPRDVALP
RQAFHSKSAQPIEKFVTDIDELGKDLLLKCLTFNPAKRISAYSALSHPYF
QDLERCKENLDSHLPPSQNTSELNTA
NO:6, FLJ16046 flanking 
TGATCTCCAGATTTACATATTCAGTTCCTACTTGACAACTCCCCTTGGATATTTCA
AAGA
TATCTCAAATTCAAAGTGTCACACCTGTCACACACTCTTCTGCTCTCTGCCCCTTC
AACC
TGATCCTCTCTTTTTTTNGACTCTATGAAAGGCATCNCCTTTCATTCTATTTAGCTA
GAG
ACTANAAGGCACTCTAGCATTCTTTCTCTACCCCTTACCCAATTGATTACCTAATC
CCAT
GGATTTCACCTCCTTAAATATCTCTGTCATCTCTTGCTTCCCTTGTCCCACTTTATC
TTC
ACCACCTCCACCTCCCGCCATCCAGAGAAATTAGTCATCCAGCTAGTTTCCTTATA
TTTA
CCTTTATACTCCTTTCCTGCATTAGNCATATGAAAGCCACAATGATTTCTAACAAG
ATAC
 
TAATCTGATATCCTGTTAAACTCCTTCNTAAAAAACTTTAGTGGCTTACCTTCAGT
CTTA
AGATAGAAAATATAACTTCTAAGAAGGACCCACATGGNTCCTCAAGGACTAGTTC
TCCTG
ACCTCTCCATTCTCATCACACAGGACTTGCCCCCTTGCTGTCTTCTCTTCAGTCCTG
CTT

NTGNNTCCCCCAGAAATTTTGTGTATGCCAGGCTCCTACATGCCAAAGAGCATTT
GCAAT
GCTGTTCCCTCTGTTTTAGAAAANCTTATA
NO:14, FLJ16046 cDNA 
GATACAGATCAGATGGTGACTGAATAGAAGCTGCCCCAGTCCTGGGCTCATGATG
TACGC
ACCTGTTGAATTTTCAGAAGCTGAATTCTCACGAGCTGAATATCAAAGAAAGCAG
CAATT
TTGGGACTCAGTACGGCTAGCTCTTTTCACATTAGCAATTGTAGCAATCATAGGA
ATTGC
AATTGGTATTGTTACTCATTTTGTTGTTGAGGATGATAAGTCTTTCTATTACCTTGC
CTC
TTTTAAAGTCACAAATATCAAATATAAAGAAAATTATGGCATAAGATCTTCAAGA
GAGTT
TATAGAAAGGAGTCATCAGATTGAAAGAATGATGTCTAGGATATTTCGACATTCT
TCTGT
AGGCGGTCGATTTATCAAATCTCATGTTATCAAATTAAGTCCAGATGAACAAGGT
GTGGA
TATTCTTATAGTGCTCATATTTCGATACCCATCTACTGATAGTGCTGAACAAATCA
AGAA
 
AAAAATTGAAAAGGCTTTATATCAAAGTTTGAAGACCAAACAATTGTCTTTGACC
TTAAA
CAAACCATCATTTAGACTCACACCTATTGACAGCAAAAAGATGAGGAATCTTCTC
AACAG
TCGCTGTGGAATAAGGATGACATCTTCAAACATGCCATTACCAGCATCCTCTTCT
ACTCA
AAGAATTGTCCAAGGAAGGGAAACAGCTATGGAAGGGGAATGGCCATGGCAGGC
CAGCCT
CCAGCTCATAGGGTCAGGCCATCAGTGTGGAGCCAGCCTCATCAGTAACACATGG
CTGCT
CACAGCAGCTCACTGCTTTTGGAAAAATAAAGACCCAACTCAATGGATTGCTACT
TTTGG
TGCAACTATAACACCACCCGCAGTGAAACGAAATGTGAGGAAAATTATTCTTCAT
GAGAA
TTACCATAGAGAAACAAATGAAAATGACATTGCTTTGGTTCAGCTCTCTACTGGA
GTTGA
GTTTTCAAATATAGTCCAGAGAGTTTGCCTCCCAGACTCATCTATAAAGTTGCCAC
CTAA
AACAAGTGTGTTCGTCACAGGATTTGGATCCATTGTAGATGATGGACCTATACAA
AATAC
ACTTCGGCAAGCCAGAGTGGAAACCATAAGCACTGATGTGTGTAACAGAAAGGA
TGTGTA
TGATGGCCTGATAACTCCAGGAATGTTATGTGCTGGATTCATGGAAGGAAAAATA
GATGC
ATGTAAGGGAGATTCTGGTGGACCTCTGGTTTATGATAATCATGACATCTGGTAC
ATTGT
GGGTATAGTAAGTTGGGGACAATCATGTGCGCTTCCCAAAAAACCTGGAGTCTAC
ACCAG
 
AGTAACTAAGTATCGAGATTGGATTGCCTCAAAGACCGGTATGTAG
TGTGGATTGTCCAT
GAGTTATACACATGGCACACAGAGCTGATACTCCTGCGTATTTGTATTGTTTAAA
TTCA
TTTACTTTGGATTAGTGCTTTTGCTAGATGTCAAGAAGCCCTTCAGACCCAGACAA
ATCT
AATATCCTGAGGTGGCCTTTACATACGTAGGACCAAACCCTCTCTACCATGAGGG
AAGAA
GACACAGCAAATGACAGACAGCACCTATTCCTTACTCACAAGGGAAACTGCTTGT
GATAC
TTCCTAATAAGATAAATGAGTGGTTTCCCTCAATTGAAGACAGGAACATCATTTT
CCACA
GGATATGAAGAGCTGCCAGTAATGCCAAAATCTTACCTCATATAATACCTGGAGC
ATGTG
AGATTCTTCTAGTGAAAAAGAACAGTCTTCCCTGAAGACTCAGGGCTTCAACATT
CTAGA
ACTGATAAGTGGACCTTCAGTGTGCAAGAATGGAGAAGCATGGGATTTGCATTAT
GACTT
GAACTGGGCTTATATCTAATAATACAGAGCACTATCACTAACCTCAACAGTTGAC
ATTTT
AAAAGTTTTTAAATGTATCTGAACTTGCTGTTAACACAGTGTTATAACTCAAGCA
CTAGC
TTCAGGAAGCATGTTGTGTTGTTAAGAAGCTTTTCTGATTTATTCTTTAACAGCAT
CTTG
CCATCTATATGTTAGTAGCAGTTGGCCCAGAAAGGAC
NO:22, FLJ16046 protein 
MMYAPVEFSEAEFSRAEYQRKQQFWDSVRLALFTLAIVAIIGIAIGIVTH
FVVEDDKSFYYLASFKVTNIKYKENYGIRSSREFIERSHQIERMMSRIFR
 
HSSVGGRFIKSHVIKLSPDEQGVDILIVLIFRYPSTDSAEQIKKKIEKAL
YQSLKTKQLSLTLNKPSFRLTPIDSKKMRNLLNSRCGIRMTSSNMPLPAS
SSTQRIVQGRETAMEGEWPWQASLQLIGSGHQCGASLISNTWLLTAAHCF
WKNKDPTQWIATFGATITPPAVKRNVRKIILHENYHRETNENDIALVQLS
TGVEFSNIVQRVCLPDSSIKLPPKTSVFVTGFGSIVDDGPIQNTLRQARV
ETISTDVCNRKDVYDGLITPGMLCAGFMEGKIDACKGDSGGPLVYDNHDI
WYIVGIVSWGQSCALPKKPGVYTRVTKYRDWIASKTGM
SEQ ID NO:7, PCSK flanking 
TGTTCTATGTATTATATAGATGAAATATCTTTCTTCTATCTTCCCTGAGGACACCA
TATG
AGATAACAGAATTTATATCCTGGTCTCTGTTTTAGTTCTTGGCACANAGCTCCTGA
GAAC
CTTGTCATTTCCTGATTGGGAAGAGCAATAGGAGGATCTTTTGTTATAATATTTGC
CTTT
GACCCTGTTCCTGACTCAGTACTAACATCCTTGTAAATTCCTAAGTGATAAGAGC
ACTAG
GAACATCCTTTGTTCTACGAAGGGGACTTGGGGTGGGCTCCTGGATGGGGGCTGG
TCACC
AAAAGGACCAAGCTACGATTANAAACTTGGAATTTTCAGCCCTGTCCCCCACTTC
TCTAN
AGAGGGGAGAACAATNAAGTCCNTTACTGATCATACCTACCTGAGGAAGCCTCCT
TAAAA
TCNCAATAGNNATGAGGATCTGGNGAGATTCCNAANTGNGCNAACNCATNCNNT
NCCNNG
AGGGTGNNNNACCCNNNCNCTGCCNGGNCAGANCCNCCTNGTNTTGNNANCTNC
CCNTAC
TTAACCNTTCCNNGGAANTCNTCAGAGT
SEQ ID NO:15, PCSK6 cDNA 
TCGCGGGCCGAGGACGCCTCTGGGGCGGCACCGCGTCCCGAGAGCCCCAGAAGT
CGGCGG
GGAAGTTTCCCCGGTGGGGGCGTTTCGGCCTCCCGGACGGCTCTCGGCCCCGG
AGCCC
GGTCGCAGGAGCGCGGGCCCGGGGGCGGAACGCGCCGCGGCCGCCTCCTCCTC
CCCGGC
TCCCGCCCGCGGCGGTGTTGGCGGCGGCGGTGGCGGCGGCGGCGCTTCCCCG
GCGCG
GAGCGGCTTTAAAAGGCGGCACTCCACCCCCGGCGCACTCGCAGCTCGGGCGCC
GCGCG
AGCCTGTCGCCGCTATGCCTCCGCGCGCCGCCTGCGCCCGGCCCCGGCCGCC
GCCCC
GGGCCGCCGCCGCCACCGACACCGCCGCGGGCGCGGGGGCGCGGGGGCGCGG
GGGGCG
CCGGCGGGCCCGGGTTCCGGCCGCTCGCGCCGCGTCCCTGGCGCTGGCTGCTGCT
GCTGG
CGCTGCCTGCCGCCTGCTCCGCGCCCCCGCCGCGCCCCGTCTACACCAACCACTG
GGCGG
TGCAAGTGCTGGGCGGCCCGGCCGAGGCGGACCGCGTGGCGGCGGCGCACGGGT
ACCTCA
ACTTGGGCCAGATTGGAAACCTGGAAGATTACTACCATTTTTATCACAGCAAAAC
CTTTA
AAAGATCAACCTTGAGTAGCAGAGGCCCTCACACCTTCCTCAGAATGGACCCCCA
GGTGA
AATGGCTCCAGCAACAGGAAGTGAAACGAAGGGTGAAGAGACAGGTGCGAAGT
GACCCGC
AGGCCCTTTACTTCAACGACCCCATTTGGTCCAACATGTGGTACCTGCATTGTGGC
GACA
 
AGAACAGTCGCTGCCGGTCGGAAATGAATGTCCAGGCAGCGTGGAAGAGGGGCT
ACACAG
GAAAAAACGTGGTGGTCACCATCCTTGATGATGGCATAGAGAGAAATCACCCTG
ACCTGG
CCCCAAATTATGATTCCTACGCCAGCTACGACGTGAACGGCAATGATTATGACCC
ATCTC
CACGATATGATGCCAGCAATGAAAATAAACACGGCACTCGTTGTGCGGGAGAAG
TTGCTG
CTTCAGCAAACAATTCCTACTGCATCGTGGGCATAGCGTACAATGCCAAAATAGG
AGGCA
TCCGCATGCTGGACGGCGATGTCACAGATGTGGTCGAGGCAAAGTCGCTGGGCAT
CAGAC
CCAACTACATCGACATTTACAGTGCCAGCTGGGGGCCGGACGACGACGGCAAGA
CGGTGG
ACGGGCCCGGCCGACTGGCTAAGCAGGCTTTCGAGTATGGCATTAAAAAGGGCC
GGCAGG
GCCTGGGCTCCATTTTCGTCTGGGCATCTGGGAATGGCGGAGAGAGGGGACTA
CTGCT
CGTGCGATGGCTACACCAACAGCATCTACACCATCTCCGTCAGCAGCGCCACCGA
GAATG
GCTACAAGCCCTGGTACCTGGAAGAGTGTGCCTCCACCCTGGCCACCACCTACAG
CAGTG
GGGCCTTTTATGAGCGAAAAATCGTCACCACGGATCTGCGTCAGCGCTGTACCGA
TGGCC
ACACTGGGACCTCAGTCTCTGCCCCCATGGTGGCGGGCATCATCGCCTTGGCTCT
AGAAG
CAAACAGCCAGTTAACCTGGAGGGACGTCCAGCACCTGCTAGTGAAGACATCCC
GGCCGG
 
CCCACCTGAAAGCGAGCGACTGGAAAGTGAACGGCGCGGGTCATAAAGTTAGCC
ATTTCT
ATGGATTTGGTTTGGTGGACGCAGAAGCTCTCGTTGTGGAGGCAAAGAAGTGGAC
AGCAG
TGCCATCGCAGCACATGTGTGTGGCCGCCTCGGACAAGAGACCCAGGAGCATCCC
CTTAG
TGCAGGTGCTGCGGACTACGGCCCTGACCAGCGCCTGCGCGGAGCACTCGGACC
AGCGGG
TGGTCTACTTGGAGCACGTGGTGGTTCGCACCTCCATCTCACACCCACGCCGAGG
AGACC
TCCAGATCTACCTGGTTTCTCCCTCGGGAACCAAGTCTCAACTTCTGGCAAAGAG
GTTGC
TGGATCTTTCCAATGAAGGGTTTACAAACTGGGAATTCATGACTGTCCACTGCTG
GGGAG
AAAAGGCTGAAGGGCAGTGGACCTTGGAAATCCAAGATCTGCCATCCCAGGTCC
GCAACC
CGGAGAAGCAAGGGAAGTTGAAAGAATGGAGCCTCATACTGTATGGCACAGCAG
AGCACC
CGTACCACACCTTCAGTGCCCATCAGTCCCGCTCGCGGATGCTGGAGCTCTCAGC
CCCAG
AGCTGGAGCCACCCAAGGCTGCCCTGTCACCCTCCCAGGTGGAAGTTCCTGAAGA
TGAGG
AAGATTACACAGGTGTGTGCCATCCGGAGTGTGGTGACAAAGGCTGTGATGGCCC
CAATG
CAGACCAGTGCTTGAACTGCGTCCACTTCAGCCTGGGGAGTGTCAAGACCAGCAG
GAAGT
GCGTGAGTGTGTGCCCCTTGGGCTACTTTGGGGACACAGCAGCAAGACGCTGTCG
CCGGT
 
GCCACAAGGGGTGTGAGACCTGCTCCAGCAGAGCTGCGACGCAGTGCCTGTCTTG
CCGCC
GCGGGTTCTATCACCACCAGGAGATGAACACCTGTGTGACCCTCTGTCCTGCAGG
ATTTT
ATGCTGATGAAAGTCAGAAAAATTGCCTTAAATGCCACCCAAGCTGTAAAAAGT
GCGTGG
ATGAACCTGAGAAATGTACTGTCTGTAAAGAAGGATTCAGCCTTGCACGGGGCA
GCTGCA
TTCCTGACTGTGAGCCAGGCACCTACTTTGACTCAGAGCTGATCAGATGTGGGGA
ATGCC
ATCACACCTGCGGAACCTGCGTGGGGCCAGGCAGAGAAGAGTGCATTCACTGTG
CGAAAA
ACTTCCACTTCCACGACTGGAAGTGTGTGCCAGCCTGTGGTGAGGGCTTCTACCC
AGAAG
AGATGCCGGGCTTGCCCCACAAAGTGTGTCGAAGGTGTGACGAGAACTGCTTGA
GCTGTG
CAGGCTCCAGCAGGAACTGTAGCAGGTGTAAGACGGGCTTCACACAGCTGGGGA
CCTCCT
GCATCACCAACCACACGTGCAGCAACGCTGACGAGACATTCTGCGAGATGGTGA
AGTCCA
ACCGGCTGTGCGAACGGAAGCTCTTCATTCAGTTCTGCTGCCGCACGTGCCTCCT
GGCCG
GGTAAGGGTGCCTAGCTGCCCACAGAGGGCAGGCACTCCCATCCATCCATCCGTC
CACCT
TCCTCCAGACTGTCGGCCAGAGTCTGTTTCAGGAGCGGCGCCCTGCACCTGACAG
CTTTA
TCTCCCCAGGAGCAGCATCTCTGAGCACCCAAGCCAGGTGGGTGGTGGCTCTTAA
GGAGG
 
TGTTCCTAAAATGGTGATATCCTCTCAAATGCTGCTTGTTGGCTCCAGTCTTCCGA
CAAA
CTAACAGGAACAAAATGAATTCTGGGAATCCACAGCTCTGGCTTTGGAGCAGCTT
CTGGG
ACCATAAGTTTACTGAATCTTCAAGACCAAAGCAGAAAAGAAAGGCGCTTGGCA
TCACAC
ATCACTCTTCTCCCCGTGCTTTTCTGCGGCTGTGTAGTAAATCTCCCCGGCCCAGC
TGGC
GAACCCTGGGCCATCCTCACATGTGACAAAGGGCCAGCAGTCTACCTGCTCGTTG
CCTGC
CACTGAGCAGTCTGGGGACGGTTTGGTCAGACTATAAATAAGATAGGTTTGAGGG
CATAA
AATGTATGACCACTGGGGCCGGAGTATCTATTTCTACATAGTCAGCTACTTCTGA
AACTG
CAGCAGTGGCTTAGAAAGTCCAATTCCAAAGCCAGACCAGAAGATTCTATCCCCC
GCAGC
GCTCTCCTTTGAGCAAGCCGAGCTCTCCTTGTTACCGTGTTCTGTCTGTGTCTTCA
GGAG
TCTCATGGCCTGAACGACCACCTCGACCTGATGCAGAGCCTTCTGAGGAGAGGCA
ACAGG
AGGCATTCTGTGGCCAGCCAAAAGGTACCCCGATGGCCAAGCAATTCCTCTGAAC
AAAAT
GTAAAGCCAGCCATGCATTGTTAATCATCCATCACTTCCCATTTTATGGAATTGCT
TTTA
AAATACATTTGGCCTCTGCCCTTCAGAAGACTCGTTTTTAAGGTGGAAACTCCTGT
GTCT
GTGTATATTACAAGCCTACATGACACAGTTGGATTTATTCTGCCAAACCTGTGTA
GGCAT
 
TTTATAAGCTACATGTTCTAATTTTTACCGATGTTAATTATTTTGACAAATATTTCA
TAT
ATTTTCATTGAAATGCACAGATCTGCTTGATCAATTCCCTTGAATAGGGAAGTAA
CATTT
GCCTTAAATTTTTTCGACCTCGTCTTTCTCCATATTGTCCTGCTCCCCTGTTTGACG
ACA
GTGCATTTGCCTTGTCACCTGTGAGCTGGAGAGAACCCAGATGTTGTTTATTGAAT
CTAC
AACTCTGAAAGAGAAATCAATGAAGCAAGTACAATGTTAACCCTAAATTAATAA
AAGAGT
TAACATCCCATGGC
SEQ ID NO:23, PCSK6 Protein 
MPPRAPPAPGPRPPPRAAAATDTAAGAGGAGGAGGAGGPGFRPLAPRPWR
WLLLLALPAACSAPPPRPVYTNHWAVQVLGGPAEADRVAAAHGYLNLGQI
GNLEDYYHFYHSKTFKRSTLSSRGPHTFLRMDPQVKWLQQQEVKRRVKRQ
VRSDPQALYFNDPIWSNMWYLHCGDKNSRCRSEMNVQAAWKRGYTGKNVV
VTILDDGIERNHPDLAPNYDSYASYDVNGNDYDPSPRYDASNENKHGTRC
AGEVAASANNSYCIVGIAYNAKIGGIRMLDGDVTDVVEAKSLGIRPNYID
IYSASWGPDDDGKTVDGPGRLAKQAFEYGIKKGRQGLGSIFVWASGNGGR
EGDYCSCDGYTNSIYTISVSSATENGYKPWYLEECASTLATTYSSGAFYE
RKIVTTDLRQRCTDGHTGTSVSAPMVAGIIALALEANSQLTWRDVQHLLV
KTSRPAHLKASDWKVNGAGHKVSHFYGFGLVDAEALVVEAKKWTAVPSQH
MCVAASDKRPRSIPLVQVLRTTALTSACAEHSDQRVVYLEHVVVRTSISH
PRRGDLQIYLVSPSGTKSQLLAKRLLDLSNEGFTNWEFMTVHCWGEKAEG
QWTLEIQDLPSQVRNPEKQGKLKEWSLILYGTAEHPYHTFSAHQSRSRML
ELSAPELEPPKAALSPSQVEVPEDEEDYTGVCHPECGDKGCDGPNADQCL
NCVHFSLGSVKTSRKCVSVCPLGYFGDTAARRCRRCHKGCETCSSRAATQ
CLSCRRGFYHHQEMNTCVTLCPAGFYADESQKNCLKCHPSCKKCVDEPEK
 
CTVCKEGFSLARGSCIPDCEPGTYFDSELIRCGECHHTCGTCVGPGREEC
IHCAKNFHFHDWKCVPACGEGFYPEEMPGLPHKVCRRCDENCLSCAGSSR
NCSRCKTGFTQLGTSCITNHTCSNADETFCEMVKSNRLCERKLFIQFCCR
TCLLAG
SEQ ID NO:8, PTGDR flanking 
GGTGCCTTAGACATTACAGGCGGGGCACCATGGGTGGCATCAGTGGTTGAGATG
ACTGCC
TTTGACTCAGGGTGTGACCCATGGGGTCCTGGGATCAAGTCCTGCATCCGGCTCC
CTGCA
GGGAGCCCACTTCTCCCTCTTCCTAGGTCTCTGCCTCTCTCCTTATATCTCTCATGA
ATA
AATAAATAAAAATCTTTAAAAAAAATTAGAGGCATTATGGATGGCACGTGATGT
GATTAG
CATTGGATTGACAAATTGACAAATTGAATTTAAGTAAAAAAAAATACAGGNAAA
AATGCT
ACTGGGAGGGGTGCCTGGGTCGCTCTGTTGGTTAAAACTTTGCCTTTGGCTCAGG
TCATG
ATCTCAGGGTTCTGNGNATTGAGCCCCACCTTAGGCTCTGCTTGTTTCTCTGCCCC
TCCC
CCTGCTNNNNTTTCTATCGAATAAANAAAANCCTTAAAAAAAAATGCTATTGGGA
GTTAT
TTGATTACCTACAAGTGAAAAGATNTGACAGTCGGAGATCANAAAAACATTATGT
CTATT
ACNTATTTTANCTTTTTTTTTTTTT
SEQ ID NO:16, PTCGR cDNA 
CGCCCGAGCCGCGCGGAGCTGCCGGGGCTCCTTAGCACCCGGGCGCCGGGG
CCCTCG
CCCTTCCGCAGCCTTCACTCCAGCCCTCTGCTCCCGCACGCCATGAAGTCGCCGTT
 
CTAC
CGCTGCCAGAACACCACCTCTGTGGAAAAAGGCAACTCGGCGGTGATGGCGGG
GTGCTC
TTCAGCACCGGCCTCCTGGGCAACCTGCTGGCCCTGGGGCTGCTGGCGCGCTCGG
GGCTG
GGGTGGTGCTCGCGGCGTCCACTGCGCCCGCTGCCCTCGGTCTTCTACATGCTGGT
GTGT
GGCCTGACGGTCACCGACTTGCTGGGCAAGTGCCTCCTAAGCCCGGTGGTGCTGG
CTGCC
TACGCTCAGAACCGGAGTCTGCGGGTGCTTGCGCCCGCATTGGACAACTCGTTGT
GCCAA
GCCTTCGCCTTCTTCATGTCCTTCTTTGGGCTCTCCTCGACACTGCAACTCCTGGCC
ATG
GCACTGGAGTGCTGGCTCTCCCTAGGGCACCCTTTCTTCTACCGACGGCACATCA
CCCTG
CGCCTGGGCGCACTGGTGGCCCCGGTGGTGAGCGCCTTCTCCCTGGCTTTCTGCG
CGCTA
CCTTTCATGGGCTTCGGGAAGTTCGTGCAGTACTGCCCCGGCACCTGGTGCTTTAT
CCAG
ATGGTCCACGAGGAGGGCTCGCTGTCGGTGCTGGGGTACTCTGTGCTCTACTCCA
GCCTC
ATGGCGCTGCTGGTCCTCGCCACCGTGCTGTGCAACCTCGGCGCCATGCGCAACC
TCTAT
GCGATGCACCGGCGGCTGCAGCGGCACCCGCGCTCCTGCACCAGGGACTGTGCC
GAGCCG
CGCGCGGACGGGAGGGAAGCGTCCCCTCAGCCCCTGGAGGAGCTGGATCACCTC
CTGCTG
CTGGCGCTGATGACCGTGCTCTTCACTATGTGTTCTCTGCCCGTAATTTATCGCGC
 
TTAC
TATGGAGCATTTAAGGATGTCAAGGAGAAAAACAGGACCTCTGAAGAAGCAGAA
GACCTC
CGAGCCTTGCGATTTCTATCTGTGATTTCAATTGTGGACCCTTGGATTTTTATCATT
TTC
AGATCTCCAGTATTTCGGATATTTTTCACAAGATTTTCATTAGACCTCTTAGGTA
CAGG
AGCCGGTGCAGCAATTCCACTAACATGGAATCCAGTCTGTGA1182CAGTGTTTTT
CACTCTGT
GGTAAGCTGAGGAATATGTCACATTTTCAGTCAAAGAACCATGATTAAAAAAAA
AAAGAC
AACTTACAATTTAAATCCTTAAAAGTTACCTCCCATAACAAAAGCATGTATATGT
ATTTT
CAAAAGTATTTGATATCTTAACAATGTGTTACCATTCTATAGTCATGAACCCCTTC
AGTG
CATTTTCATTTTTTATTAACAGCAACTAAAATTTATATATTGTAACCAGTGTTA
AAAG
TCTTAAAAAACAATGGTATTAATTGTCCCTACATTTGTGCTTGGTGGCCCTATTTT
TTTT
TTTTAGAGAGGCCTTGAGACATACAGGTCTTTTAAAATACAGTAGAAACACCACT
GTTTA
CGATTATACGATGGACATTCATAAAAAGCATAATTTCTTACCCTATTCATTTTTTG
GTGA
AACCTGATTCATTGATTTTATATCATTGCCGATGTTTAGTTCATTTCTTTGCCAATT
GAT
CTAAGCATAGCCTGAATTATGATGTTCCTCAGAGAAGTGAGGTGGGAAATATGAC
CAGGT
CAGGCAGTTGGAGGGGCTTCCCCAGCCACCATCGGGGAGTACTTGCTGCCTCAGG
 
TGGAG
ACCTGAAGCTGTAACTAGATGCAGAGCAAGATATGACTATAGCCCACAACCCAA
AGAAGC
AAAAATTCGTTTTTATCTTTTGAAATCCAGTTTCTTTTGTATTGAGTCAAGGGTGT
CAGT
AGGAATCAAAAGTTGGGGGTGGGTTGCAAAATGTTCTTTCAGTTTTTAGAACCTC
CATTT
TATAAAAGAATTATCCTATCAATGGATTCTTTAGTGGAAGGATTTATGCTTCTTTG
AAAA
CCAGTGTGTGACTCACTGTAGAGCCATGTTTACTGTTTGACTGTGTGGCACAGGG
GGGCA
TTTGGCACAGCAAAAAGCCCACCCAGGACTTAGCCTCAGTTGACGATAGTAACAA
TGGCC
TTAACATCTACCTTAACAGCTACCTATTACAGCCGTATTCTGCTGTCCGTGGAGAC
GGTA
AGATCTTAGGTTCCAAGATTTACTTCAAATTACACCTTCAAAACTGGAGCAGCA
TATAG
CCGAAAAGGAGCACAACTGAGCACTTTAATAGTAATTTAAAAGTTTTCAAGGGTC
AGCAA
TATGATGACTGAAAGGGAAAAGTGGAGGAAACGCAGCTGCAACTGAAGCGGAG
ACTCTAA
ACCCAGCTTGCAGGTAAGAGCTTTCACCTTTGGTAAAAGAACAGCTGGGGAGGTT
CAAGG
GGTTTCAGCATCTCTGGAGTTCCTTTGTATCTGACAATCTCAGGACTCCAAGGTGC
AAAG
CCTGCTGCATTTGCGTGATCTCAAGACCTCCAGCCAGAAGTCCCTTCCAAATATA
AGAGT
ACTCATGTTTATTTATTTCCAACTGAGCAGCAACCTCCTTTGTTTCACTTATGTTTT
 
TTC
CAGTATCTGAGATAATATAAAGCTGGGTAATTTTTATGTAATTTTTGGTATAGC
AAAA
CTGTGAAAAAGCCAAATTAGGCATACAAGGAGTATGATTTAACAGTATGACATG
ATGAAA
AAAATACAGTTGTTTTTGAAATTTAACTTTTGTTTGTACCTTCAATGTGTAAGTAC
ATGC
ATGTTTTATTGTCAGAGGAAGAACATGTTTTTGTATTCTTTTTTGGAGAGGTGT
GTTA
GGATAATTGTCCAGTTAATTTGAAAAGGCCCCAGATGAATCAATAAATATAATTT
TATAG
TAAAAAAAAAAAAAAAAAAAAAAAAA
SEQ ID NO:24, PTCGR Protein

MKSPFYRCQNTTSVEKGNSAVMGGVLFSTGLLGNLLALGLLARSGLGWCS
RRPLRPLPSVFYMLVCGLTVTDLLGKCLLSPVVLAAYAQNRSLRVLAPAL
DNSLCQAFAFFMSFFGLSSTLQLLAMALECWLSLGHPFFYRRHITLRLGA
LVAPVVSAFSLAFCALPFMGFGKFVQYCPGTWCFIQMVHEEGSLSVLGYS
VLYSSLMALLVLATVLCNLGAMRNLYAMHRRLQRHPRSCTRDCAEPRADG
REASPQPLEELDHLLLLALMTVLFTMCSLPVIYRAYYGAFKDVKEKNRTS
EEAEDLRALRFLSVISIVDPWIFIIFRSPVFRIFFHKIFIRPLRYRSRCS
NSTNMESSL

### Preferred embodiments:

One aspect of the invention relates to a method for preventing or treating influenza in a subject. The invention provides an antibody which binds to a polypeptide comprising the amino acid sequence of SEQ ID NO: 17 (PTCH) for use in a method for enhancing the resistance of a mammal to infection by an influenza virus or a method to treat influenza virus infection in a mammal.

The antibody for use in a method for enhancing the resistance of a mammal to infection by an influenza virus may be administered to said mammal to provide an antibody titer in said mammal effective to enhance resistance of said mammal to infection by influenza. The antibody for use in a method of treating influenza virus infection in a mammal may bind to a polypeptide comprising the amino acid sequence of SEQ ID NO: 17 in an amount effective to treat influenza virus when administered.

A second aspect of the invention relates to a pharmaceutical composition for preventing or treating influenza in a subject, said composition comprising a pharmaceutically acceptable carrier and an antibody which binds to a polypeptide comprising the amino acid sequence of SEQ ID NO: 17 (PTCH). The pharmaceutical composition is for use in a method of enhancing the resistance of a mammal to influenza virus.

In a related embodiment, the pharmaceutical composition further comprises a pharmaceutically acceptable delivery vehicle.

A third aspect of the present invention relates to the pharmaceutical composition described above, for use in a method for preventing or treating influenza in a subject, comprising the step of introducing into the subject an effective amount of said pharmaceutical composition.

The antibody of the invention may bind to the polypeptide comprising the amino acid sequence of SEQ ID NO: 17 (PTCH) with a binding affinity equal to or greater than 10⁵M⁻¹. The antibody may be a humanised antibody.

## Claims

1. An antibody which binds to a polypeptide comprising the amino acid sequence of SEQ ID NO: 17 (PTCH) for use in a method for enhancing the resistance of a mammal to infection by an influenza virus or a method to treat influenza virus infection in a mammal.

2. An antibody for use according to claim 1 which binds to a polypeptide comprising the amino acid sequence of SEQ ID NO: 17 (PTCH) for use in a method for enhancing the resistance of a mammal to infection by an influenza virus.

3. The antibody for use according to claim 2, wherein said antibody is administered to said mammal to provide an antibody titer in said mammal effective to enhance the resistance of said mammal to infection by influenza.

4. A pharmaceutical composition for use in a method of enhancing the resistance of a mammal to influenza virus, wherein said composition comprises an antibody suspended in a pharmaceutically acceptable carrier, which antibody binds to a polypeptide comprising the amino acid sequence of SEQ ID NO: 17 (PTCH).

5. An antibody for use according to claim 1 which binds to a polypeptide comprising the amino acid sequence of SEQ ID NO: 17 in an amount effective to treat influenza virus when administered to a mammal for use in a method of treating influenza virus infection in a mammal.

6. The antibody or composition for use according to any preceding claim wherein said antibody binds to the polypeptide comprising the amino acid sequence of SEQ ID NO: 17 (PTCH) with a binding affinity equal to or greater than 10⁵M⁻¹.

7. The antibody or composition for use according to any preceding claim wherein said antibody is a humanised antibody.

## Patentansprüche

1. Antikörper, welcher an ein Polypeptid bindet, welches die Aminosäuresequenz von SEQ ID NO: 17 (PTCH) umfasst, zur Verwendung in einem Verfahren zur Steigerung der Resistenz eines Säugetiers gegenüber Infektion durch ein Influenzavirus oder einem Verfahren zur Behandlung einer Influenzavirusinfektion in einem Säugetier.

2. Antikörper zur Verwendung gemäß Anspruch 1, welcher an ein Polypeptid bindet, welches die Aminosäuresequenz von SEQ ID NO: 17 (PTCH) umfasst, zur Verwendung in einem Verfahren zur Steigerung der Resistenz eines Säugetiers gegenüber Infektion durch ein Influenzavirus.

3. Antikörper zur Verwendung gemäß Anspruch 2, wobei der Antikörper an das Säugetier verabreicht wird, um einen Antikörpertiter in dem Säugetier bereitzustellen, der wirksam ist, die Resistenz des Säugetiers gegenüber Infektion durch Influenza zu steigern.

4. Pharmazeutische Zusammensetzung zur Verwendung in einem Verfahren der Steigerung der Resistenz eines Säugetiers gegenüber Influenzavirus, worin die Zusammensetzung einen Antikörper, suspendiert in einem pharmazeutisch annehmbaren Träger, umfasst, wobei der Antikörper an ein Polypeptid bindet, welches die Aminosäuresequenz von SEQ ID NO: 17 (PTCH) umfasst.

5. Antikörper zur Verwendung gemäß Anspruch 1, welcher an ein Polypeptid bindet, welches die Aminosäuresequenz von SEQ ID NO: 17 umfasst, in einer Menge, welche wirksam ist, Influenzavirus zu behandeln, wenn sie einem Säugetier zur Verwendung in einem Verfahren der Behandlung einer Influenzavirusinfektion in einem Säugetier verabreicht wird.

6. Antikörper oder Zusammensetzung zur Verwendung gemäß einem der vorangehenden Ansprüche, wobei der Antikörper an das Polypeptid, welches die Aminosäuresequenz von SEQ ID NO: 17 (PTCH) umfasst, mit einer Bindungsaffinität gleich oder größer als 10⁵ M⁻¹ bindet.

7. Antikörper oder Zusammensetzung zur Verwendung gemäß einem der vorangehenden Ansprüche, wobei der Antikörper ein humanisierter Antikörper ist.

## Revendications

1. Anticorps qui se lie à un polypeptide comprenant la séquence d'acides aminés de la Séquence N° 17 (PTCH), pour utilisation dans un procédé visant à renforcer la résistance d'un mammifère à une infection à virus grippal ou dans un procédé de traitement d'une infection à virus grippal chez un mammifère.

2. Anticorps pour utilisation conforme à la revendication 1, qui se lie à un polypeptide comprenant la séquence d'acides aminés de la Séquence N° 17 (PTCH), pour utilisation dans un procédé visant à renforcer la résistance d'un mammifère à une infection à virus grippal.

3. Anticorps pour utilisation conforme à la revendication 2, dans laquelle on administre ledit anticorps audit mammifère de façon à procurer audit mammifère cet anticorps en un titre efficace pour renforcer la résistance dudit mammifère à une infection à virus grippal.

4. Composition pharmaceutique pour utilisation dans un procédé visant à renforcer la résistance d'un mammifère à une infection à virus grippal, laquelle composition comprend un anticorps en suspension dans un véhicule pharmacologiquement admissible, lequel anticorps se lie à un polypeptide comprenant la séquence d'acides aminés de la Séquence N° 17 (PTCH).

5. Anticorps pour utilisation conforme à la revendication 1, qui se lie à un polypeptide comprenant la séquence d'acides aminés de la Séquence N° 17 en une quantité efficace pour traiter un virus grippal, quand on l'administre à un mammifère, pour utilisation dans un procédé visant à traiter une infection à virus grippal chez un mammifère.

6. Anticorps ou composition pour utilisation conforme à l'une des revendications précédentes, lequel anticorps se lie au polypeptide comprenant la séquence d'acides aminés de la Séquence N° 17 (PTCH) avec une affinité de liaison supérieure ou égale à 10⁵ M⁻¹.

7. Anticorps ou composition pour utilisation conforme à l'une des revendications précédentes, lequel anticorps est un anticorps humanisé.
